# EUROPEAN PATENT SPECIFICATION

(11) **EP 3 844 159 B1**
(45) Date of publication and mention of the grant of the patent: **03.05.2023**
(21) Application number: 18759951.9
(22) Date of filing: 28.08.2018
(51) Int. Cl.: C07D 403/04, C07D 471/04, A61P 37/00, A61K 31/498, A61K 31/47

(54) **NOVEL PYRROLIDINYL AMIDE COMPOUNDS FOR THE TREATMENT OF AUTOIMMUNE DISEASE**
NEUARTIGE PYRROLIDINYLAMIDVERBINDUNGEN ZUR BEHANDLUNG VON AUTOIMMUNERKRANKUNGEN
NOUVEAUX COMPOSÉS D'AMIDE PYRROLIDINYLE DESTINÉS À ÊTRE UTILISÉS DANS LE TRAITEMENT D'UNE MALADIE AUTO-IMMUNE

(43) Date of publication of application: 07.07.2021
(73) Proprietor: F. Hoffmann-La Roche AG, 4070 Basel (CH)
(72) Inventor: LIU, Haixia, Shanghai 201203 (CN); WU, Guolong, Shanghai 201203 (CN); ZHANG, Weixing, Shanghai 201203 (CN); ZHU, Wei, Shanghai 201203 (CN); SHEN, Hong, Shanghai 201203 (CN); DEY, Fabian, 4070 Basel (CH)
(74) Representative: Belkacemi, Doria
(86) International application number: PCT/EP2018/073079
(87) International publication number: WO 2020/043271

(56) References cited:
- WO-A1-2016/096778
- WO-A1-2016/141092

## Description

The present invention relates to organic compounds useful for therapy and/or prophylaxis in a mammal, and in particular to antagonist of TLR7 and/or TLR8 and/or TLR9 useful for treating systemic lupus erythematosus or lupus nephritis.

### FIELD OF THE INVENTION

Autoimmune connective tissue disease (CTD) include prototypical autoimmune syndromes such as Systemic Lupus Erythematosus (SLE), primary Sjögren's syndrome (pSjS), mixed connective tissue disease (MCTD), Dermatomyositis/Polymyositis (DM/PM), Rheumatoid Arthritis (RA), and systemic sclerosis (SSc). With the exception of RA, no really effective and safe therapies are available to patients. SLE represents the prototypical CTD with a prevalence of 20-150 per 100,000 and causes broad inflammation and tissue damage in distinct organs, from commonly observed symptoms in the skin and joints to renal, lung, or heart failure. Traditionally, SLE has been treated with nonspecific anti-inflammatory or immunosuppressive drugs. However, long term usage of immunosuppressive drug, e.g. corticosteroids is only partially effective, and is associated with undesirable toxicity and side effects. Belimumab is the only FDA-approved drug for lupus in the last 50 years, despite its modest and delayed efficacy in only a fraction of SLE patients (Navarra, S. V. et al Lancet 2011, 377, 721.). Other biologics, such as anti-CD20 mAbs, mAbs against or soluble receptors of specific cytokines, have failed in most clinical studies. Thus, novel therapies are required that provide sustained improvement in a greater proportion of patient groups and are safer for chronic use in many autoimmune as well as auto-inflammation diseases.

Toll Like Receptors (TLR) are an important family of pattern recognition receptors (PRR) which can initiate broad immune responses in a wide variety of immune cells. As natural host defense sensors, endosomal TLRs 7, 8 and 9 recognize nucleic acids derived from viruses, bacteria; specifically, TLR7/8 and TLR9 recognize single-stranded RNA (ssRNA) and single-stranded CpG-DNA, respectively. However, aberrant nucleic acid sensing of TRL7/8/9 is considered as a key node in a broad of autoimmune and auto-inflammatory diseases (Krieg, A. M. et al. Immunol. Rev. 2007, 220, 251. Jiménez-Dalmaroni, M. J. et al Autoimmun Rev. 2016, 15, 1. Chen, J. Q., et al. Clinical Reviews in Allergy & Immunology 2016, 50, 1.) Therefore, TLR7/8/9 represents a new therapeutic target for autoimmune and auto-inflammatory diseases, for which no effective steroid-free and non-cytotoxic oral drugs exist, and inhibition of these pathways from the very upstream may deliver satisfying therapeutic effects. From a safety perspective, because there are multiple nucleic acid sensing pathways (e.g. other TLRs, cGAS/STING), such redundancy should still allow responses to infection in the presence of TLR7/8/9 inhibition. As such, we proposed and invented oral compounds that target and suppress TLR7/8/9 for the treatment of autoimmune and auto-inflammatory diseases.

### SUMMARY OF THE INVENTION

The present invention relates to novel compounds of formula (I), 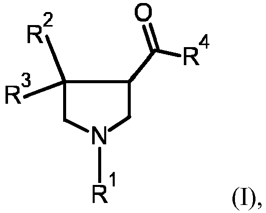 wherein
R¹ is 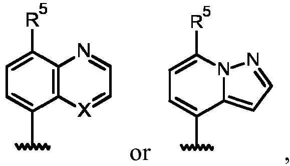 wherein
   R⁵ is cyano, C₁₋₆alkyl, halogen, haloC₁₋₆alkyl or nitro;
   X is N or CH;
R² and R³ are independently selected from H, C₁₋₆alkyl, C₃₋₇cycloalkyl, haloC₁₋₆alkyl, or R² and R³ together with the carbon they are attached to form C₃₋₇cycloalkyl;
R⁴ is heterocyclyl, heterocyclylamino, heterocyclylC₁₋₆alkylamino, (C₁₋₆alkyl)₂aminoC₁₋₆alkylamino, aminoC₃₋₇cycloalkylamino, [(C₁₋₆alkyl)₂aminoC₁₋₆alkyl]C₃₋₇cycloalkylamino, aminoC₃₋₇cycloalkylC₁₋₆alkylamino or aminoC₁₋₆alkylamino;
or a pharmaceutically acceptable salt, enantiomer or diastereomer thereof.

Another object of the present invention is related to novel compounds of formula (I), their manufacture, medicaments based on a compound in accordance with the invention and their production as well as the use of compounds of formula (I) as TLR7 and/or TLR8 and/or TLR9 antagonist, and for the treatment or prophylaxis of systemic lupus erythematosus or lupus nephritis. The compounds of formula (I) show superior TLR7 and/or TLR8 and/or TLR9 antagonism activity. In addition, the compounds of formula (I) also show good cytotoxicity, solubility, human microsome stability and SDPK profiles, as well as low CYP inhibition.

### DETAILED DESCRIPTION OF THE INVENTION

### DEFINITIONS

The term "C₁₋₆alkyl" denotes a saturated, linear or branched chain alkyl group containing 1 to 6, particularly 1 to 4 carbon atoms, for example methyl, ethyl, *n*-propyl, isopropyl, *n*-butyl, isobutyl, *tert*-butyl and the like. Particular "C₁₋₆alkyl" groups are methyl, ethyl and isopropyl.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group has been replaced by same or different halogen atoms, particularly fluoro atoms. Examples of haloC₁₋₆alkyl include monofluoro-, difluoro-or trifluoro-methyl, - ethyl or -propyl, for example 3,3,3-trifluoropropyl, 2-fluoroethyl, 2,2,2-trifluoroethyl, fluoromethyl, difluoromethyl, trifluoromethyl and trifluoroethyl.

The term "C₃₋₇cycloalkyl" denotes a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "C₃₋₇cycloalkyl" groups are cyclopropyl and cyclohexyl.

The term "halopyrrolidinyl" denotes a pyrrolidinyl group wherein at least one of the hydrogen atoms of the pyrrolidinyl group has been replaced by same or different halogen atoms, particularly fluoro atoms. Examples of halopyrrolidinyl include fluoropyrrolidinyl and difluoropyrrolidinyl.

The term "heterocyclyl" denotes a monovalent saturated or partly unsaturated mono- or bicyclic ring system of 3 to 12 ring atoms, comprising 1, 2, or 3 ring heteroatoms selected from N, O and S, the remaining ring atoms being carbon. In particular embodiments, heterocyclyl is a monovalent saturated monocyclic ring system of 4 to 10 ring atoms, comprising 1, 2, or 3 ring heteroatoms selected from N, O and S, the remaining ring atoms being carbon. Examples for monocyclic saturated heterocyclyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Heterocyclyl can be fully or partially saturated. Examples for bicyclic saturated heterocyclyl are bicyclo[3.1.0]hexanyl, spiro[3.3]heptanyl, azabicyclo[2.2.1]heptanyl, azabicyclo[3.2.1]octanyl, azabicyclo[3.3.1]nonanyl, azaspiro[3.5]nonanyl, diazaspiro[4.4]nonanyl, diazaspiro[5.5]undecanyl, 1,2,3,3a,4,5,6,6a-octahydrocyclopenta[c]pyrrolyl, oxaazabicyclo[3.3.1]nonanyl. Examples for partially saturated heterocyclyl are dihydrofuryl, imidazolinyl, dihydro-oxazolyl, tetrahydropyridinyl, and dihydropyranyl. Monocyclic or bicyclic heterocyclyl can be further substituted by halogen, hydroxy, amino, C₁₋₆alkyl, haloC₁₋₆alkyl or heterocyclyl.

The term "heterocyclylamino" denotes heterocyclyl-NH-, wherein "heterocyclyl" is defined as above.

The term "enantiomer" denotes two stereoisomers of a compound which are non-superimposable mirror images of one another.

The term "diastereomer" denotes a stereoisomer with two or more centers of chirality and whose molecules are not mirror images of one another. Diastereomers have different physical properties, e.g. melting points, boiling points, spectral properties, and reactivities.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### ANTAGONIST OF TLR7 AND/OR TLR8 AND/OR TLR9

The present invention relates to a compound of formula (I), 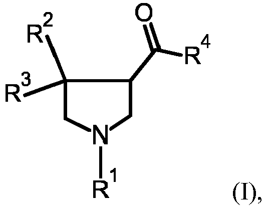 wherein
R¹ is 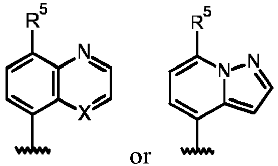 , wherein
   R⁵ is cyano, C₁₋₆alkyl, halogen, haloC₁₋₆alkyl or nitro;
   X is N or CH;
R² and R³ are independently selected from H, C₁₋₆alkyl, C₃₋₇cycloalkyl, haloC₁₋₆alkyl, or R² and R³ together with the carbon they are attached to form C₃₋₇cycloalkyl;
R⁴ is heterocyclyl, heterocyclylamino, heterocyclylC₁₋₆alkylamino, (C₁₋₆alkyl)₂aminoC₁₋₆alkylamino, aminoC₃₋₇cycloalkylamino, [(C₁₋₆alkyl)₂aminoC₁₋₆alkyl]C₃₋₇cycloalkylamino, aminoC₃₋₇cycloalkylC₁₋₆alkylamino or aminoC₁₋₆alkylamino;
or a pharmaceutically acceptable salt, enantiomer or diastereomer thereof.

A further embodiment of present invention is (ii) a compound of formula (I), wherein
R¹ is 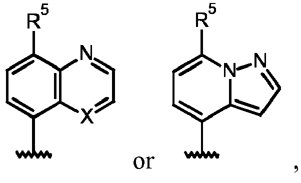 wherein
   R⁵ is cyano, C₁₋₆alkyl, halogen, haloC₁₋₆alkyl or nitro;
   X is N or CH;
R² is H;
R³ is H, C₁₋₆alkyl, C₃₋₇cycloalkyl, haloC₁₋₆alkyl;
   or R² and R³ together with the carbon they are attached to form C₃₋₇cycloalkyl;
R⁴ is heterocyclyl, heterocyclylamino, heterocyclylC₁₋₆alkylamino, (C₁₋₆alkyl)₂aminoC₁₋₆alkylamino, aminoC₃₋₇cycloalkylamino, [(C₁₋₆alkyl)₂aminoC₁₋₆alkyl]C₃₋₇cycloalkylamino, aminoC₃₋₇cycloalkylC₁₋₆alkylamino or aminoC₁₋₆alkylamino;
or a pharmaceutically acceptable salt, enantiomer or diastereomer thereof.

A further embodiment of present invention is (iii) a compound of formula (I) according to (ii), wherein R⁴ is (C₁₋₆alkyl)₂aminoC₁₋₆alkylamino, (C₁₋₆alkylmorpholinyl)C₁₋₆alkylamino, (C₁₋₆alkylpiperidyl)C₁₋₆alkylamino, (morpholinylC₁₋₆alkyl)amino, [(C₁₋₆alkyl)₂aminoC₁₋₆alkyl]C₃₋₇cycloalkylamino, aminoazetidinyl, aminobicyclo[3.1.0]hexanylamino, aminoC₁₋₆alkylamino, aminoC₃₋₇cycloalkylamino, aminoC₃₋₇cycloalkylC₁₋₆alkylamino, aminospiro[3.3]heptanylamino, azabicyclo[2.2.1]heptanylamino, azabicyclo[3.2.1]octanylamino, azabicyclo[3.3.1]nonanylamino, azaspiro[3.5]nonanylamino, azepanylamino, C₁₋₆alkoxycarbonylpyrrolidinylamino, C₁₋₆alkylazaspiro[2.4]heptanylamino, C₁₋₆alkylpiperidylamino, diazaspiro[4.4]nonanyl, diazaspiro[5.5]undecanyl, halopyrrolidinylamino, morpholinylC₁₋₆alkylamino, octahydrocyclopenta[c]pyrrolylamino, oxaazabicyclo[3.3.1]nonanylamino or piperidylpiperidylamino.

A further embodiment of present invention is (iv) a compound of formula (I) according to (iii), wherein R⁵ is cyano, methyl, chloro, trifluoromethyl or nitro.

A further embodiment of present invention is (v) a compound of formula (I) according to (iv), wherein R³ is H, methyl, ethyl, isopropyl, difluoromethyl, trifluoromethyl or cyclopropyl; or R² and R³ together with the carbon they are attached to form cyclopropyl.

A further embodiment of present invention is (vi) a compound of formula (I) according to (v), wherein R³ is methyl or trifluoromethyl; or R² and R³ together with the carbon they are attached to form cyclopropyl.

A further embodiment of present invention is (vii) a compound of formula (I) according to (v) or (vi), wherein R⁴ is (3-aminocyclobutyl)methylamino, (dimethylamino)ethylamino, (methylmorpholinyl)methylamino, (methylpiperidyl)methylamino, (morpholinylethyl)amino, (morpholinylmethyl)amino, [(dimethylamino)methyl]cyclobutylamino, 1,2,3,3a,4,5,6,6a-octahydrocyclopenta[c]pyrrol-5-ylamino, 2,7-diazaspiro[4.4]nonanyl, 2-azabicyclo[2.2.1]heptan-5-ylamino, 3,9-diazaspiro[5.5]undecanyl, 3-azabicyclo[3.2.1]octan-8-ylamino, 3-azabicyclo[3.3.1]nonan-7-ylamino, 3-azabicyclo[3.3.1]nonan-9-ylamino, 3-oxa-7-azabicyclo[3.3.1]nonan-9-ylamino, 3-oxa-9-azabicyclo[3.3.1]nonan-7-ylamino, 5-methyl-5-azaspiro[2.4]heptan-7-ylamino, 6-aminospiro[3.3]heptan-2-ylamino, 7-azaspiro[3.5]nonan-2-ylamino, 8-azabicyclo[3.2.1]octan-3-ylamino, 9-azabicyclo[3.3.1]nonan-3-ylamino, amino-2-bicyclo[3.1.0]hexanylamino, aminoazetidinyl, aminocyclobutylamino, aminocyclohexylamino, aminomethylpropylamino, azepanylamino, fluoropyrrolidinylamino, methoxycarbonylpyrrolidinylamino, methylpiperidylamino or piperidylpiperidylamino.

A further embodiment of present invention is (viii) a compound of formula (I) according to (vii), wherein R⁴ is methylpiperidylamino.

Another embodiment of present invention is that (ix) particular compounds of formula (I) are selected from:
(*3R*,*4R*)-1-(8-cyanoquinoxalin-5-yl)-*N*(1-methyl-4-piperidyl)-4-(trifluoromethyl) pyrrolidine-3-carboxamide;
*(3R,4R)-N-*(1-methyl-4-piperidyl)-4-(trifluoromethyl)-1-[8-(trifluoromethyl)quinoxalin-5-yl]pyrrolidine-3-carboxamide;
*(3R*,*4R)*-1-(8-cyanoquinoxalin-5-yl)-4-methyl-*N*-(1-methyl-4-piperidyl)pyrrolidine-3-carboxamide;
*(3R,4R)*-1-(8-cyano-5-quinolyl)-*N-*(1-methyl-4-piperidyl)-4-(trifluoromethyl)pyrrolidine-3-carboxamide;
*(3R*,*4R)*-4-methyl-*N*-(1-methyl-4-piperidyl)-1-[8-(trifluoromethyl)quinoxalin-5-yl]pyrrolidine-3-carboxamide;
*N*-(3-oxa-9-azabicyclo[3.3.1]nonan-7-yl)-5-[8-(trifluoromethyl)quinoxalin-5-yl]-5-azaspiro[2.4]heptane-7-carboxamide;
5-(8-Cyanoquinoxalin-5-yl)-*N*-(1-methyl-4-piperidyl)-5-azaspiro[2.4]heptane-7-carboxamide;
(7R)-5-(8-Cyanoquinoxalin-5-yl)-*N*-(1-methyl-4-piperidyl)-5-azaspiro[2.4]heptane-7-carboxamide;
(7S)-5-(8-Cyanoquinoxalin-5-yl)-*N*-(1-methyl-4-piperidyl)-5-azaspiro[2.4]heptane-7-carboxamide;
*N*-(3-azabicyclo[3.3.1]nonan-9-yl)-5-[8-(trifluoromethyl)quinoxalin-5-yl]-5-azaspiro[2.4]heptane-7-carboxamide;
*N*-(9-azabicyclo[3.3.1]nonan-3-yl)-5-[8-(trifluoromethyl)quinoxalin-5-yl]-5-azaspiro[2.4]heptane-7-carboxamide;
*N*-(1-methyl-4-piperidyl)-5-[8-(trifluoromethyl)quinoxalin-5-yl]-5-azaspiro[2.4]heptane-7-carboxamide;
*N-*(3-azabicyclo[3.2.1]octan-8-yl)-5-[8-(trifluoromethyl)quinoxalin-5-yl]-5-azaspiro[2.4]heptane-7-carboxamide;
5-(8-Cyanoquinoxalin-5-yl)-*N*-(morpholin-2-ylmethyl)-5-azaspiro[2.4]heptane-7-carboxamide;
*Trans-*1-(8-cyano-5-quinolyl)-4-cyclopropyl-*N*-(1-methyl-4-piperidyl)pyrrolidine-3-carboxamide;
*N*-(3-azabicyclo[3.3.1]nonan-7-yl)-5-[8-(trifluoromethyl)quinoxalin-5-yl]-5-azaspiro[2.4]heptane-7-carboxamide;
*(3R*,*4R)*-1-(8-cyano-5-quinolyl)-4-methyl-*N*-(1-methyl-4-piperidyl)pyrrolidine-3-carboxamide;
*Trans-*1*-*(8-cyano-5-quinolyl)-*N*-(1-methyl-4-piperidyl)-4-(trifluoromethyl)pyrrolidine-3-carboxamide;
1-(8-Cyano-5-quinolyl)-*N-*(1-methyl-4-piperidyl)-4-(trifluoromethyl)pyrrolidine-3-carboxamide;
*Trans*-1-(8-cyano-5-quinolyl)-4-cyclopropyl-*N*-[2-(dimethylamino)ethyl]pyrrolidine-3-carboxamide;
*N*-(5-methyl-5-azaspiro[2.4]heptan-7-yl)-5-[8-(trifluoromethyl)quinoxalin-5-yl]-5-azaspiro[2.4]heptane-7-carboxamide;
*N*-(1,2,3,3a,4,5,6,6a-octahydrocyclopenta[c]pyrrol-5-yl)-5-[8-(trifluoromethyl)quinoxalin-5-yl]-5-azaspiro[2.4]heptane-7-carboxamide;
*N-*(6-aminospiro[3.3]heptan-2-yl)-5-[8-(trifluoromethyl)quinoxalin-5-yl]-5-azaspiro[2.4]heptane-7-carboxamide;
*N*-(8-azabicyclo[3.2.1]octan-3-yl)-5-[8-(trifluoromethyl)quinoxalin-5-yl]-5-azaspiro[2.4]heptane-7-carboxamide;
*N*-(4-aminocyclohexyl)-5-[8-(trifluoromethyl)quinoxalin-5-yl]-5-azaspiro[2.4]heptane-7-carboxamide;
*(3S*,*4S)*-4-Methyl-*N-*(1-methyl-4-piperidyl)-1-[8-(trifluoromethyl)quinoxalin-5-yl]pyrrolidine-3-carboxamide;
*N*-[3-[(dimethylamino)methyl]cyclobutyl]-5-[8-(trifluoromethyl)quinoxalin-5-yl]-5-azaspiro[2.4]heptane-7-carboxamide;
*(3R,4R)*-4-Methyl-N-(1-methyl-4-piperidyl)-1-(8-nitro-5-quinolyl)pyrrolidine-3-carboxamide;
*Trans-*1-(8-cyano-5-quinolyl)-4-methyl-*N*-(1-methyl-4-piperidyl)pyrrolidine-3-carboxamide;
*Trans*-1-(8-cyano-5-quinolyl)-4-ethyl-*N-*(1-methyl-4-piperidyl)pyrrolidine-3-carboxamide;
*N-*(azepan-4-yl)-1-(8-cyano-5-quinolyl)-4-(trifluoromethyl)pyrrolidine-3-carboxamide;
*N-*[(3-aminocyclobutyl)methyl]-5-[8-(trifluoromethyl)quinoxalin-5-yl]-5-azaspiro[2.4]heptane-7-carboxamide;
*Trans*-5-[3-(3,9-diazaspiro[5.5]undecane-3-carbonyl)-4-methyl-pyrrolidin-1-yl]quinoline-8-carbonitrile;
*(3R,4R)-*4-Methyl-*N-*(1-methyl-4-piperidyl)-1-(8-methylquinoxalin-5-yl)pyrrolidine-3-carboxamide;
*N*-(7-azaspiro[3.5]nonan-2-yl)-5-[8-(trifluoromethyl)quinoxalin-5-yl]-5-azaspiro[2.4]heptane-7-carboxamide;
*(3R,4R)*-1-(8-Cyanoquinoxalin-5-yl)-*N*-[1-(4-piperidyl)-4-piperidyl]-4-(trifluoromethyl)pyrrolidine-3-carboxamide;
*N*-[ (*1R,2S,4R,5S*)-4-amino-2-bicyclo[3.1.0]hexanyl]-5-[8-(trifluoromethyl)quinoxalin-5-yl]-5-azaspiro[2.4]heptane-7-carboxamide;
*Trans-*1-(8-cyano-5-quinolyl)-4-isopropyl-*N*-(1-methyl-4-piperidyl)pyrrolidine-3-carboxamide;
*N*-(3-Aminocyclobutyl)-5-[8-(trifluoromethyl)quinoxalin-5-yl]-5-azaspiro[2.4]heptane-7-carboxamide;
*N*-(4-Aminocyclohexyl)-5-[8-(trifluoromethyl)quinoxalin-5-yl]-5-azaspiro[2.4]heptane-7-carboxamide;
*Trans-*1-(8-cyano-5-quinolyl)-4-(difluoromethyl)-*N*-(1-methyl-4-piperidyl)pyrrolidine-3-carboxamide;
5-(8-Cyano-5-quinolyl)-*N-*(1-methyl-4-piperidyl)-5-azaspiro[2.4]heptane-7-carboxamide;
*N*-(3-Oxa-7-azabicyclo[3.3.1]nonan-9-yl)-5-[8-(trifluoromethyl)quinoxalin-5-yl]-5-azaspiro[2.4]heptane-7-carboxamide;
*Cis*-*N*-(3-aminocyclohexyl)-5-[8-(trifluoromethyl)quinoxalin-5-yl]-5-azaspiro[2.4]heptane-7-carboxamide;
*(3R*,*4R)*-1-(7-cyanopyrazolo[1,5-a]pyridin-4-yl)-4-methyl-*N-*(1-methyl-4-piperidyl)pyrrolidine-3-carboxamide;
*Trans*-1-(8-chloro-5-quinolyl)-*N*-(1-methyl-4-piperidyl)-4-(trifluoromethyl)pyrrolidine-3-carboxamide;
*Trans*-1-(8-cyano-5-quinolyl)-*N*-[(3S,4R)-4-fluoropyrrolidin-3-yl]-4-(trifluoromethyl)pyrrolidine-3-carboxamide;
*N-*(2-Azabicyclo[2.2.1]heptan-5-yl)-5-[8-(trifluoromethyl)quinoxalin-5-yl]-5-azaspiro[2.4]heptane-7-carboxamide;
*Trans*-1-(8-cyano-5-quinolyl)-*N-*[(*3S,4R*)-4-fluoropyrrolidin-3-yl]-4-isopropyl-pyrrolidine-3-carboxamide;
(*3R*,*4R*)-1-(8-chloro-5-quinolyl)-4-methyl-*N*-(1-methyl-4-piperidyl)pyrrolidine-3-carboxamide;
5-(8-Cyano-5-quinolyl)-*N-*[(1-methyl-3-piperidyl)methyl]-5-azaspiro[2.4]heptane-7-carboxamide;
*Trans*-*N*-(azepan-4-yl)-1-(8-cyano-5-quinolyl)-4-methyl-pyrrolidine-3-carboxamide;
*Trans-*5-[ 3-(2,7-diazaspiro[4.4]nonane-2-carbonyl)-4-methyl-pyrrolidin-1-yl]quinoline-8-carbonitrile;
(*3S,4S*)-1-(8-Cyano-5-quinolyl)-4-methyl-*N-*(1-methyl-4-piperidyl)pyrrolidine-3-carboxamide;
*Trans-*1-(8-cyano-5-quinolyl)-4-cyclopropyl-*N*-(2-morpholinoethyl)pyrrolidine-3-carboxamide;
*Trans*-1-(8-cyano-5-quinolyl)-*N-*[(*3S,4R*)-4-fluoropyrrolidin-3-yl]-4-methyl-pyrrolidine-3-carboxamide;
*N*-(Azepan-4-yl)-5-(8-cyano-5-quinolyl)-5-azaspiro[2.4]heptane-7-carboxamide;
(*3S,4S*)-1-(8-cyano-5-quinolyl)-*N*-(1-methyl-4-piperidyl)-4-(trifluoromethyl)pyrrolidine-3-carboxamide;
*N*-(2-Amino-2-methyl-propyl)-5-(8-cyano-5-quinolyl)-5-azaspiro[2.4]heptane-7-carboxamide;
(*3R*,*4R*)-1-(8-cyanoquinoxalin-5-yl)-4-methyl-*N*-[1-(4-piperidyl)-4-piperidyl]pyrrolidine-3-carboxamide;
*Trans-N*-(azepan-4-yl)-1-(8-cyano-5-quinolyl)-4-(difluoromethyl)pyrrolidine-3-carboxamide;
(*3S,4S*)-1-(8-Chloro-5-quinolyl)-4-methyl-*N*-(1-methyl-4-piperidyl)pyrrolidine-3-carboxamide;
5-(8-Cyano-5-quinolyl)-*N*-[(4-methylmorpholin-2-yl)methyl]-5-azaspiro[2,4]heptane-7-carboxamide;
1-(8-Cyano-5-quinolyl)-*N*-(1-methyl-4-piperidyl)pyrrolidine-3-carboxamide;
5-[7-(3-Aminoazetidine-1-carbonyl)-5-azaspiro[2.4]heptan-5-yl]quinoline-8-carbonitrile;
Methyl (*2R*,*4R*)-4-[[5-[8-(trifluoromethyl)quinoxalin-5-yl]-5-azaspiro[2.4]heptane-7-carbonyl]amino]pyrrolidine-2-carboxylate;
*Trans*-1-(8-cyano-5-quinolyl)-4-(difluoromethyl)-*N*-[(*3S,4R*)-4-fluoropyrrolidin-3-yl]pyrrolidine-3-carboxamide;
5-(8-Cyanoquinoxalin-5-yl)-*N*-[[(*2R*)-morpholin-2-yl]methyl]-5-azaspiro[2.4]heptane-7-carboxamide; and
5-(8-Cyanoquinoxalin-5-yl)-*N*-[[(*2S*)-morpholin-2-yl]methyl]-5-azaspiro[2.4]heptane-7-carboxamide;
or a pharmaceutically acceptable salt, enantiomer or diastereomer thereof.

### SYNTHESIS

The compounds of the present invention can be prepared by any conventional means. Suitable processes for synthesizing these compounds as well as their starting materials are provided in the schemes below and in the examples. All substituents, in particular, R¹ to R⁵ are as defined above unless otherwise indicated. Furthermore, and unless explicitly otherwise stated, all reactions, reaction conditions, abbreviations and symbols have the meanings well known to a person of ordinary skill in organic chemistry.

General synthetic route for preparing the compound of formula (I) is shown below. 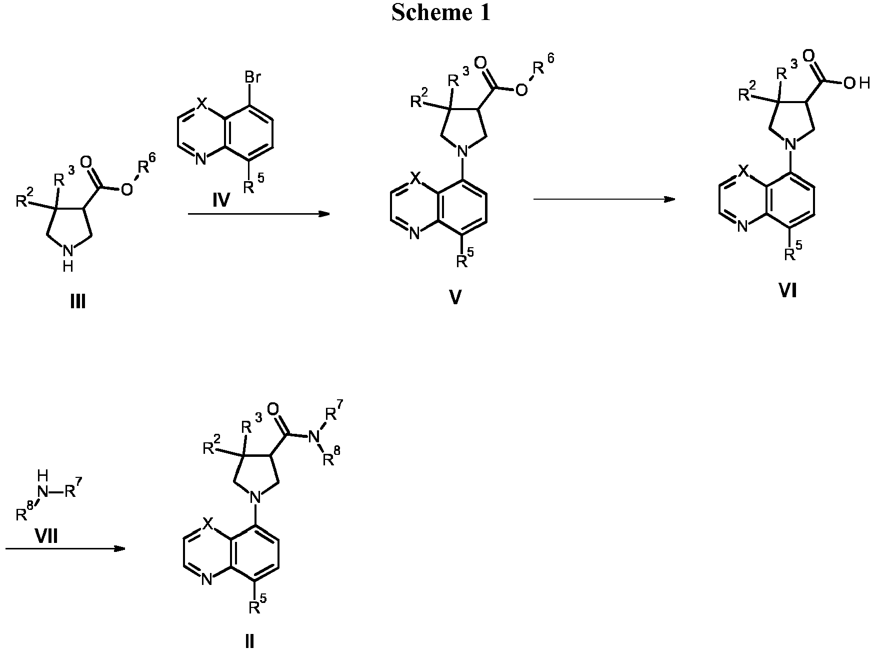 wherein R⁶ is C₁₋₆alkyl; R⁷ and R⁸ are independently selected from H, heterocyclyl, heterocyclylC₁₋₆alkyl, (C₁₋₆alkyl)₂aminoC₁₋₆alkyl, aminoC₃₋₇cycloalkyl, [(C₁₋₆alkyl)₂aminoC₁₋₆alkyl]C₃₋₇cycloalkyl, aminoC₃₋₇cycloalkylC₁₋₆alkyl or aminoC₁₋₆alkyl; or R⁷ and R⁸ together with the nitrogen they are attached to form heterocyclyl.

The coupling of halide (IV) with compound of formula (III) can be achieved by direct coupling in the presence of a base, such as DIPEA or K₂CO₃, or under Buchwald-Hartwig amination conditions (ref: Acc. Chem. Res. 1998, 31, 805-818; Chem. Rev. 2016, 116, 12564-12649; Topics in Current Chemistry, 2002, 219, 131-209; and references cited therein) with a catalyst, such as Ruphos Pd-G2, and a base, such as Cs₂CO₃, to provide compound of formula (V). Hydrolysis of compound of formula (V) in basic condition, such as LiOH in THF/water, gives carboxylic acid (VI), which is condensed with amine (VII) in the presence of a coupling reagent, such as HATU, to give the compound of formula (II). In some embodiment, the coupling of compound of formula (VI) and amine (VII) may give a product containing a protecting group, e.g. Boc, originated from amine (VII), which will be removed before affording the final compound of formula (II).

This invention also relates to a process for the preparation of a compound of formula (I) comprising the following step:
a) condensation of carboxylic acid (VI), 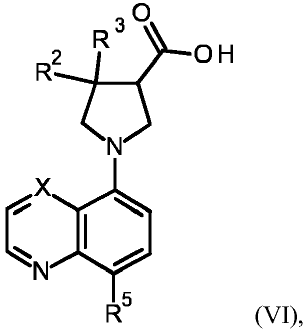
   with amine (VII) in the presence of a coupling reagent;
   wherein R², R³, R⁵, R⁶, R⁷, R⁸ and X are defined above.

In step a), the coupling reagent can be for example HATU.

A compound of formula (I) or (II) when manufactured according to the above process is also an object of the invention.

Compounds of this invention can be obtained as mixtures of diastereomers or enantiomers, which can be separated by methods well known in the art, e.g. (chiral) HPLC or SFC.

### INDICATIONS AND METHODS OF TREATMENT

The present invention provides compounds that can be used as TLR7 and/or TLR8 and/or TLR9 antagonist, which inhibits pathway activation through TLR7 and/or TLR8 and/or TLR9 as well as respective downstream biological events including, but not limited to, innate and adaptive immune responses mediated through the production of all types of cytokines and all forms of auto-antibodies. Accordingly, the compounds of the invention are useful for blocking TLR7 and/or TLR8 and/or TLR9 in all types of cells that express such receptor(s) including, but not limited to, plasmacytoid dendritic cell, B cell, T cell, macrophage, monocyte, neutrophil, keratinocyte, epithelial cell. As such, the compounds can be used as a therapeutic or prophylactic agent for systemic lupus erythematosus and lupus nephritis.

The present invention provides methods for treatment or prophylaxis of systemic lupus erythematosus and lupus nephritis in a patient in need thereof.

Another embodiment includes a compound of formula (I), a steroisomer, tautomer or pharmaceutically acceptable salt thereof for use in a method of treating or preventing systemic lupus erythematosus and lupus nephritis in a mammal in need of such treatment.

### EXAMPLES

The invention will be more fully understood by reference to the following examples. They should not, however, be construed as limiting the scope of the invention.

### ABBREVIATIONS

The invention will be more fully understood by reference to the following examples. They should not, however, be construed as limiting the scope of the invention.

Abbreviations used herein are as follows:

| | |
|---|---|
| ACN: | acetonitrile |
| Boc₂O: | di-tert-butyl dicarbonate |
| DCM: | dichloromethane |
| DIPEA or DIEA: | *N,N-*diisopropylethylamine |
| TEA | triethylamine |
| EA or EtOAc: | ethyl acetate |
| FA: | formic acid |
| HATU: | 1-[Bis(dimethylamino)methylene]-1H-1,2,3-triazolo[4,5-b]pyridinium 3-oxid hexafluorophosphate |
| IC₅₀: | half inhibition concentration |
| LCMS | liquid chromatography-mass spectrometry |
| MS: | mass spectrometry |
| PE: | petroleum ether |
| prep-HPLC: | preparative high performance liquid chromatography |
| rt: | room temperature |
| RT: | retention time |
| RuPhos Pd G2: | chloro(2-dicyclohexylphosphino-2',6'-diisopropoxy-1,1'-biphenyl)[2-(2'-amino-1,1'-biphenyl)]palladium(II) 2nd generation |
| SFC: | supercritical fluid chromatography |
| TFA: | trifluoroacetic acid |
| v/v | volume ratio |
| DDI | drug-drug-interaction |
| LYSA | lyophilisation solubility assay |
| HLM | human liver microsome |

### GENERAL EXPERIMENTAL CONDITIONS

Intermediates and final compounds were purified by flash chromatography using one of the following instruments: i) Biotage SP1 system and the Quad 12/25 Cartridge module, ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge^{™} Prep-C18 (5 µm, OBDTM 30 × 100 mm) column, SunFire^{™} Prep-C18 (5 µm, OBD^{™} 30 × 100 mm) column, Phenomenex Synergi-C18 (10 µm, 25 × 150 mm) or Phenomenex Gemini-C18 (10 µm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 µm, 30 × 250 mm), AS (10 µm, 30 × 250 mm) or AD (10 µm, 30 × 250 mm) using Mettler Toledo Multigram III system SFC, Waters 80Q preparative SFC or Thar 80 preparative SFC, solvent system: CO₂ and IPA (0.5% TEA in IPA) or CO₂ and MeOH (0.1% NH₃·H₂O in MeOH), back pressure 100bar, detection UV@ 254 or 220 nm.

LC/MS spectra of compounds were obtained using a LC/MS (Waters^{™} Alliance 2795-Micromass ZQ, Shimadzu Alliance 2020-Micromass ZQ or Agilent Alliance 6110-Micromass ZQ), LC/MS conditions were as follows (running time 3 or 1.5 mins):
Acidic condition I: A: 0.1% TFA in H₂O; B: 0.1% TFA in acetonitrile;
Acidic condition II: A: 0.0375% TFA in H₂O; B: 0.01875% TFA in acetonitrile;
Basic condition I: A: 0.1% NH₃·H₂O in H₂O; B: acetonitrile;
Basic condition II: A: 0.025% NH₃·H₂O in H₂O; B: acetonitrile;
Neutral condition: A: H₂O; B: acetonitrile.

Mass spectra (MS): generally only ions which indicate the parent mass are reported, and unless otherwise stated the mass ion quoted is the positive mass ion (MH)⁺.

NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

### PREPARATIVE EXAMPLES

The following examples are intended to illustrate the meaning of the present invention but should by no means represent a limitation within the meaning of the present invention:

### Intermediate A

### 5-Bromo-8-(trifluoromethyl)quinoxaline

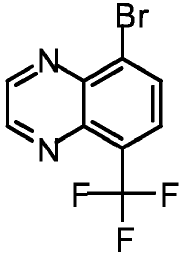

A detailed synthetic route is provided in the following Scheme

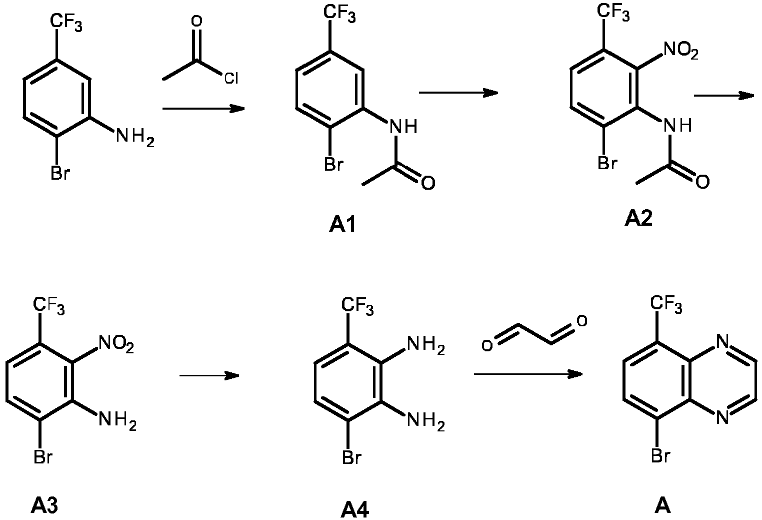

### Preparation of Compound A1

To a solution of 2-bromo-5-(trifluoromethyl)aniline (7.4 g, 30.0 mmol, 1 eq) and TEA (6.1 g, 60.0 mmol, 2 eq) in DCM (160 mL) was added acetyl chloride (7.0 g, 90.0 mmol, 3 eq) dropwise at 0 °C. The mixture was stirred at 20 °C for 2 hrs, then basified with NaHCO₃ and washed with water (100 mL). The aqueous layer was extracted with DCM (100 mL) twice. The combined organic layer was dried and concentrated under vacuum. The crude product was triturated in PE to give compound **A1** (6.2 g, 75.6%) as a white solid. MS: calc'd 282 (MH⁺), measured 282 (MH⁺).

### Preparation of Compound A2

H₂SO₄ (40 mL) was added slowly into a flask containing HNO₃ (25 mL) at 0 °C followed by the addition of compound **A1** (6.2 g, 22.1 mmol, 1 eq) in small portions over 40 mins. After the mixture was stirred at 0 °C for additional 20 mins, it was warmed to 25 °C and stirred for 1 hour, and extracted with EA (200 mL) twice. The combined organic layer was washed with sat. NaHCO₃ and brine (100 mL) twice, dried and concentrated under vacuum to give compound **A2** (8.1 g, crude) as a red solid. MS: calc'd 325 (MH⁺), measured 325 (MH⁺).

### Preparation of Compound A3

A solution of compound **A2** (8.1 g, 24.8 mmol, 1 eq) and HCl (6 M, 40 mL) in MeOH (160 mL) was stirred at 90 °C for 18 hrs. The mixture was basified with aq. NaHCO₃ and extracted with EA (200 mL) twice. The combined organic layer was washed with brine (100 mL) twice, dried and concentrated under vacuum to give the crude product, which was purified by column chromatography to give compound **A3** (1.9 g, 26.7%) as a yellow solid. MS: calc'd 285 (MH⁺), measured 285 (MH⁺).

### Preparation of Compound A4

A solution of compound **A3** (1.9 g, 6.7 mmol, 1 eq) and Fe (1.9 g, 33.5 mmol, 5 eq) in AcOH (60 mL) was stirred at 50 °C for 3 hours. The solid was removed by filtration and the filtrate was neutralized with sat. NaHCO₃ and extracted with EA (200 mL) twice. The combined organic layer was washed with brine (100 mL) twice, dried over Na₂SO₄, and concentrated to give a crude product which was purified by column chromatography (EA/PE = 1/10) to give compound **A4** (1.3 g, 76.5%) as a yellow solid. MS: calc'd 255 (MH⁺), measured 255 (MH⁺).

### Preparation of Intermediate A

A solution of compound **A4** (1.9 g, 4.7 mmol, 1 eq) and oxaldehyde (6.8 g, 47.0 mmol, 10 eq) in EtOH (30 mL) was stirred at 80 °C for 2 hrs. The mixture was neutralized with aq. NaHCO₃ (100 mL) and extracted with EA (200 mL) twice. The organic layers were combined and washed with brine (100 mL) twice. The organic layer was dried and concentrated under vacuum to give the crude product, which was purified by column chromatography (EA/PE = 1/8) to give Intermediate **A** (0.95 g, 67.8%) as a yellow solid. MS: calc'd 277 (MH⁺), measured 277 (MH⁺).

### Example 1

### (3R,4R)-1-(8-cyanoquinoxalin-5-yl)-N-(1-methyl-4-piperidyl)-4-(trifluoromethyl) pyrrolidine-3-carboxamide

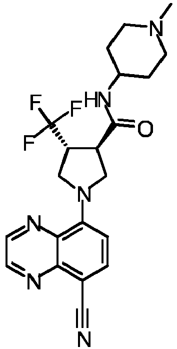

The title compound was prepared according to the following scheme:

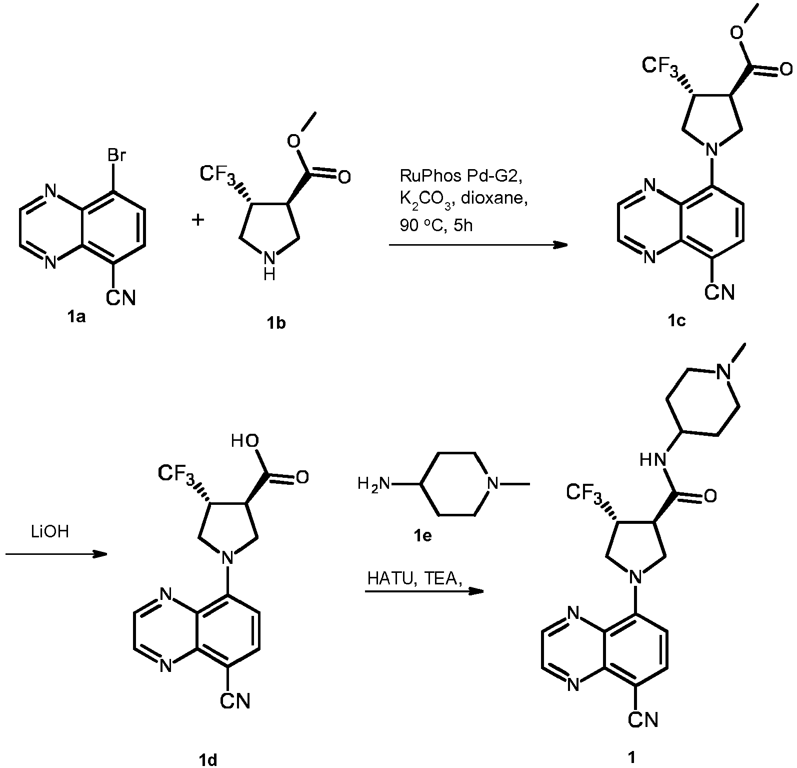

### Step 1: preparation of methyl (3R,4R)-1-(8-cyanoquinoxalin-5-yl)-4-(trifluoromethyl)pyrrolidine-3-carboxylate (compound 1c)

To a solution of methyl *(3R,4R*)-4-(trifluoromethyl)pyrrolidine-3-carboxylate HCl salt (compound **1b,** 22 mg, 94 µmol, Pharmablock, PBXA3229-1) and 8-bromoquinoxaline-5-carbonitrile (compound **1a**, 20 mg, 85.5 µmol) (Reference: WO2017/106607) in 1,4-dioxane (2 mL) was added K₂CO₃ (59 mg, 427 µmol). The mixture was degassed three times, then Ruphos Pd G2 (CAS: 1375325-68-0, 6.6 mg, 8.55 µmol) was added. After the reaction mixture was stirred at 90 °C for 5 hrs under N₂, it was cooled to rt, diluted with EA (50 mL) and washed with water. The organic layer was evaporated to give a crude product (30 mg) which was used in the next step without purification. MS: calc'd 351 (MH⁺), measured 351 (MH⁺).

### Step 2: preparation of (3R,4R)-1-(8-cyanoquinoxalin-5-yl)-4-(trifluoromethyl)pyrrolidine-3-carboxylic acid (compound 1d)

To a solution of *(3R,4R)*-methyl 1-(8-cyanoquinoxalin-5-yl)-4-(trifluoromethyl)pyrrolidine-3-carboxylate (compound **1c**, 30 mg, 85.6 µmol) in acetonitrile (10 mL) was added lithium hydroxide (20.5 mg, 856 µmol) in water (2 mL). After the mixture was stirred at rt for 4 hrs, the solvent was removed under vacuum to give a crude product. The crude product was dissolved in 10 mL H₂O and washed with EA. The aqueous layer was acidified to PH 4-5 by use of 1M HCl. The aqueous solution was extracted with 100 mL EA. The organic layer was separated, dried over Na₂SO₄ and evaporated under vacuum to give the crude product (28 mg) which was used in the next step without further purification. MS: calc'd 337 (MH⁺), measured 337 (MH⁺).

### Step 3: (3R,4R)-1-(8-cyanoquinoxalin-5-yl)-N-(1-methyl-4-piperidyl)-4-(trifluoromethyl) pyrrolidine-3-carboxamide (Example 1)

To a solution of *(3R,4R)*-1-(8-cyanoquinoxalin-5-yl)-4-(trifluoromethyl)pyrrolidine-3-carboxylic acid (compound **1d**, 28 mg, 83.3 µmol) and 1-methylpiperidin-4-amine (compound **1e**, 10 mg, 91.6 µmol, WuXi Apptec , CAS :41838-46-4) in DCM (10 mL) was added HATU (95 mg, 250 µmol) and triethylamine (84 mg, 833 µmol). The mixture was stirred at rt overnight, then diluted with DCM (50 mL) and washed with water (50 mL). The organic layer was evaporated under vacuum to give an oil, which was purified by prep-HPLC to give **Example 1** (30 mg) as a yellow solid. MS: calc'd 433 (MH⁺), measured 433 (MH⁺). ¹H NMR (400 MHz, METHANOL-d₄) *δ* = 8.88 (d, *J* = 1.7 Hz, 1H), 8.81 (d, *J* = 1.7 Hz, 1H), 8.02 (d, *J* = 8.7 Hz, 1H), 6.85 (d, *J=* 8.7 Hz, 1H), 4.45 - 4.21 (m, 3H), 4.10 - 3.99 (m, 2H), 3.69 - 3.53 (m, 3H), 3.24 - 3.08 (m, 2H), 2.91 (s, 3H), 2.29 - 2.02 (m, 3H), 1.85 - 1.66 (m, 2H).

### Example 2

### (3R,4R)-N-(1-methyl-4-piperidyl)-4-(trifluoromethyl)-1-[8-(trifluoromethyl)quinoxalin-5-yl]pyrrolidine-3-carboxamide

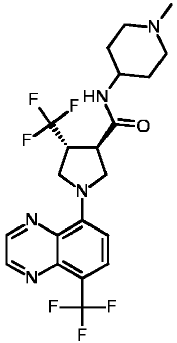

The title compound was prepared in analogy to the preparation of **Example 1** by using 5-bromo-8-(trifluoromethyl)quinoxaline (Intermediate **A**) instead of bromoquinoxaline-5-carbonitrile (compound **1a**). **Example 2** (40 mg) was obtained as a yellow solid. MS: calc'd 476 (MH⁺), measured 476 (MH⁺). ¹H NMR (400 MHz, METHANOL-d₄) *δ* = 8.74 (d, *J* = 1.7 Hz, 1H), 8.69 (d, *J* = 1.7 Hz, 1H), 7.86 (d, *J* = 8.7 Hz, 1H), 6.78 - 6.66 (m, 1H), 4.27 - 4.15 (m, 2H), 4.14 (d, *J* = 8.4 Hz, 2H), 3.96 - 3.82 (m, 2H), 3.56 - 3.38 (m,2H), 3.14 - 2.99 (m, 2H), 2.79 (s, 3H), 2.19 - 1.92 (m, 3H), 1.77 - 1.51 (m, 2H).

### Example 3

### (3R,4R)-1-(8-cyanoquinoxalin-5-yl)-4-methyl-N-(1-methyl-4-piperidyl)pyrrolidine-3-carboxamide

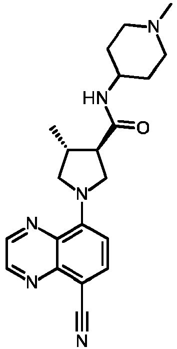

The title compound was prepared in analogy to the preparation of **Example 1** by using methyl *(3R,4R)*-4-methylpyrrolidine-3-carboxylate HCl salt (Pharmablock, PBXA3221-1) instead of methyl *(3R,4R*)-4-(trifluoromethyl)pyrrolidine-3-carboxylate HCl salt (compound **1b**). **Example 3** (18 mg) was obtained as a yellow solid. MS: calc'd 379 (MH⁺), measured 379 (MH⁺). ¹H NMR (400 MHz, METHANOL-d₄) *δ* = 8.82 (d, *J* = 1.7 Hz, 1H), 8.75 (d, *J* = 1.6 Hz, 1H), 7.95 (d, *J* = 8.7 Hz, 1H), 6.74 (d, *J* = 8.7 Hz, 1H), 4.28 - 4.12 (m, 3H), 4.07 - 3.93 (m, 1H), 3.66 - 3.45 (m, 3H), 3.25 - 3.11 (m, 2H), 2.91 (s, 3H), 2.77 - 2.53 (m, 2H), 2.31 - 2.04 (m, 2H), 1.86 - 1.70 (m, 2H), 1.24 - 1.15 (m, 3H).

### Example 4

### (3R,4R)-1-(8-cyano-5-quinolyl)-N-(1-methyl-4-piperidyl)-4-(trifluoromethyl)pyrrolidine-3-carboxamide

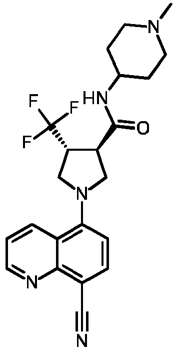

The title compound was prepared in analogy to the preparation of **Example 1** by using 5-bromoquinoline-8-carbonitrile instead of bromoquinoxaline-5-carbonitrile (compound **1a**). **Example 4** (62 mg) was obtained as a yellow solid. MS: calc'd 432 (MH⁺), measured 432 (MH⁺). 1H NMR (400 MHz, METHANOL-d4) δ = 8.72 (br dd, *J* = 1.5, 8.6 Hz, 1H), 8.66 - 8.53 (m, 1H), 8.09 (d, *J* = 8.2 Hz, 1H), 7.60 (dd, *J* = 4.2, 8.6 Hz, 1H), 7.12 - 6.99 (m, 1H), 4.08 - 3.81 (m, 5H), 4.05 - 3.45 (m, 3H), 3.23 - 3.06 (m, 2H), 2.90 (s, 3H), 2.33 - 2.02 (m, 3H), 1.87 - 1.62 (m, 2H).

### Example 5

### (3R,4R)-4-methyl-N-(1-methyl-4-piperidyl)-1-[8-(trifluoromethyl)quinoxalin-5-yl]pyrrolidine-3-carboxamide

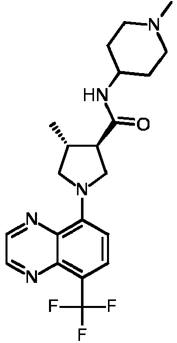

The title compound was prepared in analogy to the preparation of **Example 1** by using methyl *(3R,4R)*-4-methylpyrrolidine-3-carboxylate HCl salt (Pharmablock, PBXA3221-1) and 5-bromo-8-(trifluoromethyl)quinoxaline (Intermediate **A**) instead of methyl *(3R,4R)-*4-(trifluoromethyl)pyrrolidine-3-carboxylate HCl salt (compound **1b**) and bromoquinoxaline-5-carbonitrile (compound **1a**). **Example 5** (35 mg) was obtained as a yellow solid. MS: calc'd 422 (MH⁺), measured 422 (MH⁺). ¹H NMR (400 MHz, METHANOL-d₄) *δ* = 8.80 (d, *J* = 1.7 Hz, 1H), 8.75 (d, *J* = 1.7 Hz, 1H), 7.92 (d, *J* = 8.7 Hz, 1H), 6.78 - 6.68 (m, 1H), 4.22 - 3.94 (m, 4H), 3.64 - 3.50 (m, 3H), 3.17 (dt, *J* = 2.2, 13.0 Hz, 2H), 2.91 (s, 3H), 2.74 - 2.53 (m, 2H), 2.31 - 2.01 (m, 2H), 1.85 - 1.69 (m, 2H), 1.21 (d, *J* = 6.4 Hz, 3H).

### Example 6

### N-(3-oxa-9-azabicyclo[3.3.1]nonan-7-yl)-5-[8-(trifluoromethyl)quinoxalin-5-yl]-5-azaspiro[2.4]heptane-7-carboxamide

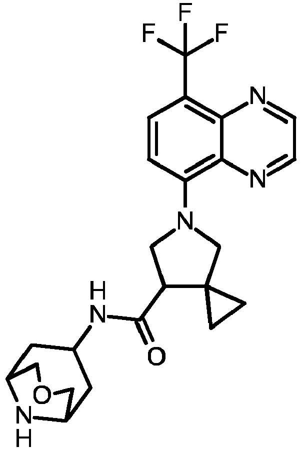

The title compound was prepared according to the following scheme:

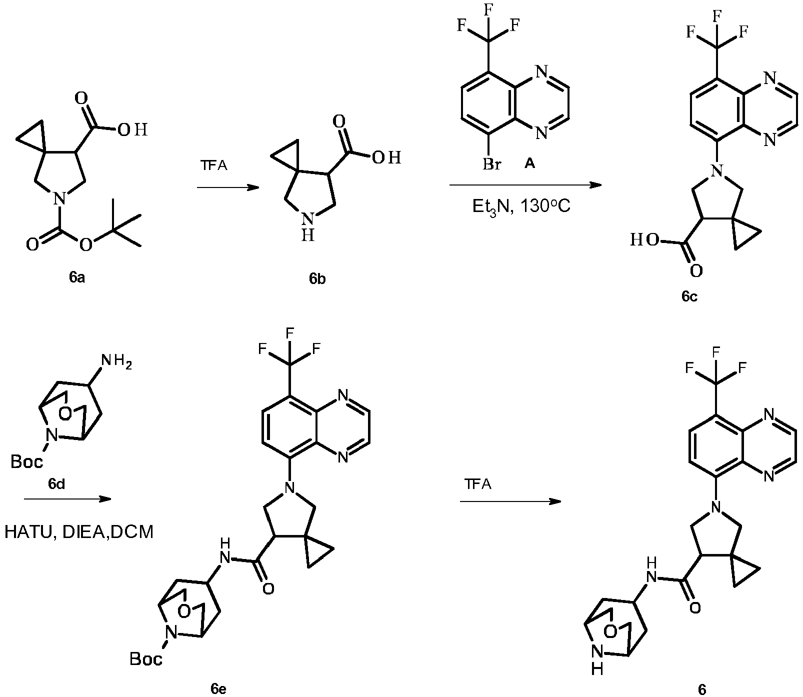

### Step 1: preparation of 5-azaspiro[2.4]heptane-7-carboxylic acid (compound 6b)

To a solution of 5-(*tert*-butoxycarbonyl)-5-azaspiro[2.4]heptane-7-carboxylic acid (compound **6a,** 0.5 g, 2.07 mmol, Pharmablock, PBLJ7032) in DCM (5 mL) was added 2,2,2-trifluoroacetic acid (1.65 g, 1.08 mL, 14.5 mmol). The reaction mixture was stirred at rt for 3 hrs, then concentrated *in vacuo* to give the crude product (282 mg) which was used in the next step without purification. MS: calc'd 142 (MH⁺), measured 142 (MH⁺).

### Step 2: preparation of 5-(8-(trifluoromethyl)quinoxalin-5-yl)-5-azaspiro[2.4]heptane-7-carboxylic acid (compound 6c)

A mixture of 5-bromo-8-(trifluoromethyl)quinoxaline ((Intermediate **A,** 554 mg, 2 mmol), 5-azaspiro[2.4]heptane-7-carboxylic acid (compound **6b,** 282 mg, 2 mmol) and TEA (1.01 g, 1.39 mL, 10 mmol) in DMSO (10 mL) was charged with argon. The mixture was heated with microwave at 130 °C for 3 hrs, then partitioned between EA and water. The separated aqueous layer was extracted with EA twice. The combined organic layer was dried over Na₂SO₄ and concentrated *in vacuo.* The residue was purified by silica gel column chromatography eluting with a gradient of PE:EA (100:10 to 100:50) to give 5-(8-(trifluoromethyl)quinoxalin-5-yl)-5-azaspiro[2.4]heptane-7-carboxylic acid (compound **6c,** 200 mg) as a yellow solid. MS: calc'd 338 (MH⁺), measured 338 (MH⁺).

### Step 3: preparation of tert-butyl 7-(5-(8-(trifluoromethyl)quinoxalin-5-yl)-5-azaspiro[2.4]heptane-7-carboxamido)-3-oxa-9-azabicyclo[3.3.1]nonane-9-carboxylate (compound 6e)

To a solution of 5-(8-(trifluoromethyl)quinoxalin-5-yl)-5-azaspiro[2.4]heptane-7-carboxylic acid (compound **6c,** 50 mg, 148 µmol), *tert*-butyl 7-amino-3-oxa-9-azabicyclo[3.3.1]nonane-9-carboxylate (compound **6d,** 72 mg, 296 µmol) and DIEA (38 mg, 51.8 µL, 296 µmol) in CH₂Cl₂ (2 mL) was added 2-(3H-[1,2,3]triazolo[4,5-b]pyridin-3-yl)-1,1,3,3-tetramethylisouronium hexafluorophosphate(V) (113 mg, 296 µmol). The reaction mixture was stirred at rt for 2 hrs. The mixture was partitioned between EA and Water. The separated aqueous layer was extracted twice with EA. The combined organic phase was dried over Na₂SO₄ and concentrated *in vacuo.* The residue was purified by silica gel chromatography on a 12 g column using a MPLC system (CombiFlash Companion, Isco Inc.) eluting with a gradient of PE:EA(100:10 to 100:50) to give *tert*-butyl 7-(5-(8-(trifluoromethyl)quinoxalin-5-yl)-5-azaspiro[2.4]heptane-7-carboxamido)-3-oxa-9-azabicyclo[3.3.1]nonane-9-carboxylate (compound **6e ,** 80 mg) as a yellow solid. MS: calc'd 562 (MH⁺), measured 562 (MH⁺).

### Step 4: preparation of N-(3-oxa-9-azabicyclo[3.3.1]nonan-7-yl)-5-[8-(trifluoromethyl)quinoxalin-5-yl]-5-azaspiro[2.4]heptane-7-carboxamide (Example 6)

To a solution of *tert*-butyl 7-(5-(8-(trifluoromethyl)quinoxalin-5-yl)-5-azaspiro[2.4]heptane-7-carboxamido)-3-oxa-9-azabicyclo[3.3.1]nonane-9-carboxylate (compound **6e,** 80 mg, 142 µmol) in CH₂Cl₂ (3 mL) was added 2,2,2-trifluoroacetic acid (49 mg, 427 µmol). The reaction mixture was stirred at rt for 3 hrs. Then the reaction mixture was concentrated *in vacuo.* The residue was purified by prep-HPLC to give **Example 6** (70 mg) as a yellow solid. MS: calc'd 462 (MH⁺), measured 462 (MH⁺). ¹H NMR (400 MHz, METHANOL-d4) δ 8.82 (d, *J* = 1.59 Hz, 1H), 8.77 (d, *J* = 1.71 Hz, 1H), 8.37 (d, *J* = 9.54 Hz, 1H), 6.79 (d, *J* = 8.68 Hz, 1H), 4.42-4.51 (m, 1H), 4.37 (dd, *J* = 1.65, 11.31 Hz, 1H), 4.20-4.31 (m, 2H), 3.95-4.02 (m, 1H), 3.84-3.93 (m, 3H), 3.51-3.62 (m, 3H), 2.65-2.73 (m, 1H), 2.43-2.58 (m, 2H), 1.96 (t, *J* = 15.53 Hz, 2H), 0.95-1.04 (m, 1H), 0.74-0.93 (m, 3H).

### Example 7

### 5-(8-Cyanoquinoxalin-5-yl)-N-(1-methyl-4-piperidyl)-5-azaspiro[2.4]heptane-7-carboxamide

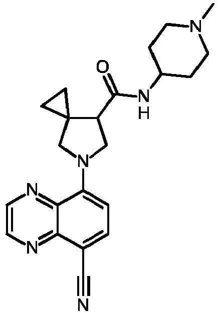

The title compound was prepared in analogy to the preparation of **Example 6** by using 8-bromoquinoxaline-5-carbonitrile and 1-methylpiperidin-4-amine instead of 5-bromo-8-(trifluoromethyl)quinoxaline (Intermediate **A**) and *tert*-butyl 7-amino-3-oxa-9-azabicyclo[3.3.1]nonane-9-carboxylate (compound **6d**). **Example 7** (200 mg) was obtained as a yellow solid. MS: calc'd 391(MH⁺), measured 391 (MH⁺). ¹H NMR (400 MHz, METHANOL-d₄) *δ* = 8.83 (d, *J* = 1.5 Hz, 1H), 8.75 (d, *J* = 1.5 Hz, 1H), 7.98 (d, *J* = 8.7 Hz, 1H), 6.76 (d, *J* = 8.8 Hz, 1H), 4.43 - 4.33 (m, 1H), 4.33 - 4.26 (m, 1H), 4.15 (d, *J* = 11.2 Hz, 1H), 3.99 - 3.86 (m, 1H), 3.77 (d, *J* = 11.3 Hz, 1H), 3.60 - 3.50 (m, 2H), 3.21 - 3.06 (m, 2H), 2.81 (s, 3H), 2.80 - 2.73 (m, 1H), 2.24 - 2.10 (m, 2H), 1.83 -1.67 (m, 2H), 0.91 - 0.75 (m, 4H).

SFC-HPLC separation gave two isomers **Example 7A** (17 mg) and **Example 7B** (20 mg). The compound names of the two isomers are (7R)-5-(8-Cyanoquinoxalin-5-yl)-*N*-(1-methyl-4-piperidyl)-5-azaspiro[2.4]heptane-7-carboxamide and (7S)-5-(8-Cyanoquinoxalin-5-yl)-*N*-(1-methyl-4-piperidyl)-5-azaspiro[2.4]heptane-7-carboxamide.

**Example 7A:** MS: calc'd 391 (MH⁺), measured 391 (MH⁺). ¹H NMR (400 MHz, METHANOL-d₄) *δ* = 8.81 (d, *J* = 1.5 Hz, 1H), 8.73 (d, *J* = 1.8 Hz, 1H), 7.96 (d, *J* = 8.8 Hz, 1H), 6.74 (d, *J* = 8.8 Hz, 1H), 4.39 - 4.31 (m, 1H), 4.31 - 4.24 (m, 1H), 4.17 - 4.08 (m, 1H), 3.94 - 3.84 (m, 1H), 3.79 - 3.70 (m, 1H), 3.53 (br d, *J* = 12.5 Hz, 2H), 3.16 - 3.04 (m, 2H), 2.85 (s, 3H), 2.74 (dd, *J* = 3.1, 6.9 Hz, 1H), 2.20 - 2.08 (m, 2H), 1.75 (q, *J* = 12.8 Hz, 2H), 0.86 - 0.73 (m, 4H).

**Example 7B:** MS: calc'd 391 (MH⁺), measured 391 (MH⁺). ¹H NMR (400 MHz, METHANOL-d₄) *δ* = 8.82 (d, *J* = 1.8 Hz, 1H), 8.74 (d, *J* = 1.8 Hz, 1H), 7.96 (d, *J* = 8.5 Hz, 1H), 6.75 (d, *J* = 8.8 Hz, 1H), 4.46 - 4.33 (m, 1H), 4.33 - 4.23 (m, 1H), 4.18 - 4.09 (m, 1H), 3.91 (tt, *J* = 4.0, 11.9 Hz, 1H), 3.78 - 3.71 (m, 1H), 3.55 (br d, *J* = 12.5 Hz, 2H), 3.16 - 3.05 (m, 2H), 2.86 (s, 3H), 2.77 (dd, *J* = 3.5, 7.0 Hz, 1H), 2.24 - 2.09 (m, 2H), 1.85 - 1.72 (m, 2H), 0.88 - 0.75 (m, 4H).

### Example 8

### N-(3-azabicyclo[3.3.1]nonan-9-yl)-5-[8-(trifluoromethyl)quinoxalin-5-yl]-5-azaspiro[2.4]heptane-7-carboxamide

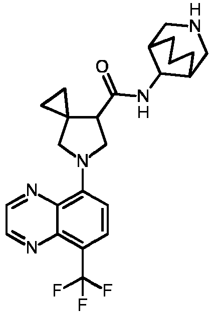

The title compound was prepared in analogy to the preparation of **Example 6** by using *tert*-butyl 9-amino-3-azabicyclo[3.3.1]nonane-3-carboxylate instead of *tert*-butyl 7-amino-3-oxa-9-azabicyclo[3.3.1]nonane-9-carboxylate (compound **6d**). **Example 8** (14 mg) was obtained as a yellow solid. MS: calc'd 460 (MH⁺), measured 460 (MH⁺). ¹H NMR (400 MHz, METHANOL-d₄) *δ* = 8.69 (d, *J* = 1.71 Hz, 1H), 8.64 (d, *J* = 1.71 Hz, 1H), 7.82 (d, *J* = 8.80 Hz, 1H), 6.64 (d, *J* = 8.68 Hz, 1H), 4.20-4.29 (m, 1H), 4.12-4.19 (m, 1H), 3.94-4.05 (m, 2H), 3.57 (d, *J* = 10.76 Hz, 1H), 3.35-3.42 (m, 2H), 3.26-3.33 (m, 2H), 2.82 (dd, *J* = 3.30, 6.85 Hz, 1H), 1.87-2.11 (m, 4H), 1.59-1.72 (m, 4H), 0.67-0.81 (m, 4H).

### Example 10

### N-(9-azabicyclo[3.3.1]nonan-3-yl)-5-[8-(trifluoromethyl)quinoxalin-5-yl]-5-azaspiro[2.4]heptane-7-carboxamide

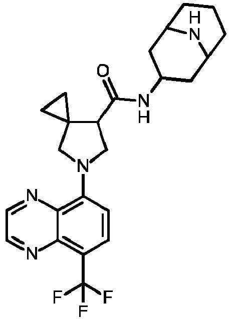

The title compound was prepared in analogy to the preparation of **Example 6** by using *tert*-butyl 3-amino-9-azabicyclo[3.3.1]nonane-9-carboxylate instead of *tert*-butyl 7-amino-3-oxa-9-azabicyclo[3.3.1]nonane-9-carboxylate (compound **6d**). **Example 10** (62 mg) was obtained as a yellow solid. MS: calc'd 460(MH⁺), measured 460(MH⁺). ¹H NMR (400 MHz, METHANOL-d₄) *δ* = 8.67-8.71 (m, 1H), 8.63 (d, *J* = 1.71 Hz, 1H), 7.81 (d, *J* = 8.68 Hz, 1H), 6.62 (d, *J* = 8.68 Hz, 1H), 4.58 (dt, *J* = 6.24, 12.23 Hz, 1H), 4.21-4.29 (m, 1H), 4.10-4.16 (m, 1H), 3.94-4.02 (m, 1H), 3.60 (s, 3H), 2.60-2.68 (m, 1H), 2.05-2.23 (m, 2H), 1.94 (m, 8H), 0.62-0.78 (m, 4H).

### Example 11

### N-(1-methyl-4-piperidyl)-5-[8-(trifluoromethyl)quinoxalin-5-yl]-5-azaspiro[2.4]heptane-7-carboxamide

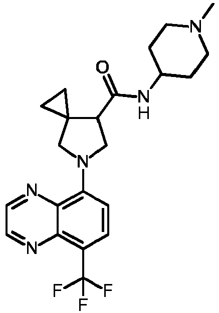

The title compound was prepared in analogy to the preparation of **Example 6** by using 1-methylpiperidin-4-amine instead of and *tert*-butyl 7-amino-3-oxa-9-azabicyclo[3.3.1]nonane-9-carboxylate (compound **6d**). **Example 11** (20 mg) was obtained as a yellow solid. MS: calc'd 434 (MH⁺), measured 434(MH⁺). ¹H NMR (400 MHz, METHANOL-d₄) *δ* = 8.92 (d, *J* = 1.71 Hz, 1H), 8.81 (d, *J* = 1.71 Hz, 1H), 7.84 (d, *J* = 7.70 Hz, 1H), 6.70 (d, *J* = 8.68 Hz, 1H), 4.13-4.22 (m, 1H), 4.06 (dd, *J* = 4.40, 11.37 Hz, 1H), 3.92 (d, *J* = 11.13 Hz, 1H), 3.71 (d, *J* = 10.64 Hz, 1H), 3.48 (s, 1H), 2.64-2.79 (m, 3H), 2.13 (s, 3H), 1.91 (m, 2H), 1.67 (m, 2H), 1.30-1.45 (m, 2H), 0.59-0.80 (m, 4H).

### Example 12

### N-(3-azabicyclo[3.2.1]octan-8-yl)-5-[8-(trifluoromethyl)quinoxalin-5-yl]-5-azaspiro[2.4]heptane-7-carboxamide

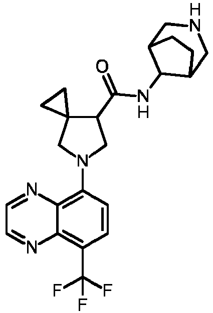

The title compound was prepared in analogy to the preparation of **Example 6** by using *tert*-butyl 8-amino-3-azabicyclo[3.2.1]octane-3-carboxylate instead of and *tert*-butyl 7-amino-3-oxa-9-azabicyclo[3.3.1]nonane-9-carboxylate (compound **6d**). **Example 12** (65 mg) was obtained as a yellow solid. MS: calc'd 446 (MH⁺), measured 446 (MH⁺). ¹H NMR (400 MHz, METHANOL-d₄) *δ* = 8.80 (d, *J* = 1.71 Hz, 1H), 8.74 (d, *J* = 1.59 Hz, 1H), 7.93 (d, *J* = 8.80 Hz, 1H), 6.74 (d, *J* = 8.80 Hz, 1H), 4.31-4.44 (m, 1H), 4.21-4.31 (m, 1H), 4.06-4.15 (m, 1H), 3.75-3.97 (m, 1H), 3.70 (d, *J* = 10.76 Hz, 1H), 3.36-3.42 (m, 2H), 3.21-3.28 (m, 1H), 3.01-3.13 (m, 1H), 2.79-2.96 (m, 1H), 2.37-2.60 (m, 2H), 2.00-2.19 (m, 2H), 1.76-1.89 (m, 2H), 0.74-0.94 (m, 4H).

### Example 13

### 5-(8-Cyanoquinoxalin-5-yl)-N-(morpholin-2-ylmethyl)-5-azaspiro[2.4]heptane-7-carboxamide

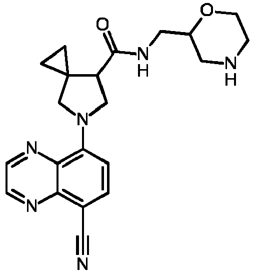

The title compound was prepared in analogy to the preparation of **Example 6** by using bromoquinoxaline-5-carbonitrile and *tert*-butyl 2-(aminomethyl)morpholine-4-carboxylate (CAS: 140645-53-0) instead of 5-bromo-8-(trifluoromethyl)quinoxaline (Intermediate **A**) and *tert*-butyl 7-amino-3-oxa-9-azabicyclo[3.3.1]nonane-9-carboxylate (compound **6d**). **Example 13** (44 mg) was obtained as a yellow solid. MS: calc'd 393(MH⁺), measured 393(MH⁺). ¹H NMR (400 MHz, METHANOL-d₄) *δ* = 8.72 (d, *J* = 1.83 Hz, 1H), 8.63 (d, *J* = 1.71 Hz, 1H), 7.86 (d, *J* = 8.68 Hz, 1H), 6.64 (d, *J* = 8.80 Hz, 1H), 4.15-4.30 (m, 2H), 3.96-4.11 (m, 2H), 3.57-3.73 (m, 3H), 3.30-3.41 (m, 1H), 3.23-3.29 (m, 1H), 3.12-3.18 (m, 2H), 2.95-3.07 (m, 1H), 2.72-2.82 (m, 1H), 2.64-2.72 (m, 1H), 0.76-0.85 (m, 1H), 0.66-0.76 (m, 3H).

### Example 14

### Trans-1-(8-cyano-5-quinolyl)-4-cyclopropyl-N-(1-methyl-4-piperidyl)pyrrolidine-3-carboxamide

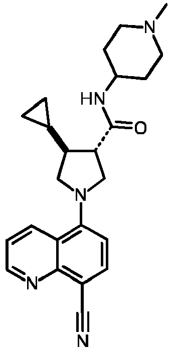

The title compound was prepared in analogy to the preparation of **Example** 1 by using *trans*-ethyl-4-cyclopropylpyrrolidine-3-carboxylate hydrochloride (Pharmablock, PBXA3214-1) and 5-bromoquinoline-8-carbonitrile instead of methyl *(3R,4R)*-4-(trifluoromethyl)pyrrolidine-3-carboxylate HCl salt (compound **1b**) and bromoquinoxaline-5-carbonitrile (compound **1a**). **Example 14** (8 mg) was obtained as a yellow solid. MS: calc'd 404 (MH⁺), measured 404 (MH⁺). ¹H NMR (400 MHz, METHANOL-d₄) *δ* =8.67 (dd, *J* = 1.4, 4.6 Hz, 1H), 8.29 (br d, *J* = 7.2 Hz, 1H), 7.87 - 7.76 (m, 1H), 7.45 - 7.30 (m, 1H), 6.73 - 6.54 (m, 1H), 3.95 - 3.48 (m, 5H), 3.35 (br d, *J* = 12.8 Hz, 2H), 3.00 - 2.87 (m, 3H), 2.86 - 2.68 (m, 2H), 2.13 - 1.81 (m, 3H), 1.74 - 1.46 (m, 3H), 0.70 - 0.52 (m, 1H), 0.32 (br d, *J* = 8.4 Hz, 2H), 0.06 -0.07 (m, 2H).

### Example 15

### N-(3-azabicyclo[3.3.1]nonan-7-yl)-5-[8-(trifluoromethyl)quinoxalin-5-yl]-5-azaspiro[2.4]heptane-7-carboxamide

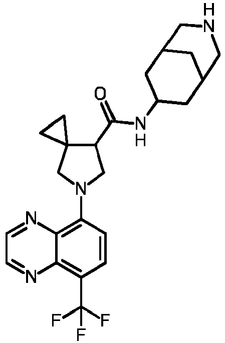

The title compound was prepared in analogy to the preparation of **Example 6** by using *tert*-butyl 7-amino-3-azabicyclo[3.3.1]nonane-3-carboxylate instead of *tert*-butyl 7-amino-3-oxa-9-azabicyclo[3.3.1]nonane-9-carboxylate (compound **6d**). **Example 15** (17 mg) was obtained as a yellow solid. MS: calc'd 460(MH⁺), measured 460(MH⁺). ¹H NMR (400 MHz, METHANOL-d₄) *δ* = 8.81 (d, *J* = 1.71 Hz, 1H), 8.75 (d, *J* = 1.71 Hz, 1H), 7.93 (d, *J* = 8.80 Hz, 1H), 6.74 (d, *J* = 8.80 Hz, 1H), 4.32-4.41 (m, 1H), 4.20-4.29 (m, 1H), 4.10 (d, *J* = 10.76 Hz, 1H), 3.89-4.00 (m, 1H), 3.72 (d, *J* = 10.76 Hz, 1H), 3.14-3.21 (m, 2H), 3.04-3.13 (m, 2H), 2.78 (dd, *J* = 3.79, 7.09 Hz, 1H), 2.34-2.49 (m, 4H), 1.97 (d, *J* = 13.45 Hz, 1H), 1.47 (d, *J* = 13.69 Hz, 1H), 1.30-1.41 (m, 2H), 0.76-0.91 (m, 4H).

### Example 16

### (3R,4R)-1-(8-cyano-5-quinolyl)-4-methyl-N-(1-methyl-4-piperidyl)pyrrolidine-3-carboxamide

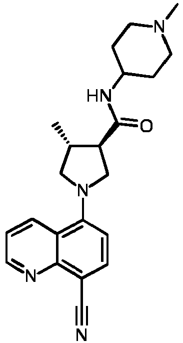

The title compound was prepared in analogy to the preparation of **Example 1** by using methyl *(3R,4R)*-4-methylpyrrolidine-3-carboxylate HCl salt (Pharmablock, PBXA3221-1) and 5-bromoquinoline-8-carbonitrile instead of methyl *(3R,4R)*-4-(trifluoromethyl)pyrrolidine-3-carboxylate HCl salt (compound **1b**) and bromoquinoxaline-5-carbonitrile (compound **1a**). **Example 16** (10 mg) was obtained as a yellow solid. MS: calc'd 378 (MH⁺), measured 378 (MH⁺). ¹H NMR (400 MHz, METHANOL-d₄) *δ* =8.86 (dd, *J* = 1.5, 4.3 Hz, 1H), 8.77 (dd, *J* = 1.5, 8.8 Hz, 1H), 7.95 (d, *J* = 8.6 Hz, 1H), 7.47 (dd, *J* = 4.2, 8.7 Hz, 1H), 6.78 (d, *J* = 8.6 Hz, 1H), 4.06 (t, *J* = 9.6 Hz, 1H), 3.88 - 3.69 (m, 2H), 3.53 (t, *J* = 9.8 Hz, 1H), 2.95 - 2.83 (m, 2H), 2.80 - 2.65 (m, 1H), 2.31 (s, 3H), 2.26 - 2.08 (m, 3H), 1.99 - 1.84 (m, 3H), 1.77 - 1.51 (m, 2H), 1.24 - 1.16 (m, 3H).

### Example 17

### Trans-1-(8-cyano-5-quinolyl)-N-(1-methyl-4-piperidyl)-4-(trifluoromethyl)pyrrolidine-3-carboxamide

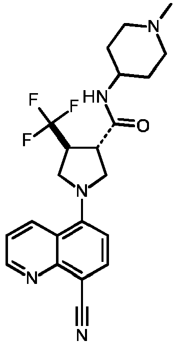

The title compound was prepared in analogy to the preparation of **Example 1** by using *trans*-methyl-4-(trifluoromethyl)pyrrolidine-3-carboxylate HCl salt (Pharmablock, PBXA3194-1) and 5-bromoquinoline-8-carbonitrile instead of methyl *(3R,4R)*-4-(trifluoromethyl)pyrrolidine-3-carboxylate HCl salt (compound **1b**) and bromoquinoxaline-5-carbonitrile (compound **1a**). **Example 17** (8 mg) was obtained as a yellow solid. MS: calc'd 432 (MH⁺), measured 432 (MH⁺). ¹H NMR (400 MHz, METHANOL-d₄) *δ* = 8.84 (dd, *J* = 1.5, 4.2 Hz, 1H), 8.58 (dd, *J* = 1.6, 8.7 Hz, 1H), 7.97 (d, *J* = 8.2 Hz, 1H), 7.48 (dd, *J* = 4.3, 8.7 Hz, 1H), 6.96 (d, *J* = 8.3 Hz, 1H), 3.81 - 3.62 (m, 3H), 3.59 - 3.48 (m, 1H), 3.43 - 3.35 (m, 2H), 2.89 (s, 3H), 2.38 - 2.20 (m, 2H), 2.16 - 2.05 (m, 1H), 1.91 - 1.81 (m, 3H), 1.65 - 1.45 (m, 3H).

### Example 18

### 1-(8-Cyano-5-quinolyl)-N-(1-methyl-4-piperidyl)-4-(trifluoromethyl)pyrrolidine-3-carboxamide

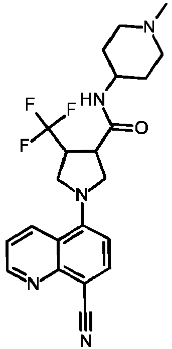

The title compound was prepared in analogy to the preparation of **Example 1** by using methyl 4-(trifluoromethyl)pyrrolidine-3-carboxylate and 5-bromoquinoline-8-carbonitrile instead of methyl *(3R,4R*)-4-(trifluoromethyl)pyrrolidine-3-carboxylate HCl salt (compound **1b**) and bromoquinoxaline-5-carbonitrile (compound **1a**). **Example 18** (6 mg) was obtained as a yellow solid. MS: calc'd 432 (MH⁺), measured 432 (MH⁺). ¹H NMR (400 MHz, METHANOL-d₄) *δ* = 8.96 (dd, *J* = 1.6, 4.3 Hz, 1H), 8.74 - 8.67 (m, 1H), 8.09 (d, *J* = 8.3 Hz, 1H), 7.64 - 7.54 (m, 1H), 7.16 - 7.03 (m, 1H), 4.11 - 3.93 (m, 1H), 3.98 - 3.52 (m, 7H), 3.25 - 3.09 (m, 2H), 2.97 - 2.85 (m, 3H), 2.35 - 2.02 (m, 3H), 1.84 - 1.61 (m, 2H).

### Example 19

### Trans-1-(8-cyano-5-quinolyl)-4-cyclopropyl-N-[2-(dimethylamino)ethyl]pyrrolidine-3-carboxamide

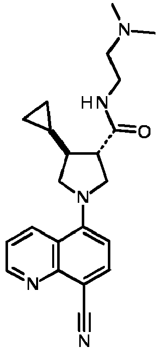

The title compound was prepared in analogy to the preparation of **Example 1** by using *trans*-ethyl-4-cyclopropylpyrrolidine-3-carboxylate hydrochloride (Pharmablock, PBXA3214-1), 5-bromoquinoline-8-carbonitrile and *N',N'*-dimethylethane-1,2-diamine instead of methyl *(3R,4R)*-4-(trifluoromethyl)pyrrolidine-3-carboxylate HCl salt (compound **1b**), bromoquinoxaline-5-carbonitrile (compound **1a**) and 1-methylpiperidin-4-amine (compound **1e**). **Example 19** (20 mg) was obtained as a yellow solid. MS: calc'd 378 (MH⁺), measured 378 (MH⁺). ¹H NMR (400 MHz, METHANOL-d₄) *δ* =9.03 (dd, *J* = 1.4, 8.7 Hz, 1H), 8.90 (dd, *J* = 1.5, 4.6 Hz, 1H), 8.03 (d, *J* = 8.7 Hz, 1H), 7.61 (dd, *J* = 4.6, 8.8 Hz, 1H), 6.87 (d, *J* = 8.7 Hz, 1H), 4.15 - 4.01 (m, 1H), 3.98 - 3.85 (m, 2H), 3.83 - 3.62 (m, 2H), 3.58 - 3.41 (m, 1H), 3.11 - 2.89 (m, 9H), 1.97 - 1.84 (m, 1H), 0.94 - 0.79 (m, 1H), 0.62 - 0.50 (m, 2H), 0.24 (q, *J* = 4.4 Hz, 2H).

### Example 20

### N-(5-methyl-5-azaspiro[2.4]heptan-7-yl)-5-[8-(trifluoromethyl)quinoxalin-5-yl]-5-azaspiro[2.4]heptane-7-carboxamide

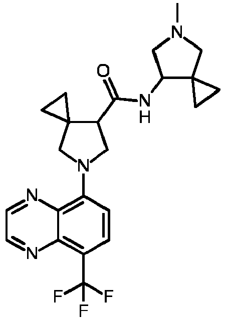

The title compound was prepared in analogy to the preparation of **Example 6** by using 5-methyl-5-azaspiro[2.4]heptan-7-amine instead of *tert*-butyl 7-amino-3-oxa-9-azabicyclo[3.3.1]nonane-9-carboxylate (compound **6d**). **Example 20** (47 mg) was obtained as a yellow solid. MS: calc'd 446 (MH⁺), measured 446 (MH⁺). ¹H NMR (400 MHz, METHANOL-d₄) *δ* = 8.69 (d, *J* = 1.59 Hz, 1H), 8.63 (s, 1H), 7.81 (d, *J* = 8.68 Hz, 1H), 6.61 (d, *J* = 8.68 Hz, 1H), 4.22-4.32 (m, 1H), 4.09-4.21 (m, 1H), 3.95-4.09 (m, 2H), 3.60-3.90 (m, 1H), 3.37-3.58 (m, 3H), 3.01-3.17 (m, 1H), 2.80-2.93 (m, 3H), 2.52-2.68 (m, 1H), 0.63-0.86 (m, 8H).

### Example 21

### N-(1,2,3,3a,4,5,6,6a-octahydrocyclopenta[c]pyrrol-5-yl)-5-[8-(trifluoromethyl)quinoxalin-5-yl]-5-azaspiro[2.4]heptane-7-carboxamide

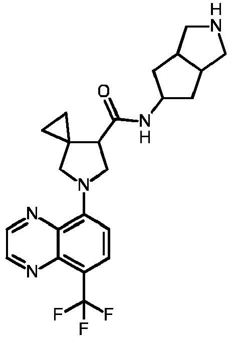

The title compound was prepared in analogy to the preparation of **Example 6** by using *tert*-butyl 5-amino-3,3a,4,5,6,6a-hexahydro-1H-cyclopenta[c]pyrrole-2-carboxylate instead of *tert*-butyl 7-amino-3-oxa-9-azabicyclo[3.3.1]nonane-9-carboxylate (compound **6d**). **Example 21** (25 mg) was obtained as a yellow solid. MS: calc'd 446 (MH⁺), measured 446 (MH⁺). ¹H NMR (400 MHz, METHANOL-d₄) *δ* = 8.80 (d, *J* = 1.83 Hz, 1H), 8.75 (d, *J* = 1.71 Hz, 1H), 7.93 (d, *J* = 8.68 Hz, 1H), 6.74 (d, *J* = 8.68 Hz, 1H), 4.31-4.41 (m, 1H), 4.20-4.26 (m, 1H), 4.03-4.12 (m, 2H), 3.73 (d, *J* = 10.76 Hz, 1H), 3.35-3.44 (m, 2H), 3.19 (td, *J* = 3.82, 11.92 Hz, 2H), 2.84-2.94 (m, 2H), 2.77 (dd, *J* = 3.85, 7.03 Hz, 1H), 2.30-2.46 (m, 2H), 1.26-1.39 (m, 2H), 0.73-0.91 (m, 4H).

### Example 22

### N-(6-aminospiro[3.3]heptan-2-yl)-5-[8-(trifluoromethyl)quinoxalin-5-yl]-5-azaspiro[2.4]heptane-7-carboxamide

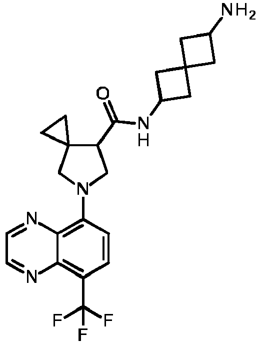

The title compound was prepared in analogy to the preparation of **Example 6** by using *tert*-butyl *N*-(6-aminospiro[3.3]heptan-2-yl)carbamate instead of *tert*-butyl 7-amino-3-oxa-9-azabicyclo[3.3.1]nonane-9-carboxylate (compound **6d**). **Example 22** (32 mg) was obtained as a yellow solid. MS: calc'd 446 (MH⁺), measured 446 (MH⁺). ¹H NMR (400 MHz, METHANOL-d₄) *δ* = 8.68 (d, *J* = 1.71 Hz, 1H), 8.62 (d, *J* = 1.71 Hz, 1H), 7.80 (d, *J* = 8.80 Hz, 1H), 6.61 (d, *J* = 8.80 Hz, 1H), 4.18-4.24 (m, 1H), 4.01-4.14 (m, 2H), 3.95 (d, *J* = 10.76 Hz, 1H), 3.51-3.63 (m, 2H), 2.53-2.64 (m, 1H), 2.36-2.47 (m, 2H), 2.19-2.28 (m, 2H), 1.99-2.12 (m, 2H), 1.91 (q, *J* = 8.40 Hz, 2H), 0.58-0.77 (m, 4H).

### Example 23

### N-(8-azabicyclo[3.2.1]octan-3-yl)-5-[8-(trifluoromethyl)quinoxalin-5-yl]-5-azaspiro[2.4]heptane-7-carboxamide

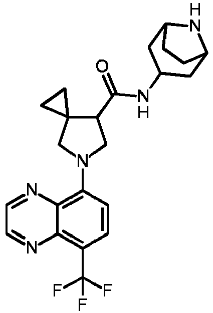

The title compound was prepared in analogy to the preparation of **Example 6** by using *tert*-butyl 3-amino-8-azabicyclo[3.2.1]octane-8-carboxylate instead of *tert*-butyl 7-amino-3-oxa-9-azabicyclo[3.3.1]nonane-9-carboxylate (compound **6d**). **Example 23** (27 mg) was obtained as a yellow solid. MS: calc'd 446 (MH⁺), measured 446 (MH⁺). ¹H NMR (400 MHz, METHANOL-d₄) *δ* = 8.68 (d, *J* = 1.71 Hz, 1H), 8.63 (d, *J* = 1.71 Hz, 1H), 7.81 (d, *J* = 8.68 Hz, 1H), 6.62 (d, *J* = 8.68 Hz, 1H), 4.18-4.29 (m, 1H), 4.02-4.16 (m, 2H), 3.91-4.02 (m, 3H), 3.59 (d, *J* = 10.76 Hz, 1H), 2.63 (dd, *J* = 3.67, 7.09 Hz, 1H), 1.91-2.06 (m, 6H), 1.58-1.74 (m, 2H), 0.60-0.75 (m, 4H).

### Example 24

### N-(4-aminocyclohexyl)-5-[8-(trifluoromethyl)quinoxalin-5-yl]-5-azaspiro[2.4]heptane-7-carboxamide

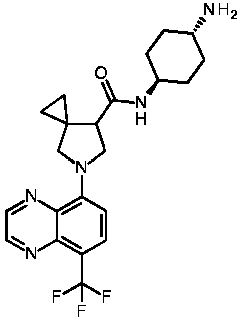

The title compound was prepared in analogy to the preparation of **Example 6** by using *tert*-butyl *N*-(4-aminocyclohexyl)carbamate instead of *tert*-butyl 7-amino-3-oxa-9-azabicyclo[3.3.1]nonane-9-carboxylate (compound **6d**). **Example 24** (6 mg) was obtained as a yellow solid. MS: calc'd 434 (MH⁺), measured 434 (MH⁺). ¹H NMR (400 MHz, METHANOL-d₄) *δ* = 8.68 (d, *J* = 1.83 Hz, 1H), 8.63 (d, *J* = 1.83 Hz, 1H), 7.81 (d, *J* = 8.68 Hz, 1H), 6.62 (d, *J* = 8.80 Hz, 1H), 4.19-4.29 (m, 1H), 4.08-4.16 (m, 1H), 3.96 (d, *J* = 10.64 Hz, 1H), 3.62 (d, *J* = 10.64 Hz, 1H), 3.55 (dd, *J* = 3.79, 7.70 Hz, 1H), 2.93-3.04 (m, 1H), 2.63 (dd, *J* = 3.79, 6.97 Hz, 1H), 1.85-2.03 (m, 4H), 1.34-1.45 (m, 2H), 1.18-1.33 (m, 2H), 0.61-0.74 (m, 4H).

### Example 25

### (3S,4S)-4-Methyl-N-(1-methyl-4-piperidyl)-1-[8-(trifluoromethyl)quinoxalin-5-yl]pyrrolidine-3-carboxamide

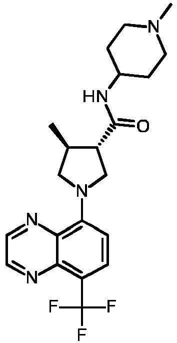

The title compound was prepared in analogy to the preparation of **Example 1** by using 5-bromo-8-(trifluoromethyl)quinoxaline and methyl (3S,4S)-4-methylpyrrolidine-3-carboxylate hydrochloride (Pharmablock, PBXA3220-1) instead of bromoquinoxaline-5-carbonitrile (compound **1a**) and methyl *(3R,4R*)-4-(trifluoromethyl)pyrrolidine-3-carboxylate HCl salt (compound **1b**). **Example 25** (8 mg) was obtained as a yellow solid. MS: calc'd 422 (MH⁺), measured 422 (MH⁺). ¹H NMR (400 MHz, METHANOL-d₄) *δ* = 8.86 - 8.72 (m, 2H), 7.98 - 7.86 (m, 1H), 6.74 (d, *J* = 8.7 Hz, 1H), 4.22 - 3.96 (m, 3H), 3.67 - 3.49 (m, 3H), 3.23 - 3.09 (m, 2H), 2.98 - 2.87 (m, 3H), 2.77 - 2.55 (m, 2H), 2.38 - 2.07 (m, 3H), 1.90 - 1.74 (m, 2H), 1.21 (d, *J* = 6.4 Hz, 3H).

### Example 26

### N-[3-[(dimethylamino)methyl]cyclobutyl]-5-[8-(trifluoromethyl)quinoxalin-5-yl]-5-azaspiro[2.4]heptane-7-carboxamide

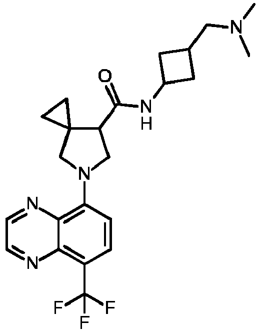

The title compound was prepared in analogy to the preparation of **Example 6** by using 3-[(dimethylamino)methyl]cyclobutanamine (CAS:1479049-07-4) instead of *tert*-butyl 7-amino-3-oxa-9-azabicyclo[3.3.1]nonane-9-carboxylate (compound **6d**). **Example 26** (8 mg) was obtained as a yellow solid. MS: calc'd 448 (MH⁺), measured 448 (MH⁺). ¹H NMR (400 MHz, METHANOL-d₄) *δ* = 8.68 (d, *J* = 1.71 Hz, 1H), 8.63 (d, *J* = 1.59 Hz, 1H), 7.81 (d, *J* = 8.68 Hz, 1H), 6.61 (d, *J* = 8.68 Hz, 1H), 4.17-4.28 (m, 2H), 4.06-4.14 (m, 1H), 3.96 (d, *J* = 10.76 Hz, 1H), 3.57-3.66 (m, 1H), 2.84-3.02 (m, 2H), 2.38-2.69 (m, 9H), 2.11 (dt, *J* = 3.85, 7.67 Hz, 2H), 1.64 (q, *J* = 9.82 Hz, 1H), 0.62-0.77 (m, 4H).

### Example 27

### (3R,4R)-4-Methyl-N-(1-methyl-4-piperidyl)-1-(8-nitro-5-quinolyl)pyrrolidine-3-carboxamide

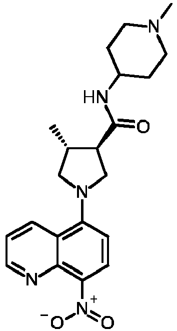

The title compound was prepared in analogy to the preparation of **Example 1** by using methyl *(3R,4R)*-4-methylpyrrolidine-3-carboxylate HCl salt (Pharmablock, PBXA3221-1) and 5-bromo-8-nitro-quinoline instead of methyl *(3R,4R*)-4-(trifluoromethyl)pyrrolidine-3-carboxylate HCl salt (compound **1b**) and bromoquinoxaline-5-carbonitrile (compound **1a**). **Example 27** (6 mg) was obtained as a yellow solid. MS: calc'd 398 (MH⁺), measured 398 (MH⁺). ¹H NMR (400 MHz, METHANOL-d₄) *δ* = 8.89 (dd, *J* = 1.5, 4.2 Hz, 1H), 8.80 (dd, *J* = 1.6, 8.8 Hz, 1H), 8.27 (d, *J* = 8.9 Hz, 1H), 7.51 (dd, *J* = 4.3, 8.8 Hz, 1H), 6.78 (d, *J* = 9.0 Hz, 1H), 4.09 (t, *J* = 9.6 Hz, 1H), 4.01 - 3.92 (m, 1H), 3.84 (ddd, *J* = 7.3, 9.9, 19.3 Hz, 2H), 3.61 - 3.46 (m, 1H), 3.13 - 2.88 (m, 3H), 2.79 - 2.69 (m, 3H), 2.41 (m, 1H), 2.33 - 1.99 (m, 3H), 1.84 - 1.63 (m, 2H), 1.37 (t, *J* = 7.3 Hz, 1H), 1.22 (d, *J* = 6.5 Hz, 3H).

### Example 28

### Trans-1-(8-cyano-5-quinolyl)-4-methyl-N-(1-methyl-4-piperidyl)pyrrolidine-3-carboxamide

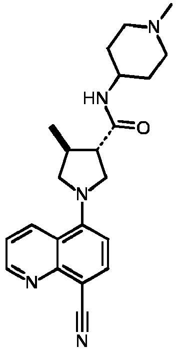

The title compound was prepared in analogy to the preparation of **Example 1** by using *trans*-methyl-4-methylpyrrolidine-3-carboxylate (Bepharm, B162777) and 5-bromoquinoline-8-carbonitrile instead of methyl *(3R,4R*)-4-(trifluoromethyl)pyrrolidine-3-carboxylate HCl salt (compound **1b**) and bromoquinoxaline-5-carbonitrile (compound 1a). **Example 28** (5 mg) was obtained as a yellow solid. MS: calc'd 378 (MH⁺), measured 378 (MH⁺). ¹H NMR (400 MHz, METHANOL-d₄) *δ* = 8.88 (dd, *J* = 1.5, 4.2 Hz, 1H), 8.79 (dd, *J* = 1.5, 8.7 Hz, 1H), 7.98 (d, *J* = 8.6 Hz, 1H), 7.49 (dd, *J* = 4.2, 8.7 Hz, 1H), 6.82 (d, *J* = 8.6 Hz, 1H), 4.07 (t, *J* = 9.5 Hz, 1H), 3.95 - 3.75 (m, 3H), 3.62 - 3.49 (m, 1H), 3.25 (br d, *J* = 12.5 Hz, 2H), 2.85 - 2.56 (m, 7H), 2.18 - 2.00 (m, 2H), 1.72 (br d, *J* = 12.3 Hz, 2H), 1.21 (d, *J* = 6.4 Hz, 3H).

### Example 29

### Trans-1-(8-cyano-5-quinolyl)-4-ethyl-N-(1-methyl-4-piperidyl)pyrrolidine-3-carboxamide

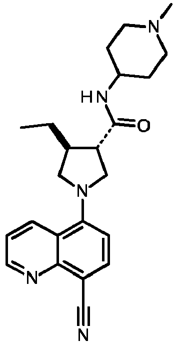

The title compound was prepared in analogy to the preparation of **Example 1** by using 5-bromoquinoline-8-carbonitrile and *trans*-ethyl-4-ethylpyrrolidine-3-carboxylate hydrochloride (Pharmablock, PBXA3209-1) instead of bromoquinoxaline-5-carbonitrile (compound **1a**) and methyl *(3R,4R*)-4-(trifluoromethyl)pyrrolidine-3-carboxylate HCl salt (compound **1b**).**Example 29** (43 mg) was obtained as a yellow solid. MS: calc'd 392 (MH⁺), measured 392 (MH⁺). 1H NMR (400 MHz, METHANOL-d4) δ = 8.84 - 8.71 (m, 2H), 7.88 (d, *J* = 8.6 Hz, 1H), 7.48 - 7.37 (m, 1H), 6.74 (d, *J* = 8.7 Hz, 1H), 3.95 - 3.83 (m, 2H), 3.81 - 3.68 (m, 2H), 3.53 - 3.42 (m, 3H), 3.11 - 2.98 (m, 2H), 2.84 - 2.76 (m, 3H), 2.74 - 2.63 (m, 1H), 2.47 - 2.38 (m, 1H), 2.11 (dt, *J* = 2.6, 10.0 Hz, 2H), 2.00 - 1.89 (m, 1H), 1.71 - 1.40 (m,3H), 0.93 (t, *J* = 7.5 Hz, 3H).

### Example 30

### N-(azepan-4-yl)-1-(8-cyano-5-quinolyl)-4-(trifluoromethyl)pyrrolidine-3-carboxamide

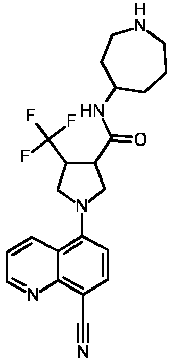

The title compound was prepared according to the scheme below.

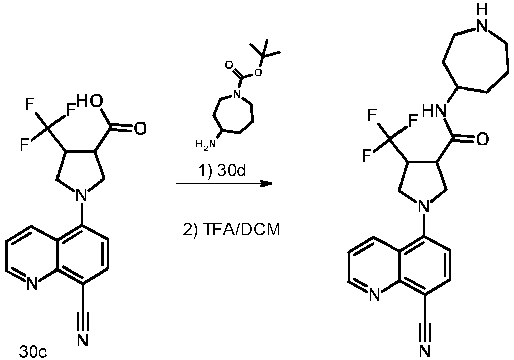

### Preparation of 1-(8-cyanoquinolin-5-yl)-4-(trifluoromethyl)pyrrolidine-3-carboxylic acid (Example 30c)

Compound 30c was prepared in analogy to the preparation of *(3R,4R)-*1-(8-cyanoquinoxalin-5-yl)-4-(trifluoromethyl)pyrrolidine-3-carboxylic acid (compound **1d**) of **Example 1** by using methyl 4-(trifluoromethyl)pyrrolidine-3-carboxylate and 5-bromoquinoline-8-carbonitrile instead of methyl *(3R,4R*)-4-(trifluoromethyl)pyrrolidine-3-carboxylate HCl salt (compound **1b**) and bromoquinoxaline-5-carbonitrile (compound **1a**).

### Preparation of N-(azepan-4-yl)-1-(8-cyano-5-quinolyl)-4-(trifluoromethyl)pyrrolidine-3-carboxamide (Example 30)

To a solution of 1-(8-cyanoquinolin-5-yl)-4-(trifluoromethyl)pyrrolidine-3-carboxylic acid (compound **30c,** 15 mg, 44.7 µmol) and *tert*-butyl 4-aminoazepane-1-carboxylate (compound **30d,** 9.6 mg, 44.7 µmol) in DCM (10 mL) was added triethylamine (22.6 mg, 224 µmol) and HATU (51 mg, 134 µmol). After the mixture was stirred at rt for 12 hrs, the solvent was removed under vacuum. The residue was then dissolved in DCM (2.0 mL), to which was added TFA (0.5 mL). After the mixture was stirred at rt for 2 hrs, the reaction mixture was concentrated to give a crude product which was purified by prep-HPLC to afford two isomers: **Example 30A** (5 mg) and **Example 30B** (5 mg).

**Example 30A:** MS: calc'd 432 (MH⁺), measured 432 (MH⁺). ¹H NMR (400 MHz, METHANOL-d₄) *δ* = 8.70 (dd, *J* = 1.5, 8.7 Hz, 1H), 8.59 (br d, *J* = 7.3 Hz, 1H), 8.09 (d, *J* = 8.2 Hz, 1H), 7.60 (dd, *J* = 4.2, 8.7 Hz, 1H), 7.08 (d, *J* = 8.2 Hz, 1H), 4.09 - 3.97 (m, 1H), 3.93 - 3.72 (m, 4H), 3.65 (td, *J* = 8.2, 16.4 Hz, 1H), 3.29 - 3.12 (m, 4H), 2.29 - 1.59 (m, 7H).

**Example 30B:** MS: calc'd 432 (MH⁺), measured 432 (MH⁺). ¹H NMR (400 MHz, METHANOL-d₄) *δ* = 8.70 (dd, *J* = 1.5, 8.7 Hz, 1H), 8.58 (br d, *J* = 7.3 Hz, 1H), 8.08 (d, *J* = 8.2 Hz, 1H), 7.60 (dd, *J* = 4.3, 8.7 Hz, 1H), 7.08 (d, *J* = 8.3 Hz, 1H), 4.09 - 3.96 (m, 1H), 3.93 - 3.60 (m, 5H), 3.30 - 3.14 (m, 4H), 2.25 - 1.57 (m, 7H).

### Example 31

### N-[(3-aminocyclobutyl)methyl]-5-[8-(trifluoromethyl)quinoxalin-5-yl]-5-azaspiro[2.4]heptane-7-carboxamide

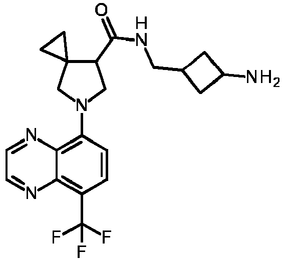

The title compound was prepared in analogy to the preparation of **Example 6** by using *tert*-butyl *N-*[3-(aminomethyl)cyclobutyl]carbamate instead of *tert*-butyl 7-amino-3-oxa-9-azabicyclo[3.3.1]nonane-9-carboxylate (compound **6d**). **Example 31** (33 mg) was obtained as a yellow solid. MS: calc'd 420 (MH⁺), measured 420 (MH⁺). ¹H NMR (400 MHz, METHANOL-d₄) *δ* = 8.68 (d, *J* = 1.59 Hz, 1H), 8.62 (d, *J* = 1.71 Hz, 1H), 7.80 (d, *J* = 8.68 Hz, 1H), 6.61 (d, *J* = 8.68 Hz, 1H), 4.16-4.28 (m, 1H), 4.10-4.16 (m, 1H), 3.93-4.02 (m, 1H), 3.65-3.76 (m, 1H), 3.47-3.63 (m, 1H), 3.24-3.31 (m, 1H), 3.05-3.16 (m, 1H), 2.64 (dd, *J* = 3.79, 6.97 Hz, 1H), 2.28-2.54 (m, 2H), 2.04-2.26 (m, 2H), 1.64-1.78 (m, 1H), 0.59-0.77 (m, 4H).

### Example 32

### Trans-5-[3-(3,9-diazaspiro[5.5]undecane-3-carbonyl)-4-methyl-pyrrolidin-1-yl]quinoline-8-carbonitrile

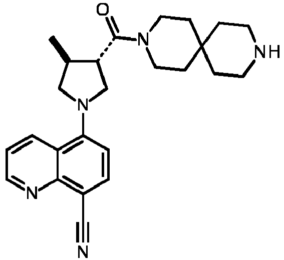

The title compound was prepared in analogy to the preparation of **Example 30** by using *trans*-methyl-4-methylpyrrolidine-3-carboxylate and *tert*-butyl 3,9-diazaspiro[5.5]undecane-3-carboxylate instead of methyl 4-(trifluoromethyl)pyrrolidine-3-carboxylate HCl salt and *tert-*butyl 4-aminoazepane-1-carboxylate (compound **30d**). **Example 32** (10 mg) was obtained as a yellow solid. MS: calc'd 418 (MH⁺), measured 418 (MH⁺). ¹H NMR (400 MHz, METHANOL-d₄) *δ* = 8.90 - 8.76 (m, 2H), 7.98 (d, *J* = 8.4 Hz, 1H), 7.50 (dd, *J* = 4.3, 8.8 Hz, 1H), 6.82 (d, *J* = 8.6 Hz, 1H), 4.00 - 3.79 (m, 3H), 3.69 (br t, *J* = 5.7 Hz, 4H), 3.60 - 3.48 (m, 1H), 3.22 (br s, 5H), 2.80 - 2.67 (m, 1H), 1.87 - 1.74 (m, 4H), 1.71 - 1.56 (m, 4H), 1.22 (d, *J* = 6.6 Hz, 3H).

### Example 33

### (3R,4R)-4-Methyl-N-(1-methyl-4-piperidyl)-1-(8-methylquinoxalin-5-yl)pyrrolidine-3-carboxamide

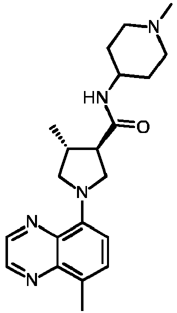

The title compound was prepared in analogy to the preparation of **Example 1** by using methyl *(3R,4R)*-4-methylpyrrolidine-3-carboxylate HCl salt (Pharmablock, PBXA3221-1) and 5-bromo-8-methyl-quinoline instead of methyl *(3R,4R)*-4-(trifluoromethyl)pyrrolidine-3-carboxylate HCl salt (compound **1b**) and bromoquinoxaline-5-carbonitrile (compound **1a**). **Example 33** (10 mg) was obtained as a yellow solid. MS: calc'd 368 (MH⁺), measured 368 (MH⁺). ¹H NMR (400 MHz, METHANOL-d₄) *δ* = 9.13 - 8.99 (m, 1H), 9.03 - 8.92 (m, 1H), 7.82 (q, *J* = 8.0 Hz, 2H), 4.35 - 4.22 (m, 1H), 4.19 - 3.97 (m, 3H), 3.77 - 3.52 (m, 3H), 3.26 - 3.00 (m, 4H), 2.97 - 2.75 (m, 7H), 2.32 - 2.15 (m, 2H), 1.88 - 1.69 (m, 2H), 1.35 - 1.27 (m, 3H).

### Example 34

### N-(7-azaspiro[3.5]nonan-2-yl)-5-[8-(trifluoromethyl)quinoxalin-5-yl]-5-azaspiro[2.4]heptane-7-carboxamide

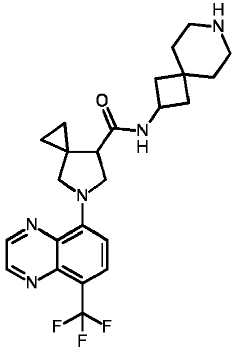

The title compound was prepared in analogy to the preparation of **Example 6** by using *tert*-butyl 2-amino-7-azaspiro[3.5]nonane-7-carboxylate instead of *tert*-butyl 7-amino-3-oxa-9-azabicyclo[3.3.1]nonane-9-carboxylate (compound **6d**). **Example 34** (26 mg) was obtained as a yellow solid. MS: calc'd 460 (MH⁺), measured 460 (MH⁺). ¹H NMR (400 MHz, METHANOL-d₄) *δ* = 8.68 (d, *J* = 1.71 Hz, 1H), 8.63 (d, *J* = 1.71 Hz, 1H), 7.80 (d, *J* = 8.68 Hz, 1H), 6.61 (d, *J* = 8.68 Hz, 1H), 4.07-4.26 (m, 3H), 3.96 (d, *J* = 10.76 Hz, 1H), 3.60 (d, *J* = 10.76 Hz, 1H), 3.01-3.07 (m, 2H), 2.94-3.01 (m, 2H), 2.62 (dd, *J* = 3.91, 7.09 Hz, 1H), 2.17-2.31 (m, 2H), 1.65-1.80 (m, 6H), 0.63-0.76 (m, 4H).

### Example 35

### (3R,4R)-1-(8-Cyanoquinoxalin-5-yl)-N-[1-(4-piperidyl)-4-piperidyl]-4-(trifluoromethyl)pyrrolidine-3-carboxamide

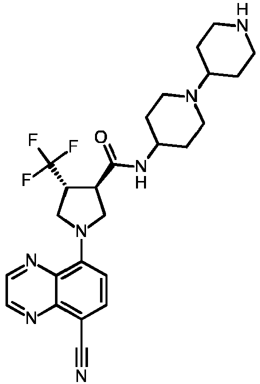

The title compound was prepared in analogy to the preparation of **Example 30** by using *tert*-butyl 4-(4-amino-1-piperidyl)piperidine-1-carboxylate instead of *tert*-butyl 4-aminoazepane-1-carboxylate (compound **30d**). **Example 35** (15 mg) was obtained as a yellow solid. MS: calc'd 502 (MH⁺), measured 502 (MH⁺). ¹H NMR (400 MHz, METHANOL-d₄) *δ* = 8.89 (d, *J* = 1.7 Hz, 1H), 8.83 (d, *J* = 1.8 Hz, 1H), 8.04 (d, *J* = 8.7 Hz, 1H), 6.87 (d, *J* = 8.8 Hz, 1H), 4.46 - 4.20 (m, 2H), 4.13 - 3.99 (m, 1H), 3.81 - 3.52 (m, 2H), 3.24 - 3.11 (m, 3H), 3.00 (br d, *J* = 11.7 Hz, 3H), 2.76 - 2.60 (m, 2H), 2.57 - 2.31 (m, 3H), 1.94 (br d, *J* = 11.7 Hz, 4H), 1.66 - 1.43 (m, 4H).

### Example 36

### N-[ (1R,2S,4R,5S)-4-amino-2-bicyclo[3.1.0]hexanyl]-5-[8-(trifluoromethyl)quinoxalin-5-yl]-5-azaspiro[2.4]heptane-7-carboxamide

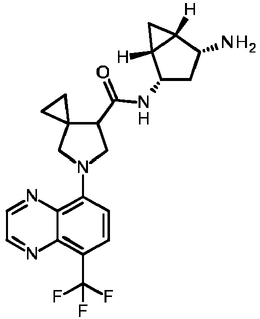

The title compound was prepared in analogy to the preparation of **Example 6** by using *tert-*butyl *N-*[(*1S,2R,4S,5R*)*-*4-amino-2-bicyclo[3.1.0]hexanyl]carbamate instead of *tert*-butyl 7-amino-3-oxa-9-azabicyclo[3.3.1]nonane-9-carboxylate (compound **6d**). **Example 36** (34 mg) was obtained as a yellow solid. MS: calc'd 432 (MH⁺), measured 432 (MH⁺). ¹H NMR (400 MHz, METHANOL-d₄) *δ* = 9.02 (d, *J* = 1.71 Hz, 1H), 8.96 (s, 1H), 8.14 (d, *J* = 8.68 Hz, 1H), 6.95 (d, *J* = 8.68 Hz, 1H), 4.70 (m, 1H), 4.52-4.61 (m, 1H), 4.41-4.51 (m, 1H), 4.30 (dd, *J* = 4.28, 10.76 Hz, 1H), 4.01-4.11 (m, 1H), 3.94 (dd, *J* = 2.81, 10.76 Hz, 1H), 3.02 (dt, *J* = 3.91, 7.09 Hz, 1H), 2.48-2.66 (m, 1H), 1.95-2.07 (m, 1H), 1.86-1.95 (m, 1H), 1.24-1.35 (m, 1H), 0.96-1.19 (m, 5H), 0.84-0.94 (m, 1H).

### Example 37

### Trans-1-(8-cyano-5-quinolyl)-4-isopropyl-N-(1-methyl-4-piperidyl)pyrrolidine-3-carboxamide

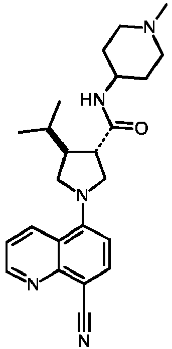

The title compound was prepared in analogy to the preparation of **Example 1** by using *trans*-methyl 4-isopropyl-pyrrolidine-3-carboxylate hydrochloride salt (CAS:1820575-33-4) and 5-bromoquinoline-8-carbonitrile instead of methyl *(3R,4R)*-4-(trifluoromethyl)pyrrolidine-3-carboxylate HCl salt (compound **1b**) and bromoquinoxaline-5-carbonitrile (compound 1a). **Example 37** (50 mg) was obtained as a yellow solid. MS: calc'd 406 (MH⁺), measured 406 (MH⁺). ¹H NMR (400 MHz, METHANOL-d₄) *δ* =8.93 - 8.85 (m, 2H), 8.00 (d, *J* = 8.6 Hz, 1H), 7.56 (dd, *J* = 4.5, 8.7 Hz, 1H), 6.87 (d, *J* = 8.7 Hz, 1H), 4.05 - 3.88 (m, 2H), 3.90 - 3.74 (m, 2H), 3.71 - 3.50 (m, 3H), 3.25 - 3.09 (m, 2H), 2.97 - 2.85 (m, 4H), 2.62 - 2.49 (m, 1H), 2.30 - 2.04 (m, 2H), 1.86 - 1.67 (m, 3H), 1.12 - 0.95 (m, 6H).

### Example 38

### N-(3-Aminocyclobutyl)-5-[8-(trifluoromethyl)quinoxalin-5-yl]-5-azaspiro[2.4]heptane-7-carboxamide

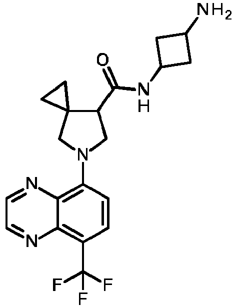

The title compound was prepared in analogy to the preparation of **Example 6** by using *tert*-butyl N-(3-aminocyclobutyl)carbamate instead of *tert*-butyl 7-amino-3-oxa-9-azabicyclo[3.3.1]nonane-9-carboxylate (compound **6d**). **Example 38** (25 mg) was obtained as a yellow solid. MS: calc'd 406 (MH⁺), measured 406 (MH⁺). ¹H NMR (400 MHz, METHANOL-d₄) *δ* = 8.68 (d, *J* = 1.71 Hz, 1H), 8.62 (d, *J* = 1.71 Hz, 1H), 7.80 (d, *J* = 8.68 Hz, 1H), 6.61 (d, *J* = 8.68 Hz, 1H), 4.30-4.42 (m, 1H), 4.19-4.29 (m, 1H), 4.04-4.14 (m, 1H), 3.88-4.03 (m, 1H), 3.72-3.81 (m, 1H), 3.53-3.63 (m, 1H), 2.58-2.71 (m, 2H), 2.27-2.47 (m, 3H), 0.63-0.81 (m, 4H).

### Example 39

### N-(4-Aminocyclohexyl)-5-[8-(trifluoromethyl)quinoxalin-5-yl]-5-azaspiro[2.4]heptane-7-carboxamide

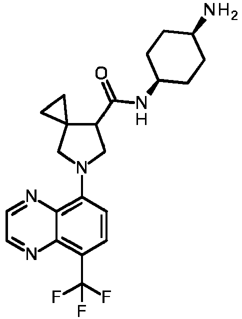

The title compound was prepared in analogy to the preparation of **Example 6** by using *tert*-butyl N-(4-aminocyclohexyl)carbamate instead of *tert*-butyl 7-amino-3-oxa-9-azabicyclo[3.3.1]nonane-9-carboxylate (compound **6d**). **Example 39** (33 mg) was obtained as a yellow solid. MS: calc'd 434 (MH⁺), measured 434 (MH⁺). ¹H NMR (400 MHz, METHANOL-d₄) *δ* = 8.69 (d, *J* = 1.71 Hz, 1H), 8.63 (d, *J* = 1.71 Hz, 1H), 7.81 (d, *J* = 8.68 Hz, 1H), 6.63 (d, *J* = 8.68 Hz, 1H), 4.19-4.27 (m, 1H), 4.10-4.18 (m, 1H), 3.97 (d, *J* = 10.76 Hz, 1H), 3.76 (s, 1H), 3.62 (d, *J* = 10.76 Hz, 1H), 3.12 (s, 1H), 2.75 (dd, *J* = 3.97, 7.03 Hz, 1H), 1.69-1.84 (m, 4H), 1.53-1.69 (m, 4H), 0.63-0.80 (m, 4H).

### Example 40

### Trans-1-(8-cyano-5-quinolyl)-4-(difluoromethyl)-N-(1-methyl-4-piperidyl)pyrrolidine-3-carboxamide

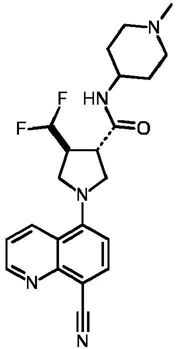

The title compound was prepared in analogy to the preparation of **Example 1** by using 5-bromoquinoline-8-carbonitrile and *trans*-ethyl-4-(difluoromethyl)pyrrolidine-3-carboxylate hydrochloride (Pharmablock, PBXA3200-1) instead of bromoquinoxaline-5-carbonitrile (compound **1a**) and methyl *(3R,4R*)-4-(trifluoromethyl)pyrrolidine-3-carboxylate HCl salt (compound **1b**). **Example 40** (11 mg) was obtained as a yellow solid. MS: calc'd 414 (MH⁺), measured 414 (MH⁺). ¹H NMR (400 MHz, METHANOL-d4) δ = 8.93 (dd, *J* = 1.5, 4.2 Hz, 1H), 8.75 (dd, *J* = 1.6, 8.7 Hz, 1H), 8.05 (d, *J* = 8.4 Hz, 1H), 7.56 (dd, *J* = 4.2, 8.7 Hz, 1H), 7.00 (d, *J* = 8.4 Hz, 1H), 6.14 - 5.94 (m, 1H), 3.91 - 3.69 (m, 5H), 3.29 - 3.13 (m, 2H), 3.06 - 2.92 (m, 2H), 2.45 - 2.25 (m, 5H), 1.97 (br t, *J* = 13.1 Hz, 2H), 1.70 - 1.51 (m, 2H).

### Example 41

### 5-(8-Cyano-5-quinolyl)-N-(1-methyl-4-piperidyl)-5-azaspiro[2.4]heptane-7-carboxamide

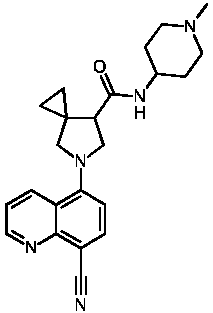

The title compound was prepared in analogy to the preparation of **Example** 6 by using 5-bromoquinoline-8-carbonitrile and 1-methylpiperidin-4-amine instead of 5-bromo-8-(trifluoromethyl)quinoxaline (Intermediate A) and *tert*-butyl 7-amino-3-oxa-9-azabicyclo[3.3.1]nonane-9-carboxylate (compound **6d**). **Example 41** (30 mg) was obtained as a yellow solid. MS: calc'd 390 (MH⁺), measured 390 (MH⁺). ¹H NMR (400 MHz, METHANOL-d₄) *δ* = 8.87 (dd, *J* = 1.5, 4.2 Hz, 1H), 8.83 (dd, *J* = 1.6, 8.8 Hz, 1H), 7.99 (d, *J* = 8.4 Hz, 1H), 7.49 (dd, *J* = 4.2, 8.7 Hz, 1H), 6.84 (d, *J* = 8.6 Hz, 1H), 4.15 (dd, *J* = 6.8, 10.0 Hz, 1H), 4.03 - 3.93 (m, 2H), 3.75 - 3.64 (m, 1H), 3.47 (d, *J* = 9.5 Hz, 1H), 2.97 - 2.87 (m, 2H), 2.81 (dd, *J* = 4.2, 6.7 Hz, 1H), 2.35 (s, 3H), 2.31 - 2.16 (m, 2H), 1.97 - 1.83 (m, 2H), 1.63 - 1.50 (m, 2H), 0.90 - 0.74 (m, 4H).

SFC-HPLC separation gave two isomers: **Example 41A** (5 mg) and **Example 41B** (5 mg).

**Example 41A:** MS: calc'd 390 (MH⁺), measured 390 (MH⁺). ¹H NMR (400 MHz, METHANOL-d₄) *δ* =8.87 (dd, *J* = 1.5, 4.2 Hz, 1H), 8.83 (dd, *J* = 1.5, 8.8 Hz, 1H), 7.99 (d, *J* = 8.4 Hz, 1H), 7.49 (dd, *J* = 4.2, 8.7 Hz, 1H), 6.84 (d, *J* = 8.6 Hz, 1H), 4.15 (dd, *J* = 6.9, 10.1 Hz, 1H), 4.05 - 3.91 (m, 2H), 3.76 - 3.59 (m, 1H), 3.47 (d, *J* = 9.7 Hz, 1H), 2.95 - 2.74 (m, 3H), 2.31 (s, 3H), 2.24 - 2.08 (m, 2H), 1.96 - 1.77 (m, 2H), 1.65 - 1.44 (m, 2H), 0.95 - 0.68 (m, 4H).

**Example 41B:** MS: calc'd 390 (MH⁺), measured 390 (MH⁺). ¹H NMR (400 MHz, METHANOL-d₄) *δ* = 8.88 (dd, *J* = 1.5, 4.2 Hz, 1H), 8.83 (dd, *J* = 1.5, 8.7 Hz, 1H), 7.99 (d, *J* = 8.6 Hz, 1H), 7.49 (dd, *J* = 4.3, 8.7 Hz, 1H), 6.84 (d, *J* = 8.6 Hz, 1H), 4.15 (dd, *J* = 6.9, 10.1 Hz, 1H), 4.06 - 3.89 (m, 2H), 3.77 - 3.61 (m, 1H), 3.47 (d, *J* = 9.7 Hz, 1H), 2.95 - 2.74 (m, 3H), 2.31 (s, 3H), 2.23 - 2.07 (m, 2H), 1.97 - 1.80 (m, 2H), 1.65 - 1.46 (m, 2H), 0.92 - 0.71 (m, 4H).

### Example 42

### N-(3-Oxa-7-azabicyclo[3.3.1]nonan-9-yl)-5-[8-(trifluoromethyl)quinoxalin-5-yl]-5-azaspiro[2.4]heptane-7-carboxamide

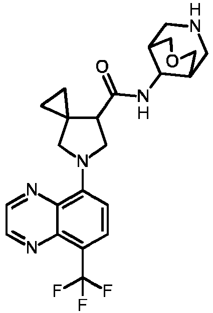

The title compound was prepared in analogy to the preparation of **Example 6** by using *tert*-butyl 9-amino-3-oxa-7-azabicyclo[3.3.1]nonane-7-carboxylate instead of *tert*-butyl 7-amino-3-oxa-9-azabicyclo[3.3.1]nonane-9-carboxylate (compound **6d**). **Example 42** (22 mg) was obtained as a yellow solid. MS: calc'd 462 (MH⁺), measured 462 (MH⁺). ¹H NMR (400 MHz, METHANOL-d₄) *δ* = 8.81 (d, *J* = 1.59 Hz, 1H), 8.70-8.78 (m, 1H), 7.93 (d, *J* = 8.68 Hz, 1H), 6.75 (dd, *J* = 2.93, 8.68 Hz, 1H), 4.34-4.44 (m, 1H), 4.24-4.34 (m, 1H), 4.06-4.24 (m, 4H), 3.96-4.06 (m, 1H), 3.91 (dd, *J* = 5.56, 12.04 Hz, 1H), 3.62-3.74 (m, 2H), 3.41-3.55 (m, 3H), 2.92 (ddd, *J* = 3.36, 7.03, 12.47 Hz, 1H), 2.02-2.22 (m, 2H), 0.79-0.96 (m, 4H).

### Example 43

### Cis-N-(3-aminocyclohexyl)-5-[8-(trifluoromethyl)quinoxalin-5-yl]-5-azaspiro[2.4]heptane-7-carboxamide

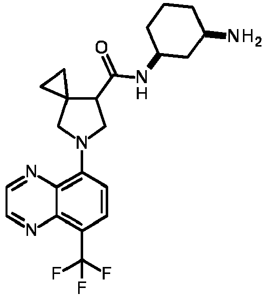

The title compound was prepared in analogy to the preparation of **Example 6** by using *cis-tert*-butyl N-(3-aminocyclohexyl)carbamate (Pharmablock, PBZS1047, CAS:849616-22-4) instead of *tert*-butyl 7-amino-3-oxa-9-azabicyclo[3.3.1]nonane-9-carboxylate (compound **6d**). **Example 43** (29 mg) was obtained as a yellow solid. MS: calc'd 434 (MH⁺), measured 434 (MH⁺). ¹H NMR (400 MHz, METHANOL-d₄) *δ* = 8.69 (d, *J* = 1.47 Hz, 1H), 8.63 (d, *J* = 1.59 Hz, 1H), 7.81 (d, *J* = 8.68 Hz, 1H), 6.62 (d, *J* = 8.68 Hz, 1H), 4.19-4.29 (m, 1H), 4.07-4.17 (m, 1H), 3.96 (dd, *J* = 3.73, 10.82 Hz, 1H), 3.57-3.69 (m, 2H), 2.99-3.10 (m, 1H), 2.63 (ddd, *J* = 3.85, 7.00, 10.79 Hz, 1H), 2.04-2.21 (m, 1H), 1.74-1.96 (m, 3H), 1.28-1.46 (m, 1H), 1.06-1.25 (m, 3H), 0.61-0.77 (m, 4H).

### Example 44

### (3R,4R)-1-(7-cyanopyrazolo[1,5-a]pyridin-4-yl)-4-methyl-N-(1-methyl-4-piperidyl)pyrrolidine-3-carboxamide

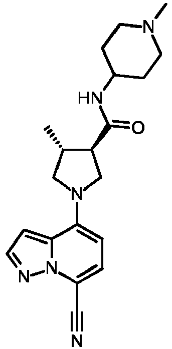

The title compound was prepared in analogy to the preparation of **Example 1** by using methyl *(3R,4R)*-4-methylpyrrolidine-3-carboxylate HCl salt (Pharmablock, PBXA3221-1) and 4-bromopyrazolo[1,5-a]pyridine-7-carbonitrile (CAS: 1268520-74-6, Pharmablock) instead of methyl *(3R,4R*)-4-(trifluoromethyl)pyrrolidine-3-carboxylate HCl salt (compound **1b**) and bromoquinoxaline-5-carbonitrile (compound **1a**). **Example 44** (35 mg) was obtained as a yellow solid. MS: calc'd 367 (MH⁺), measured 367 (MH⁺). ¹H NMR (400 MHz, METHANOL-d₄) *δ* =7.92 (d, *J* = 2.6 Hz, 1H), 7.32 (d, *J* = 8.3 Hz, 1H), 7.06 (d, *J* = 2.6 Hz, 1H), 5.99 (d, *J* = 8.4 Hz, 1H), 4.09 - 3.87 (m, 3H), 3.85 - 3.66 (m, 1H), 3.55 - 3.36 (m, 2H), 2.97 (br d, *J* = 11.6 Hz, 2H), 2.82 - 2.70 (m, 1H), 2.69 - 2.48 (m, 1H), 2.43 - 2.23 (m, 3H), 2.03 - 1.90 (m, 2H), 1.70 - 1.53 (m, 2H), 1.37 (t, *J* = 7.3 Hz, 1H), 1.20 (d, *J* = 6.6 Hz, 3H).

### Example 46

### Trans-1-(8-chloro-5-quinolyl)-N-(1-methyl-4-piperidyl)-4-(trifluoromethyl)pyrrolidine-3-carboxamide

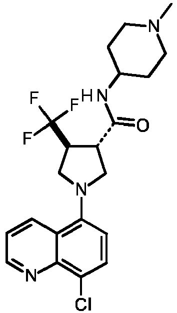

The title compound was prepared in analogy to the preparation of **Example 1** by using *trans*-methyl-4-(trifluoromethyl)pyrrolidine-3-carboxylate HCl salt (Pharmablock, PBXA3194-1) and 5-bromo-8-chloro-quinoline instead of methyl *(3R,4R)*-4-(trifluoromethyl)pyrrolidine-3-carboxylate HCl salt (compound 1b) and bromoquinoxaline-5-carbonitrile (compound **1a**). **Example 46** (27 mg) was obtained as a yellow solid. MS: calc'd 441 (MH⁺), measured 441 (MH⁺). ¹H NMR (400 MHz, METHANOL-d₄) *δ* = 8.93 - 8.84 (m, 1H), 7.88 - 7.78 (m, 1H), 7.76 - 7.67 (m, 1H), 7.49 (dd, *J* = 4.4, 8.4 Hz, 1H), 7.19 - 7.12 (m, 1H), 3.97 - 3.82 (m, 1H), 3.67 - 3.38 (m, 7H), 3.14 - 2.97 (m, 3H), 2.84 - 2.72 (m, 3H), 2.16 - 1.94 (m, 2H), 1.70 - 1.54 (m, 2H).

### Example 47

### Trans-1-(8-cyano-5-quinolyl)-N-[(3S,4R)-4-fluoropyrrolidin-3-yl]-4-(trifluoromethyl)pyrrolidine-3-carboxamide

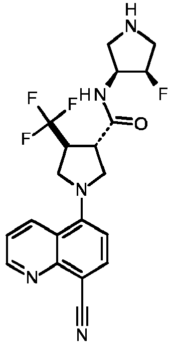

The title compound was prepared in analogy to the preparation of **Example 30 and Example 1** by using *trans*-methyl-4-trifluoromethylpyrrolidine-3-carboxylate and *(3S,4R)-tert-*butyl 3-amino-4-fluoropyrrolidine-1-carboxylate and 5-bromoquinoline-8-carbonitrile instead of methyl *(3R,4R)*-4-(trifluoromethyl)pyrrolidine-3-carboxylate HCl salt (compound **1b**) and bromoquinoxaline-5-carbonitrile (compound **1a**) and *tert*-butyl 4-aminoazepane-1-carboxylate (compound **30d**). **Example 47** (7 mg) was obtained as a yellow solid. MS: calc'd 422 (MH⁺), measured 422 (MH⁺). ¹H NMR (400 MHz, METHANOL-d₄) *δ* =8.84 (dd, *J* = 1.5, 4.2 Hz, 1H), 8.57 (dd, *J =* 1.6, 8.7 Hz, 1H), 7.97 (d, *J=* 8.2 Hz, 1H), 7.48 (dd, *J=* 4.3, 8.7 Hz, 1H), 6.98 (d, *J* = 8.3 Hz, 1H), 5.30 - 5.11 (m, 1H), 4.71 - 4.54 (m, 1H), 3.85 - 3.76 (m, 1H), 3.74 - 3.54 (m, 6H), 3.52 - 3.46 (m, 1H), 3.39 (q, *J =* 7.6 Hz, 1H), 3.10 (t, *J =* 11.4 Hz, 1H).

### Example 48

### N-(2-Azabicyclo[2.2.1]heptan-5-yl)-5-[8-(trifluoromethyl)quinoxalin-5-yl]-5-azaspiro[2.4]heptane-7-carboxamide

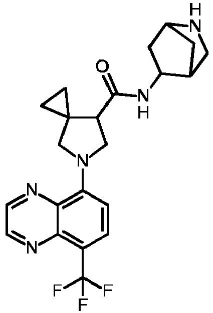

The title compound was prepared in analogy to the preparation of **Example 6** by using *tert*-butyl 5-amino-2-azabicyclo[2.2.1]heptane-2-carboxylate instead of tert-butyl 7-amino-3-oxa-9-azabicyclo[3.3.1]nonane-9-carboxylate (compound **6d**). **Example 48** (21 mg) was obtained as a yellow solid. MS: calc'd 432 (MH⁺), measured 432 (MH⁺). ¹H NMR (400 MHz, METHANOL-d₄) *δ* = 8.80 (d, *J=* 1.47 Hz, 1H), 8.75 (s, 1H), 7.93 (d, *J=* 8.56 Hz, 1H), 6.74 (d, *J =* 8.68 Hz, 1H), 4.34-4.43 (m, 1H), 4.26 (dt, *J=* 4.16, 7.83 Hz, 1H), 3.89-4.19 (m, 3H), 3.63-3.75 (m, 1H), 3.36-3.41 (m, 1H), 3.09-3.23 (m, 1H), 2.90-3.06 (m, 1H), 2.84 (dd, *J* = 3.48, 7.03 Hz, 1H), 2.21-2.39 (m, 1H), 1.90-2.00 (m, 1H), 1.73-1.87 (m, 1H), 1.46-1.71 (m, 1H), 0.69-0.93 (m, 4H).

### Example 49

### Trans-1-(8-cyano-5-quinolyl)-N-[(3S,4R)-4-fluoropyrrolidin-3-yl]-4-isopropyl-pyrrolidine-3-carboxamide

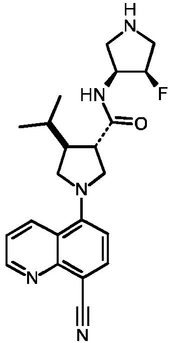

The title compound was prepared in analogy to the preparation of **Example 30** by using *trans*-methyl-4-isopropylpyrrolidine-3-carboxylate and *(3S,4R)-tert*-butyl 3-amino-4-fluoropyrrolidine-1-carboxylate instead of methyl 4-(trifluoromethyl)pyrrolidine-3-carboxylate HCl salt and *tert*-butyl 4-aminoazepane-1-carboxylate (compound **30d**). **Example 49** (12 mg) was obtained as a yellow solid. MS: calc'd 396 (MH⁺), measured 396 (MH⁺). ¹H NMR (400 MHz, METHANOL-d₄) *δ* = 8.90 (dd, *J* = 1.5, 4.3 Hz, 1H), 8.79 (dd, *J* = 1.6, 8.8 Hz, 1H), 8.00 (d, *J* = 8.6 Hz, 1H), 7.52 (dd, *J* = 4.3, 8.8 Hz, 1H), 6.89 (d, *J* = 8.6 Hz, 1H), 5.44 - 5.23 (m, 1H), 4.80 - 4.63 (m, 1H), 4.00 - 3.89 (m, 1H), 3.87 - 3.56 (m, 5H), 3.28 - 2.95 (m, 3H), 2.66 - 2.54 (m, 1H), 1.82 (qd, *J* = 6.9, 14.0 Hz, 1H), 1.05 (dd, *J* = 5.2, 6.7 Hz, 6H).

### Example 50

### (3R,4R)-1-(8-chloro-5-quinolyl)-4-methyl-N (1-methyl-4-piperidyl)pyrrolidine-3-carboxamide

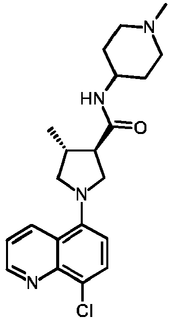

The title compound was prepared in analogy to the preparation of **Example 1** by using methyl *(3R*,*4R)*-4-methylpyrrolidine-3-carboxylate HCl salt (Pharmablock, PBXA3221-1) and 5-bromo-8-chloro-quinoline instead of methyl *(3R,4R)*-4-(trifluoromethyl)pyrrolidine-3-carboxylate HCl salt (compound **1b**) and bromoquinoxaline-5-carbonitrile (compound **1a**). **Example 50** (3 mg) was obtained as a yellow solid. MS: calc'd 387 (MH⁺), measured 387 (MH⁺). ¹H NMR (400 MHz, METHANOL-d₄) *δ* = 9.03 (dd, *J* = 1.3, 5.4 Hz, 1H), 8.04 (d, *J =* 9.0 Hz, 1H), 7.92 (dd, *J* = 5.5, 8.6 Hz, 1H), 7.55 (dd, *J* = 4.4, 8.4 Hz, 1H), 7.15 - 7.03 (m, 1H), 4.13 - 3.96 (m, 2H), 3.93 - 3.76 (m, 2H), 3.64 - 3.52 (m, 3H), 3.27 - 3.09 (m, 3H), 2.98 - 2.61 (m, 4H), 2.23 (brt, *J* = 12.5 Hz, 2H), 1.88 - 1.72 (m, 2H), 1.29 - 1.16 (m, 3H).

### Example 51

### 5-(8-Cyano-5-quinolyl)-N-[(1-methyl-3-piperidyl)methyl]-5-azaspiro[2.4]heptane-7-carboxamide

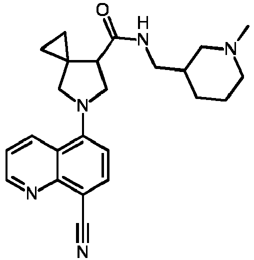

The title compound was prepared in analogy to the preparation of **Example 6** by using 5-bromoquinoline-8-carbonitrile and (1-methyl-3-piperidyl)methanamine instead of 5-bromo-8-(trifluoromethyl)quinoxaline (Intermediate **A**) and *tert*-butyl 7-amino-3-oxa-9-azabicyclo[3.3.1]nonane-9-carboxylate (compound **6d**). **Example 51** (9 mg) was obtained as a yellow solid. MS: calc'd 404 (MH⁺), measured 404 (MH⁺). ¹H NMR (400 MHz, METHANOL-d₄) *δ* = 8.88 (d, *J* = 4.0 Hz, 1H), 8.83 (d, *J* = 8.8 Hz, 1H), 7.99 (d, *J* = 8.4 Hz, 1H), 7.49 (dd, *J* = 4.3, 8.7 Hz, 1H), 6.84 (d, *J* = 8.6 Hz, 1H), 4.22 (dd, *J* = 7.0, 9.9 Hz, 1H), 4.07 - 3.92 (m, 2H), 3.41 (d, *J=* 9.7 Hz, 1H), 3.31 - 3.25 (m, 2H), 3.25 - 3.08 (m, 2H), 2.84 - 2.68 (m, 5H), 2.61 - 2.46 (m, 1H), 2.05 - 1.63 (m, 4H), 1.29 - 1.14 (m, 1H), 0.95 - 0.74 (m, 4H).

### Example 52

### Trans-N-(azepan-4-yl)-1-(8-cyano-5-quinolyl)-4-methyl-pyrrolidine-3-carboxamide

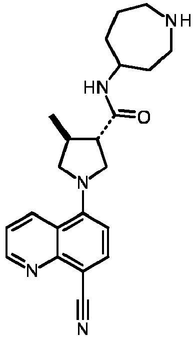

The title compound was prepared in analogy to the preparation of **Example 30** by using trans-methyl-4-methylpyrrolidine-3-carboxylate (Bepharm, B162777) instead of methyl 4-(trifluoromethyl)pyrrolidine-3-carboxylate HCl salt. Prep-HPLC separation gave two isomers **Example 52A** (12 mg) and **Example 52B** (8 mg).

**Example 52A:** MS: calc'd 378 (MH⁺), measured 378 (MH⁺). ¹H NMR (400 MHz, METHANOL-d₄) *δ* = 9.09 (dd, *J* = 1.3, 8.8 Hz, 1H), 8.92 (dd, *J =* 1.5, 4.8 Hz, 1H), 8.06 (d, *J* = 8.8 Hz, 1H), 7.65 (dd, *J* = 4.8, 8.8 Hz, 1H), 6.89 (d, *J=* 8.8 Hz, 1H), 4.13 - 3.98 (m, 2H), 3.92 (ddd, *J=* 7.3, 10.1, 14.2 Hz, 2H), 3.60 (t, *J=* 9.8 Hz, 1H), 3.30 - 3.17 (m,3H), 2.82 - 2.53 (m, 2H), 2.26 - 1.64 (m, 7H), 1.22 (d, *J=* 6.6 Hz, 3H).

**Example 52B:** MS: calc'd 378 (MH⁺), measured 378 (MH⁺). ¹H NMR (400 MHz, METHANOL-d₄) *δ* = 9.09 (dd, *J* = 1.3, 8.8 Hz, 1H), 8.92 (dd, *J =* 1.3, 4.8 Hz, 1H), 8.06 (d, *J* = 8.7 Hz, 1H), 7.65 (dd, *J* = 4.8, 8.8 Hz, 1H), 6.89 (d, *J=* 8.7 Hz, 1H), 4.15 - 3.99 (m, 2H), 3.92 (ddd, J = 7.3, 10.1, 14.2 Hz, 2H), 3.60 (t, *J* = 9.8 Hz, 1H), 3.27 - 3.13 (m, 3H), 2.82 - 2.61 (m, 2H), 2.31 - 1.61 (m, 7H), 1.22 (d, *J =* 6.5 Hz, 3H).

### Example 53

### Trans-5-[3-(2,7-diazaspiro[4.4]nonane-2-carbonyl)-4-methyl-pyrrolidin-1-yl]quinoline-8-carbonitrile

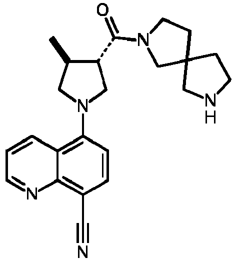

The title compound was prepared in analogy to the preparation of **Example 30** by using *trans*-methyl-4-methylpyrrolidine-3-carboxylate (Bepharm, B162777) and tert-butyl 2,7-diazaspiro[4.4]nonane-2-carboxylate instead of methyl 4-(trifluoromethyl)pyrrolidine-3-carboxylate HCl salt and tert-butyl 4-aminoazepane-1-carboxylate (compound **30d**). **Example 53** (4 mg) was obtained as a yellow solid. MS: calc'd 390 (MH⁺), measured 390 (MH⁺). ¹H NMR (400 MHz, METHANOL-d₄) *δ* = 9.00 - 8.88 (m, 2H), 8.03 (d, *J =* 8.6 Hz, 1H), 7.64 - 7.53 (m, 1H), 6.87 (d, *J =* 8.7 Hz, 1H), 4.06 - 3.96 (m, 2H), 3.94 - 3.85 (m, 1H), 3.81 (br t, *J =* 7.2 Hz, 1H), 3.66 - 3.41 (m, 7H), 3.25 - 3.05 (m, 1H), 2.83 - 2.69 (m, 1H), 2.25 - 1.97 (m, 5H), 1.25 (d, *J* = 6.6 Hz, 3H).

### Example 54

### (3S,4S)-1-(8-Cyano-5-quinolyl)-4-methyl-N-(1-methyl-4-piperidyl)pyrrolidine-3-carboxamide

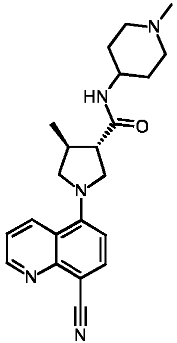

The title compound was prepared in analogy to the preparation of **Example 1** by using methyl-(3S,4S)-4-methylpyrrolidine-3-carboxylate hydrochloride (PBXA3220-1, Pharmablock) and 5-bromoquinoline-8-carbonitrile instead of methyl (*3R*,*4R*)-4-(trifluoromethyl)pyrrolidine-3-carboxylate HCl salt (compound **1b**) and bromoquinoxaline-5-carbonitrile (compound **1a**). **Example 54** (50 mg) was obtained as a yellow solid. MS: calc'd 378 (MH⁺), measured 378 (MH⁺). ¹H NMR (400 MHz, METHANOL-d₄) *δ* = 8.87 - 8.68 (m, 2H), 7.92 (d, *J* = 8.6 Hz, 1H), 7.44 (dd, *J =* 4.2, 8.7 Hz, 1H), 6.75 (d, *J=* 8.6 Hz, 1H), 4.11 - 3.96 (m, 1H), 3.84 - 3.69 (m, 3H), 3.50 (t, *J=* 9.7 Hz, 1H), 3.01 - 2.88 (m, 2H), 2.75 - 2.53 (m, 2H), 2.38 - 2.17 (m, 5H), 1.99 - 1.84 (m, 2H), 1.59 (q, *J* = 11.4 Hz, 2H), 1.18 (d, *J* = 6.4 Hz, 3H).

### Example 55

### Trans-1-(8-cyano-5-quinolyl)-4-cyclopropyl-N-(2-morpholinoethyl)pyrrolidine-3-carboxamide

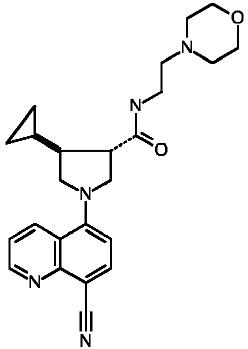

The title compound was prepared in analogy to the preparation of **Example** 1 by using *trans*-ethyl 4-cyclopropylpyrrolidine-3-carboxylate hydrochloride salt (Pharmablock, PBXA3214-1) and 2-morpholinoethanamine and 5-bromoquinoline-8-carbonitrile instead of methyl (*3R*,*4R*)-4-(trifluoromethyl)pyrrolidine-3-carboxylate HCl salt (compound **1b**) and bromoquinoxaline-5-carbonitrile (compound **1a**) and 1-methylpiperidin-4-amine (compound **1e**). **Example 55** (13 mg) was obtained as a yellow solid. MS: calc'd 420 (MH⁺), measured 420 (MH⁺). ¹H NMR (400 MHz, METHANOL-d₄) *δ* = 9.16 (d, *J* = 8.7 Hz, 1H), 8.92 (dd, *J* = 1.2, 4.9 Hz, 1H), 8.08 (d, *J=* 8.8 Hz, 1H), 7.68 (dd, *J* = 4.9, 8.8 Hz, 1H), 6.92 (d, *J=* 8.8 Hz, 1H), 4.14 - 4.03 (m, 3H), 3.96 (td, *J* = 7.1, 10.1 Hz, 2H), 3.85 - 3.62 (m, 5H), 3.62 - 3.45 (m, 2H), 3.28 - 2.95 (m, 5H), 1.99 - 1.86 (m, 1H), 0.96 - 0.80 (m, 1H), 0.60 - 0.50 (m, 2H), 0.32 - 0.21 (m, 2H).

### Example 56

### Trans-1-(8-cyano-5-quinolyl)-N-[(3S,4R)-4-fluoropyrrolidin-3-yl]-4-methyl-pyrrolidine-3-carboxamide

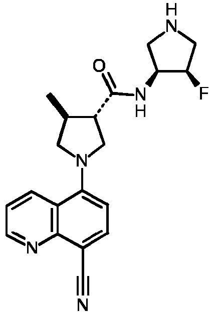

The title compound was prepared in analogy to the preparation of **Example 30** by using *trans*-methyl-4-methylpyrrolidine-3-carboxylate and *(3S,4R)-tert*-butyl 3-amino-4-fluoropyrrolidine-1-carboxylate instead of methyl 4-(trifluoromethyl)pyrrolidine-3-carboxylate HCl salt and *tert*-butyl 4-aminoazepane-1-carboxylate (compound **30d**). **Example 56** (4 mg) was obtained as a yellow solid. MS: calc'd 368 (MH⁺), measured 368 (MH⁺). ¹H NMR (400 MHz, METHANOL-d₄) *δ* = 8.94 - 8.75 (m, 2H), 7.99 (d, *J* = 8.4 Hz, 1H), 7.51 (dd, *J* = 4.3, 8.8 Hz, 1H), 6.84 (d, *J=* 8.6 Hz, 1H), 5.43 - 5.19 (m, 1H), 4.07 (t, *J=* 9.5 Hz, 1H), 3.92 - 3.48 (m, 7H), 3.25 (t, *J* = 11.3 Hz, 1H), 2.95 - 2.80 (m, 1H), 2.73 - 2.60 (m, 1H), 1.23 (d, *J* = 6.6 Hz, 3H).

### Example 57

### N (Azepan-4-yl)-5-(8-cyano-5-quinolyl)-5-azaspiro[2.4]heptane-7-carboxamide

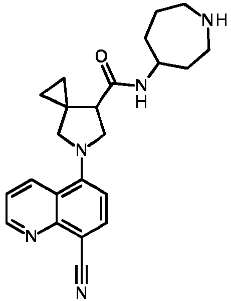

The title compound was prepared in analogy to the preparation of **Example 6** by using 5-bromoquinoline-8-carbonitrile and *tert*-butyl 4-aminoazepane-1-carboxylate instead of 5-bromo-8-(trifluoromethyl)quinoxaline (Intermediate A) and tert-butyl 7-amino-3-oxa-9-azabicyclo[3.3.1]nonane-9-carboxylate (compound **6d**). Prep-HPLC separation gave two isomers **Example 57A** (7 mg) and **Example 57B** (7 mg).

**Example 57A:** MS: calc'd 390 (MH⁺), measured 390 (MH⁺). ¹H NMR (400 MHz, METHANOL-d₄) *δ* = 8.88 (d, *J* = 3.3 Hz, 1H), 8.83 (d, *J* = 8.9 Hz, 1H), 7.99 (d, *J* = 8.4 Hz, 1H), 7.49 (dd, *J =* 4.3, 8.7 Hz, 1H), 6.84 (d, *J=* 8.6 Hz, 1H), 4.18 (dd, *J* = 7.0, 9.9 Hz, 1H), 4.02 - 3.83 (m, 3H), 3.45 (d, *J=* 9.4 Hz, 1H), 3.28 - 3.17 (m, 2H), 3.17 - 3.00 (m, 2H), 2.78 (dd, *J =* 3.7, 6.5 Hz, 1H), 2.18 - 1.89 (m, 3H), 1.88 - 1.73 (m, 2H), 1.73 - 1.56 (m, 1H), 0.91 - 0.70 (m, 4H).

**Example 57B:** MS: calc'd 390 (MH⁺), measured 390 (MH⁺). ¹H NMR (400 MHz, METHANOL-d₄) *δ* = 8.88 (dd, *J* = 1.5, 4.2 Hz, 1H), 8.83 (dd, *J =* 1.5, 8.7 Hz, 1H), 7.99 (d, *J* = 8.4 Hz, 1H), 7.49 (dd, *J =* 4.3, 8.8 Hz, 1H), 6.84 (d, *J* = 8.6 Hz, 1H), 4.18 (dd, *J =* 6.8, 10.0 Hz, 1H), 4.02 - 3.87 (m, 3H), 3.45 (d, *J=* 9.7 Hz, 1H), 3.31 - 3.22 (m, 2H), 3.21 - 3.09 (m, 2H), 2.79 (dd, *J* = 3.7, 6.8 Hz, 1H), 2.22 - 1.95 (m, 3H), 1.93 - 1.74 (m, 2H), 1.70 - 1.56 (m, 1H), 0.90 - 0.74 (m, 4H).

### Example 58

### (3S,4S)-1-(8-cyano-5-quinolyl)-N-(1-methyl-4-piperidyl)-4-(trifluoromethyl)pyrrolidine-3-carboxamide

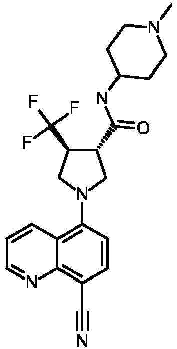

The title compound was prepared in analogy to the preparation of **Example 1** by using methyl-(*3S*,*4S*)-4-(trifluoromethyl)pyrrolidine-3-carboxylate hydrochloride (Pharmablock, PBXA3228-1) and 5-bromoquinoline-8-carbonitrile instead of methyl *(3R,4R)*-4-(trifluoromethyl)pyrrolidine-3-carboxylate HCl salt (compound **1b**) and bromoquinoxaline-5-carbonitrile (compound **1a**). **Example 58** (19 mg) was obtained as a yellow solid. MS: calc'd 432 (MH⁺), measured 432 (MH⁺). ¹H NMR (400 MHz, METHANOL-d₄) *δ* = 8.95 (dd, *J* = 1.5, 4.2 Hz, 1H), 8.70 (dd, *J* = 1.6, 8.7 Hz, 1H), 8.07 (d, *J=* 8.2 Hz, 1H), 7.59 (dd, *J=* 4.2, 8.7 Hz, 1H), 7.07 (d, *J* = 8.3 Hz, 1H), 3.94 - 3.72 (m, 3H), 3.69 - 3.57 (m, 1H), 3.49 (q, *J* = 7.3 Hz, 2H), 3.06 - 2.90 (m, 3H), 2.43 - 2.27 (m, 5H), 1.97 (br t, *J* = 12.7 Hz, 2H), 1.70 - 1.54 (m, 2H).

### Example 59

### N-(2-Amino-2-methyl-propyl)-5-(8-cyano-5-quinolyl)-5-azaspiro[2.4]heptane-7-carboxamide

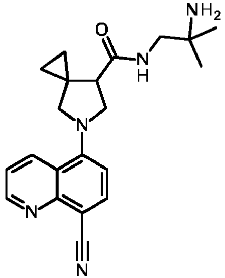

The title compound was prepared in analogy to the preparation of **Example 6** by using 5-bromoquinoline-8-carbonitrile and *tert*-butyl N-(2-amino-1,1-dimethyl-ethyl)carbamate instead of 5-bromo-8-(trifluoromethyl)quinoxaline (Intermediate **A**) and *tert*-butyl 7-amino-3-oxa-9-azabicyclo[3.3.1]nonane-9-carboxylate (compound **6d**). **Example 59** (13 mg) was obtained as a yellow solid. MS: calc'd 364 (MH⁺), measured 364 (MH⁺). ¹H NMR (400 MHz, METHANOL-d₄) *δ* = 8.89 - 8.86 (m, 1H), 8.83 (d, *J=* 8.7 Hz, 1H), 7.99 (dd, *J* = 1.3, 8.4 Hz, 1H), 7.49 (dd, *J* = 4.3, 8.7 Hz, 1H), 6.83 (d, *J* = 8.6 Hz, 1H), 4.24 (dd, *J* = 6.7, 10.0 Hz, 1H), 4.10 - 3.94 (m, 2H), 3.50 (d, *J =* 14.3 Hz, 1H), 3.41 (d, *J* = 9.5 Hz, 1H), 3.17 (d, *J =* 14.1 Hz, 1H), 2.87 - 2.80 (m, 1H), 1.31 (s, 3H), 1.30 (s, 3H), 0.97 - 0.78 (m, 4H).

### Example 60

### (3R,4R)-1-(8-cyanoquinoxalin-5-yl)-4-methyl-N-[1-(4-piperidyl)-4-piperidyl]pyrrolidine-3-carboxamide

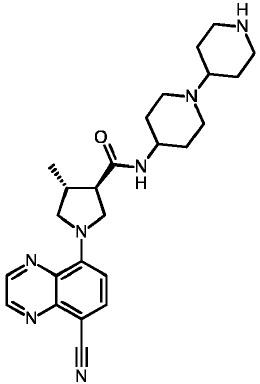

The title compound was prepared in analogy to the preparation of **Example 30** by using methyl (*3R*,*4R*)-4-methylpyrrolidine-3-carboxylate HCl salt (Pharmablock, PBXA3221-1) and *tert*-butyl 4-(4-amino-1-piperidyl)piperidine-1-carboxylate instead of methyl 4-(trifluoromethyl)pyrrolidine-3-carboxylate HCl salt and *tert*-butyl 4-aminoazepane-1-carboxylate (compound **30d**). **Example 60** (10 mg) was obtained as a yellow solid. MS: calc'd 448 (MH⁺), measured 448 (MH⁺). ¹H NMR (400 MHz, METHANOL-d₄) *δ* = 8.81 (d, *J* = 1.6 Hz, 1H), 8.72 (d, *J* = 1.5 Hz, 1H), 7.92 (d, *J=* 8.6 Hz, 1H), 6.70 (d, *J=* 8.7 Hz, 1H), 4.27 - 3.96 (m, 4H), 3.75 - 3.48 (m, 6H), 3.29 - 3.04 (m, 4H), 2.81 - 2.22 (m, 6H), 2.09 - 1.78 (m, 4H), 1.22 (d, *J* = 6.5 Hz, 3H).

### Example 61

### Trans-N-(azepan-4-yl)-1-(8-cyano-5-quinolyl)-4-(difluoromethyl)pyrrolidine-3-carboxamide

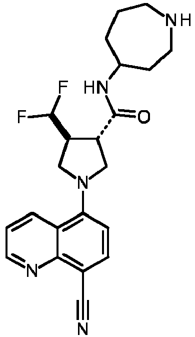

The title compound was prepared in analogy to the preparation of **Example 30** by using *trans*-ethyl-4-difluoromethyl-pyrrolidine-3-carboxylate hydrochloride (Pharmablock, PBXA3200-1) instead of methyl 4-(trifluoromethyl)pyrrolidine-3-carboxylate HCl salt (compound **1b**). Prep-HPLC separation gave two isomers **Example 61A** (14 mg) and **Example 61B** (8 mg).

**Example 61A:** MS: calc'd 414 (MH⁺), measured 414 (MH⁺). ¹H NMR (400 MHz, METHANOL-d₄) *δ* = 8.81 (dd, *J* = 1.5, 4.2 Hz, 1H), 8.64 (dd, *J =* 1.6, 8.7 Hz, 1H), 7.93 (d, *J* = 8.3 Hz, 1H), 7.44 (dd, *J =* 4.2, 8.7 Hz, 1H), 6.88 (d, *J =* 8.3 Hz, 1H), 6.08 - 5.84 (m, 1H), 3.89 (tt, *J=* 4.6, 9.4 Hz, 1H), 3.79 - 3.60 (m, 5H), 3.17 - 2.97 (m, 5H), 2.12 - 1.46 (m, 6H).

**Example 61B:** MS: calc'd 414 (MH⁺), measured 414 (MH⁺). ¹H NMR (400 MHz, METHANOL-d₄) *δ* = 8.81 (dd, *J* = 1.5, 4.2 Hz, 1H), 8.64 (dd, *J =* 1.6, 8.7 Hz, 1H), 7.93 (d, *J* = 8.3 Hz, 1H), 7.44 (dd, *J =* 4.2, 8.7 Hz, 1H), 6.88 (d, *J =* 8.3 Hz, 1H), 6.08 - 5.84 (m, 1H), 3.89 (tt, *J=* 4.6, 9.4 Hz, 1H), 3.79 - 3.60 (m, 5H), 3.17 - 2.97 (m, 5H), 2.12 - 1.46 (m, 6H).

### Example 62

### (3S,4S)-1-(8-Chloro-5-quinolyl)-4-methyl-N-(1-methyl-4-piperidyl)pyrrolidine-3-carboxamide

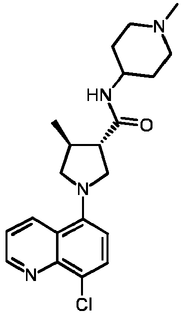

The title compound was prepared in analogy to the preparation of **Example** 1 by using methyl (3S,4S)-4-methylpyrrolidine-3-carboxylate hydrochloride (Pharmablock, PBXA3220-1) and 5-bromo-8-chloro-quinoline instead of methyl (*3R*,*4R*)-4-(trifluoromethyl)pyrrolidine-3-carboxylate HCl salt (compound 1b) and bromoquinoxaline-5-carbonitrile (compound **1a**). **Example 62** (35 mg) was obtained as a yellow solid. MS: calc'd 387 (MH⁺), measured 387 (MH⁺). ¹H NMR (400 MHz, METHANOL-d₄) *δ* = 8.75 (dd, *J* = 1.5, 4.2 Hz, 1H), 8.63 (dd, *J* = 1.6, 8.7 Hz, 1H), 7.60 (d, *J=* 8.3 Hz, 1H), 7.42 (dd, *J=* 4.3, 8.7 Hz, 1H), 6.85 (d, *J =* 8.4 Hz, 1H), 3.74 (dd, *J=* 8.1, 9.2 Hz, 1H), 3.66 - 3.57 (m, 1H), 3.45 - 3.28 (m, 2H), 3.18 - 3.09 (m, 1H), 2.91 - 2.74 (m,2H), 2.62 - 2.47 (m, 2H), 2.23 - 2.19 (m, 3H), 2.14 - 1.99 (m, 2H), 1.89 - 1.75 (m, 2H), 1.54 - 1.38 (m, 2H), 1.10 (d, *J=* 6.4 Hz, 3H).

### Example 63

### 5-(8-Cyano-5-quinolyl)-N-[(4-methylmorpholin-2-yl)methyl]-5-azaspiro[2.4]heptane-7-carboxamide

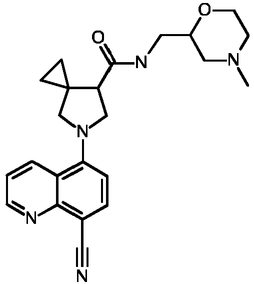

The title compound was prepared in analogy to the preparation of **Example 6** by using 5-bromoquinoline-8-carbonitrile and (4-methylmorpholin-2-yl)methanamine instead of 5-bromo-8-(trifluoromethyl)quinoxaline (Intermediate **A**) and *tert*-butyl 7-amino-3-oxa-9-azabicyclo[3.3.1]nonane-9-carboxylate (compound **6d**). **Example 63** (14 mg) was obtained as a yellow solid. MS: calc'd 406 (MH⁺), measured 406 (MH⁺). ¹H NMR (400 MHz, METHANOL-d₄) *δ* =8.88 (dd, *J* = 1.3, 4.2 Hz, 1H), 8.83 (d, *J* = 8.7 Hz, 1H), 7.99 (d, *J* = 8.4 Hz, 1H), 7.49 (dd, *J* = 4.3, 8.7 Hz, 1H), 6.84 (d, *J* = 8.6 Hz, 1H), 4.18 (ddd, *J* = 2.9, 7.0, 10.0 Hz, 1H), 4.05 - 3.94 (m, 3H), 3.75 - 3.62 (m, 2H), 3.48 - 3.36 (m, 2H), 3.30 - 3.17 (m, 1H), 3.13 - 2.97 (m, 2H), 2.83 (dt, *J* = 3.7, 6.6 Hz, 1H), 2.65 - 2.50 (m, 1H), 2.58 (s, 3H), 2.32 (d, *J* = 7.9 Hz, 1H), 0.96 - 0.75 (m, 4H).

### Example 64

### 1-(8-Cyano-5-quinolyl)-N (1-methyl-4-piperidyl)pyrrolidine-3-carboxamide

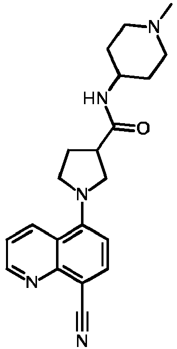

The title compound was prepared in analogy to the preparation of **Example 1** by using methyl-pyrrolidine-3-carboxylate and 5-bromoquinoline-8-carbonitrile instead of methyl (*3R*,*4R*)-4-(trifluoromethyl)pyrrolidine-3-carboxylate HCl salt (compound **1b**) and bromoquinoxaline-5-carbonitrile (compound **1a**). **Example 64** (3 mg) was obtained as a yellow solid. MS: calc'd 364 (MH⁺), measured 364 (MH⁺). ¹H NMR (400 MHz, METHANOL-d₄) *δ* = 8.81 - 8.62 (m, 2H), 7.88 (d, *J* = 8.3 Hz, 1H), 7.43 - 7.33 (m, 1H), 6.75 (d, *J=* 8.4 Hz, 1H), 3.74 - 3.45 (m, 5H), 2.86 (t, *J* = 4.2 Hz, 3H), 2.29 - 1.81 (m, 3H), 1.34 - 0.98 (m, 8H).

### Example 65

### 5-[7-(3-Aminoazetidine-1-carbonyl)-5-azaspiro[2.4]heptan-5-yl]quinoline-8-carbonitrile

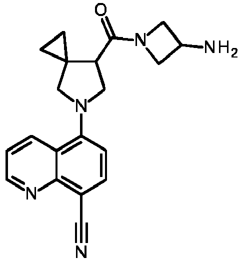

The title compound was prepared in analogy to the preparation of **Example 6** by using 5-bromoquinoline-8-carbonitrile and *tert*-butyl N-(azetidin-3-yl)carbamate instead of 5-bromo-8-(trifluoromethyl)quinoxaline (Intermediate A) and *tert*-butyl 7-amino-3-oxa-9-azabicyclo[3.3.1]nonane-9-carboxylate (compound **6d). Example 65** (13 mg) was obtained as a yellow solid. MS: calc'd 348 (MH⁺), measured 348 (MH⁺). ¹H NMR (400 MHz, METHANOL-d₄) *δ* = 8.87 (d, *J* = 4.2 Hz, 1H), 8.80 (d, *J* = 8.8 Hz, 1H), 7.97 (d, *J* = 8.4 Hz, 1H), 7.53 - 7.44 (m, 1H), 6.83 (d, *J* = 8.6 Hz, 1H), 4.55 - 4.36 (m, 1H), 4.29 - 4.08 (m, 2H), 4.06 - 3.84 (m, 4H), 3.74 (dd, *J =* 5.3, 10.8 Hz, 1H), 3.48 (dd, *J =* 3.9, 9.5 Hz, 1H), 3.05 - 2.95 (m, 1H), 0.95 - 0.74 (m, 4H).

### Example 66

### Methyl (2R,4R)-4-[[5-[8-(trifluoromethyl)quinoxalin-5-yl]-5-azaspiro[2.4]heptane-7-carbonyl]amino]pyrrolidine-2-carboxylate

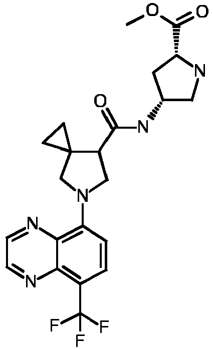

The title compound was prepared in analogy to the preparation of **Example 6** by using methyl (2*R*,4*R*)-4-aminopyrrolidine-2-carboxylate (Pharmablock, PB05111,CAS: 1217474-04-8) instead of *tert*-butyl 7-amino-3-oxa-9-azabicyclo[3.3.1]nonane-9-carboxylate (compound 6d). **Example 66** (16 mg) was obtained as a yellow solid. MS: calc'd 464 (MH⁺), measured 464 (MH⁺). ¹H NMR (400 MHz, METHANOL-d₄) *δ* = 8.69 (d, *J* = 1.71 Hz, 1H), 8.63 (d, *J* = 1.34 Hz, 1H), 7.81 (d, *J=* 8.68 Hz, 1H), 6.62 (d, *J =* 8.68 Hz, 1H), 4.37-4.59 (m, 1H), 4.20-4.35 (m, 2H), 4.09-4.19 (m, 1H), 3.94-4.03 (m, 1H), 3.72-3.81 (m, 3H), 3.46-3.60 (m, 2H), 3.11-3.19 (m, 1H), 2.36-2.72 (m, 2H), 1.99-2.35 (m, 1H), 0.61-0.80 (m, 4H).

### Example 67

### Trans-1-(8-cyano-5-quinolyl)-4-(difluoromethyl)-N-[(3S,4R)-4-fluoropyrrolidin-3-yl]pyrrolidine-3-carboxamide

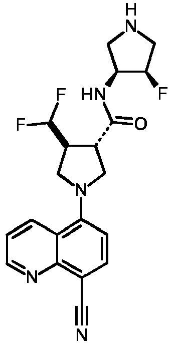

The title compound was prepared in analogy to the preparation of **Example 30** by using *trans*-ethyl-4-difluoromethyl-pyrrolidine-3-carboxylate hydrochloride (Pharmablock, PBXA3200-1) and *(3S,4R)-tert*-butyl 3-amino-4-fluoropyrrolidine-1-carboxylate instead of methyl 4-(trifluoromethyl)pyrrolidine-3-carboxylate HCl salt and *tert*-butyl 4-aminoazepane-1-carboxylate (compound **30d). Example 67** (11 mg) was obtained as a yellow solid. MS: calc'd 404 (MH⁺), measured 404 (MH⁺). ¹H NMR (400 MHz, METHANOL-d₄) *δ* = 8.82 (dd, *J* = 1.6, 4.3 Hz, 1H), 8.64 (dd, *J* = 1.6, 8.8 Hz, 1H), 7.94 (d, *J=* 8.3 Hz, 1H), 7.45 (dd, *J=* 4.3, 8.7 Hz, 1H), 6.90 (d, *J=* 8.4 Hz, 1H), 6.08 - 5.86 (m, 1H), 5.28 - 5.10 (m, 1H), 4.70 - 4.51 (m, 1H), 3.83 - 3.74 (m, 1H), 3.69 - 3.53 (m, 3H), 3.54 - 3.42 (m, 1H), 3.31 - 3.24 (m, 3H), 3.18 - 3.01 (m, 2H).

### Example 70

### 5-(8-Cyanoquinoxalin-5-yl)-N-[[(2R)-morpholin-2-yl]methyl]-5-azaspiro[2.4]heptane-7-carboxamide

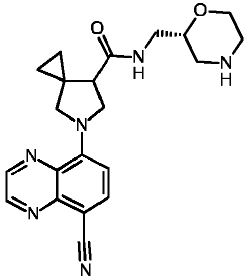

The title compound was prepared in analogy to the preparation of **Example 6** by using 8-bromoquinoxaline-5-carbonitrile and *tert*-butyl (*2S*)-2-(aminomethyl)morpholine-4-carboxylate (CAS: 879403-42-6, Pharmablock, PBN 20121306) instead of 5-bromo-8-(trifluoromethyl)quinoxaline (Intermediate **A)** and *tert*-butyl 7-amino-3-oxa-9-azabicyclo[3.3.1]nonane-9-carboxylate (compound **6d**). **Example 70** (79 mg) was obtained as a yellow solid. MS: calc'd 393 (MH⁺), measured 393 (MH⁺). ¹H NMR (400 MHz, METHANOL-d₄) *δ* = 8.72 (d, *J=* 1.83 Hz, 1H), 8.64 (d, *J* = 1.71 Hz, 1H), 7.87 (d, *J=* 8.68 Hz, 1H), 6.65 (d, *J* = 8.80 Hz, 1H), 4.15-4.31 (m, 2H), 3.96-4.10 (m, 2H), 3.61-3.74 (m, 3H), 3.29-3.39 (m, 1H), 3.24-3.28 (m, 1H), 3.10-3.17 (m, 2H), 2.95-3.07 (m, 1H), 2.77 (dt, *J* = 5.20, 12.07 Hz, 1H), 2.63-2.72 (m, 1H), 0.62-0.86 (m, 4H).

SFC-HPLC (40% CO₂/0.5%NH₃ in methanol as eluent on Daicel AD-H Column) separation gave two single isomers **Example 70A** (14 mg) and **Example 70B** (14 mg).

**Example 70A:** MS: calc'd 393 (MH⁺), measured 393 (MH⁺). ¹H NMR (400 MHz, METHANOL-d₄) *δ* = 8.84 (d, *J* = 1.71 Hz, 1H), 8.75 (d, *J* = 1.71 Hz, 1H), 7.98 (d, *J=* 8.68 Hz, 1H), 6.76 (d, *J=* 8.68 Hz, 1H), 4.27-4.42 (m, 2H), 4.07-4.20 (m, 2H), 3.72-3.84 (m, 3H), 3.40-3.52 (m, 1H), 3.30 (m, 1H), 3.21-3.29 (m, 2H), 3.10-3.19 (m, 1H), 2.85-2.96 (m, 1H), 2.81 (dd, *J* = 3.55, 6.85 Hz, 1H), 0.74-0.93 (m, 4H).

**Example 70B:** MS: calc'd 393 (MH⁺), measured 393 (MH⁺). ¹H NMR (400 MHz, METHANOL-d₄) *δ* = 8.82 (d, *J* = 1.83 Hz, 1H), 8.74 (d, *J* = 1.71 Hz, 1H), 7.97 (d, *J=* 8.68 Hz, 1H), 6.74 (d, *J=* 8.80 Hz, 1H), 4.26-4.40 (m, 2H), 4.18 (d, *J =* 11.25 Hz, 1H), 4.12 (dd, *J* = 3.48, 13.02 Hz, 1H), 3.71-3.87 (m, 3H), 3.36-3.43 (m, 2H), 3.25-3.30 (m, 2H), 3.08-3.17 (m, 1H), 2.88 (dd, *J=* 11.49, 12.59 Hz, 1H), 2.80 (dd, *J=* 3.30, 6.85 Hz, 1H), 0.90-0.97 (m, 1H), 0.78-0.90 (m, 3H).

### Example 71

### 5-(8-Cyanoquinoxalin-5-yl)-N-[[(2S)-morpholin-2-yl]methyl]-5-azaspiro[2.4]heptane-7-carboxamide

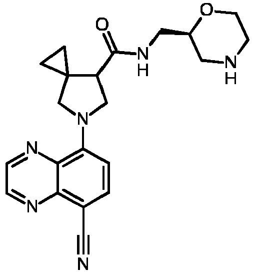

The title compound was prepared in analogy to the preparation of **Example 6** by using 8-bromoquinoxaline-5-carbonitrile and *tert*-butyl (*2R*)-2-(aminomethyl)morpholine-4-carboxylate (CAS: 1174913-80-4, Pharmablock, PBN 20121305) instead of 5-bromo-8-(trifluoromethyl)quinoxaline (Intermediate A) and *tert*-butyl 7-amino-3-oxa-9-azabicyclo[3.3.1]nonane-9-carboxylate (compound **6d**). **Example 71** (84 mg) was obtained as a yellow solid. MS: calc'd 393 (MH⁺), measured 393 (MH⁺). ¹H NMR (400 MHz, METHANOL-d₄) *δ* = 8.72 (d, *J=* 1.71 Hz, 1H), 8.64 (d, *J=* 1.71 Hz, 1H), 7.87 (d, *J=* 8.68 Hz, 1H), 6.65 (d, *J* = 8.68 Hz, 1H), 4.15-4.32 (m, 2H), 3.95-4.12 (m, 2H), 3.59-3.73 (m, 3H), 3.30-3.41 (m, 1H), 3.24-3.29 (m, 1H), 3.07-3.16 (m, 2H), 2.95-3.07 (m, 1H), 2.77 (dt, *J* = 5.07, 12.07 Hz, 1H), 2.63-2.72 (m, 1H), 0.65-0.84 (m, 4H).

SFC-HPLC (40% CO₂/0.5%NH₃ in methanol as eluent on Daicel AD-H Column) separation gave two single isomers **Example 71A** (14 mg) and **Example 71B** (18 mg).

**Example 71A:** MS: calc'd 393 (MH⁺), measured 393 (MH⁺). ¹H NMR (400 MHz, METHANOL-d₄) *δ* = 8.84 (d, *J* = 1.71 Hz, 1H), 8.76 (d, *J* = 1.71 Hz, 1H), 7.96-8.01 (m, 1H), 6.77 (d, *J=* 8.80 Hz, 1H), 4.35-4.43 (m, 1H), 4.27-4.35 (m, 1H), 4.19 (d, *J* = 11.25 Hz, 1H), 4.12 (dd, *J =* 3.36, 13.14 Hz, 1H), 3.72-3.85 (m, 3H), 3.36-3.40 (m, 2H), 3.27 (d, *J* = 10.27 Hz, 2H), 3.08-3.17 (m, 1H), 2.84-2.93 (m, 1H), 2.79 (dd, *J* = 3.24, 6.79 Hz, 1H), 0.87-0.96 (m, 1H), 0.77-0.87 (m, 3H).

**Example 71B:** MS: calc'd 393 (MH⁺), measured 393 (MH⁺). ¹H NMR (400 MHz, METHANOL-d₄) *δ* = 8.82 (d, *J* = 1.71 Hz, 1H), 8.74 (d, *J* = 1.83 Hz, 1H), 7.97 (d, *J=* 8.68 Hz, 1H), 6.74 (d, *J=* 8.80 Hz, 1H), 4.27-4.42 (m, 2H), 4.08-4.20 (m, 2H), 3.72-3.86 (m, 3H), 3.40-3.52 (m, 1H), 3.22-3.30 (m, 3H), 3.09-3.20 (m, 1H), 2.90 (s, 1H), 2.82 (d, *J=* 3.30 Hz, 1H), 0.88 (s, 1H), 0.76-0.86 (m, 3H).

### Example 72

### HEK293-Blue-hTLR-7/8/9 cells assay

The following tests were carried out in order to determine the activity of the compounds of formula (I) in HEK293-Blue-hTLR-7/8/9 cells assay.

### HEK293-Blue-hTLR-7 cells assay:

A stable HEK293-Blue-hTLR-7 cell line was purchased from InvivoGen (Cat.#: hkb-htlr7, San Diego, California, USA). These cells were originally designed for studying the stimulation of human TLR7 by monitoring the activation of NF-xB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR7 cells with TLR7 ligands. Therefore the reporter expression was declined by TLR7 antagonist under the stimulation of a ligand, such as R848 (Resiquimod), for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR7 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM R848 in above DMEM, perform incubation under 37 °C in a CO₂ incubator for 20 hrs. Then 20 µL of the supernatant from each well was incubated with 180 µL Quanti-blue substrate solution at 37 °C for 2 hrs and the absorbance was read at 620-655 nm using a spectrophotometer. The signalling pathway that TLR7 activation leads to downstream NF-κB activation has been widely accepted, and therefore similar reporter assay was modified for evaluating TLR7 antagonist.

### HEK293-Blue-hTLR-8 cells assay:

A stable HEK293-Blue-hTLR-8 cell line was purchased from InvivoGen (Cat.#: hkb-htlr8, San Diego, California, USA). These cells were originally designed for studying the stimulation of human TLR8 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR8 cells with TLR8 ligands. Therefore the reporter expression was declined by TLR8 antagonist under the stimulation of a ligand, such as R848, for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR8 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 60uM R848 in above DMEM, perform incubation under 37 °C in a CO₂ incubator for 20 hrs. Then 20 µL of the supernatant from each well was incubated with 180 µL Quanti-blue substrate solution at 37°C for 2 hrs and the absorbance was read at 620-655 nm using a spectrophotometer. The signalling pathway that TLR8 activation leads to downstream NF-κB activation has been widely accepted, and therefore similar reporter assay was modified for evaluating TLR8 antagonist.

### HEK293-Blue-hTLR-9 cells assay:

A stable HEK293-Blue-hTLR-9 cell line was purchased from InvivoGen (Cat.#: hkb-htlr9, San Diego, California, USA). These cells were originally designed for studying the stimulation of human TLR9 by monitoring the activation of NF-xB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR9 cells with TLR9 ligands. Therefore the reporter expression was declined by TLR9 antagonist under the stimulation of a ligand, such as ODN2006 (Cat.#: tlrl-2006-1, Invivogen, San Diego, California, USA), for incubation of 20 hrs. The cell culture supernatant SEAP reporter activity was determined using QUANTI-Blue^{™} kit (Cat.#: rep-qb1, Invivogen, San Diego, California, USA) at a wavelength of 640 nm, a detection medium that turns purple or blue in the presence of alkaline phosphatase.

HEK293-Blue-hTLR9 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM ODN2006 in above DMEM, perform incubation under 37 °C in a CO₂ incubator for 20 hrs. Then 20 µL of the supernatant from each well was incubated with 180 µL Quanti-blue substrate solution at 37 °C for 2 hrs and the absorbance was read at 620-655 nm using a spectrophotometer. The signalling pathway that TLR9 activation leads to downstream NF-xB activation has been widely accepted, and therefore similar reporter assay was modified for evaluating TLR9 antagonist.

The compounds of formula (I) have human TLR7 and/or TLR8 inhibitory activities (IC₅₀ value) <0.5 µM, particularly <0.020 µM. Moreover, particular compounds of this invention also have human TLR9 inhibitory activity <10 µM. Activity data of the compounds of the present invention were shown in Table 1.

**Table 1: The activity of the compounds of present invention in HEK293-Blue-hTLR-7/8/9 cells assays**

| **Example** | **TLR7 IC₅₀ (µM)** | **TLR8 IC₅₀ (µM)** | **TLR9 IC₅₀ (µM)** |
|---|---|---|---|
| **1** | 0.003 | 0.013 | 5.2 |
| **2** | 0.004 | 0.018 | 5.5 |
| **3** | 0.004 | 0.005 | 5.7 |
| **4** | 0.009 | 0.025 | 9.9 |
| **5** | 0.008 | 0.011 | 10.4 |
| **6** | 0.009 | 0.291 | 1.4 |
| **7A** | 0.004 | 0.007 | 3.2 |
| **7B** | 0.006 | 0.026 | 3.3 |
| **8** | 0.010 | 0.216 | 8.3 |
| **9** | 0.012 | 0.044 | 6.7 |
| **10** | 0.012 | 0.245 | 9.5 |
| **11** | 0.013 | 0.030 | 1.0 |
| **12** | 0.018 | 0.227 | 1.0 |
| **13** | 0.018 | 0.048 | 2.1 |
| **14** | 0.020 | 0.193 | 4.5 |
| **15** | 0.021 | 0.156 | 7.3 |
| **16** | 0.022 | 0.072 | 4.6 |
| **17** | 0.022 | 0.066 | 1.0 |
| **18** | 0.023 | 0.075 | 10.8 |
| **20** | 0.025 | 0.369 | 13.1 |
| **21** | 0.028 | 0.077 | 14.0 |
| **22** | 0.028 | 0.065 | 8.2 |
| **23** | 0.028 | 0.243 | 10.4 |
| **24** | 0.029 | 0.175 | 27.8 |
| **25** | 0.033 | 0.152 | 13.9 |
| **26** | 0.036 | 0.044 | 11.5 |
| **27** | 0.038 | 0.052 | 4.5 |
| **28** | 0.039 | 0.052 | 6.4 |
| **29** | 0.040 | 0.096 | 6.9 |
| **30A** | 0.041 | 0.067 | 17.5 |
| **30B** | 0.050 | 0.173 | 16.2 |
| **31** | 0.041 | 0.167 | 9.3 |
| **32** | 0.044 | 0.213 | 2.4 |
| **33** | 0.045 | 0.075 | 30.2 |
| **34** | 0.046 | 0.437 | 16.7 |
| **35** | 0.055 | 0.038 | 4.3 |
| **36** | 0.057 | 0.251 | 21.8 |
| **37** | 0.059 | 0.489 | 5.2 |
| **38** | 0.059 | 0.172 | 12.3 |
| **39** | 0.074 | 0.447 | 21.1 |
| **40** | 0.079 | 0.134 | 6.9 |
| **41A** | 0.079 | 0.191 | 4.5 |
| **43** | 0.081 | 0.124 | 17.9 |
| **44** | 0.084 | 0.404 | 9.1 |
| **45** | 0.097 | 0.218 | 7.0 |
| **46** | 0.108 | 0.051 | 15.6 |
| **47** | 0.124 | 0.404 | 23.1 |
| **50** | 0.178 | 0.147 | 3.4 |
| **51** | 0.201 | 0.398 | 2.0 |
| **52A** | 0.203 | 0.225 | 12.3 |
| **52B** | 0.219 | 0.274 | 11.6 |
| **53** | 0.226 | 0.225 | 2.6 |
| **56** | 0.282 | 0.373 | 18.6 |
| **60** | 0.492 | 0.048 | 3.2 |
| **68** | 0.036 | 0.056 | 3.4 |
| **69** | 0.055 | 0.081 | 3.7 |
| **70A** | 0.019 | 0.034 | 6.9 |
| **70B** | 0.233 | 0.168 | 2.8 |
| **71A** | 0.021 | 0.075 | 3.8 |
| **71B** | 0.202 | 0.134 | 1.9 |

## Claims

1. A compound of formula (I), 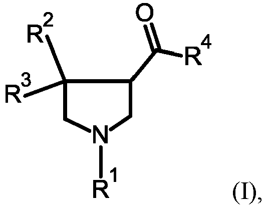 wherein
R¹ is 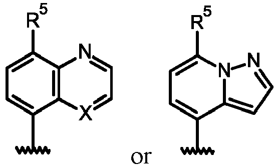 , wherein
R⁵ is cyano, C₁₋₆alkyl, halogen, haloC₁₋₆alkyl or nitro;
X is N or CH;
R² and R³ are independently selected from H, C₁₋₆alkyl, C₃₋₇cycloalkyl, haloC₁₋₆alkyl, or R² and R³ together with the carbon they are attached to form C₃₋₇cycloalkyl;
R⁴ is heterocyclyl, heterocyclylamino, heterocyclylC₁₋₆alkylamino, (C₁₋₆alkyl)₂aminoC₁₋₆alkylamino, aminoC₃₋₇cycloalkylamino, [(C₁₋₆alkyl)₂aminoC₁₋₆alkyl]C₃₋₇cycloalkylamino, aminoC₃₋₇cycloalkylC₁₋₆alkylamino or aminoC₁₋₆alkylamino;
or a pharmaceutically acceptable salt, enantiomer or diastereomer thereof.

2. A compound according to claim 1, wherein
R¹ is 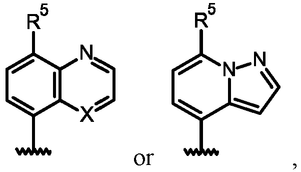 wherein
R⁵ is cyano, C₁₋₆alkyl, halogen, haloC₁₋₆alkyl or nitro;
X is N or CH;
R² is H;
R³ is H, C₁₋₆alkyl, C₃₋₇cycloalkyl, haloC₁₋₆alkyl;
or R² and R³ together with the carbon they are attached to form C₃₋₇cycloalkyl;
R⁴ is heterocyclyl, heterocyclylamino, heterocyclylC₁₋₆alkylamino, (C₁₋₆alkyl)₂aminoC₁₋₆alkylamino, aminoC₃₋₇cycloalkylamino, [(C₁₋₆alkyl)₂aminoC₁₋₆alkyl]C₃₋₇cycloalkylamino, aminoC₃₋₇cycloalkylC₁₋₆alkylamino or aminoC₁₋₆alkylamino;
or a pharmaceutically acceptable salt, enantiomer or diastereomer thereof.

3. A compound according to claim 2, wherein R⁴ is (C₁₋₆alkyl)₂aminoC₁₋₆alkylamino, (C₁₋₆alkylmorpholinyl)C₁₋₆alkylamino, (C₁₋₆alkylpiperidyl)C₁₋₆alkylamino, (morpholinylC₁₋₆alkyl)amino, [(C₁₋₆alkyl)₂aminoC₁₋₆alkyl]C₃₋₇cycloalkylamino, aminoazetidinyl, aminobicyclo[3.1.0]hexanylamino, aminoC₁₋₆alkylamino, aminoC₃₋₇cycloalkylamino, aminoC₃₋₇cycloalkylC₁₋₆alkylamino, aminospiro[3.3]heptanylamino, azabicyclo[2.2.1]heptanylamino, azabicyclo[3.2.1]octanylamino, azabicyclo[3.3.1]nonanylamino, azaspiro[3.5]nonanylamino, azepanylamino, C₁₋₆alkoxycarbonylpyrrolidinylamino, C₁₋₆alkylazaspiro[2.4]heptanylamino, C₁₋₆alkylpiperidylamino, diazaspiro[4.4]nonanyl, diazaspiro[5.5]undecanyl, halopyrrolidinylamino, morpholinylC₁₋₆alkylamino, octahydrocyclopenta[c]pyrrolylamino, oxaazabicyclo[3.3.1]nonanylamino or piperidylpiperidylamino.

4. A compound according to claim 3, wherein R⁵ is cyano, methyl, chloro, trifluoromethyl or nitro.

5. A compound according to claim 4, wherein R³ is H, methyl, ethyl, isopropyl, difluoromethyl, trifluoromethyl or cyclopropyl; or R² and R³ together with the carbon they are attached to form cyclopropyl.

6. A compound according to claim 5, wherein R³ is methyl or trifluoromethyl; or R² and R³ together with the carbon they are attached to form cyclopropyl.

7. A compound according to claim 5 or 6, wherein R⁴ is (3-aminocyclobutyl)methylamino, (dimethylamino)ethylamino, (methylmorpholinyl)methylamino, (methylpiperidyl)methylamino, (morpholinylethyl)amino, (morpholinylmethyl)amino, [(dimethylamino)methyl]cyclobutylamino, 1,2,3,3a,4,5,6,6a-octahydrocyclopenta[c]pyrrol-5-ylamino, 2,7-diazaspiro[4.4]nonanyl, 2-azabicyclo[2.2.1]heptan-5-ylamino, 3,9-diazaspiro[5.5]undecanyl, 3-azabicyclo[3.2.1]octan-8-ylamino, 3-azabicyclo[3.3.1]nonan-7-ylamino, 3-azabicyclo[3.3.1]nonan-9-ylamino, 3-oxa-7-azabicyclo[3.3.1]nonan-9-ylamino, 3-oxa-9-azabicyclo[3.3.1]nonan-7-ylamino, 5-methyl-5-azaspiro[2.4]heptan-7-ylamino, 6-aminospiro[3.3]heptan-2-ylamino, 7-azaspiro[3.5]nonan-2-ylamino, 8-azabicyclo[3.2.1]octan-3-ylamino, 9-azabicyclo[3.3.1]nonan-3-ylamino, amino-2-bicyclo[3.1.0]hexanylamino, aminoazetidinyl, aminocyclobutylamino, aminocyclohexylamino, aminomethylpropylamino, azepanylamino, fluoropyrrolidinylamino, methoxycarbonylpyrrolidinylamino, methylpiperidylamino or piperidylpiperidylamino.

8. A compound according to claim 7, wherein R⁴ is methylpiperidylamino.

9. A compound according to claim 3, selected from:
(*3R*,*4R*)-1-(8-cyanoquinoxalin-5-yl)-*N*-(1-methyl-4-piperidyl)-4-(trifluoromethyl) pyrrolidine-3-carboxamide;
*(3R,4R)*-*N*-(1-methyl-4-piperidyl)-4-(trifluoromethyl)-1-[8-(trifluoromethyl)quinoxalin-5-yl]pyrrolidine-3-carboxamide;
*(3R*,*4R)*-1-(8-cyanoquinoxalin-5-yl)-4-methyl-*N*-(1-methyl-4-piperidyl)pyrrolidine-3-carboxamide;
*(3R*,*4R)*-1-(8-cyano-5-quinolyl)-*N*-(1-methyl-4-piperidyl)-4-(trifluoromethyl)pyrrolidine-3-carboxamide;
*(3R*,*4R)*-4-methyl-*N*-(1-methyl-4-piperidyl)-1-[8-(trifluoromethyl)quinoxalin-5-yl]pyrrolidine-3-carboxamide;
*N*-(3-oxa-9-azabicyclo[3.3.1]nonan-7-yl)-5-[8-(trifluoromethyl)quinoxalin-5-yl]-5-azaspiro[2.4]heptane-7-carboxamide;
5-(8-Cyanoquinoxalin-5-yl)-*N*-(1-methyl-4-piperidyl)-5-azaspiro[2.4]heptane-7-carboxamide;
(7R)-5-(8-Cyanoquinoxalin-5-yl)-*N*-(1-methyl-4-piperidyl)-5-azaspiro[2.4]heptane-7-carboxamide;
(7S)-5-(8-Cyanoquinoxalin-5-yl)-*N*-(1-methyl-4-piperidyl)-5-azaspiro[2.4]heptane-7-carboxamide;
*N*-(3-azabicyclo[3.3.1]nonan-9-yl)-5-[8-(trifluoromethyl)quinoxalin-5-yl]-5-azaspiro[2.4]heptane-7-carboxamide;
*N*-(9-azabicyclo[3.3.1]nonan-3-yl)-5-[8-(trifluoromethyl)quinoxalin-5-yl]-5-azaspiro[2.4]heptane-7-carboxamide;
*N*-(1-methyl-4-piperidyl)-5-[8-(trifluoromethyl)quinoxalin-5-yl]-5-azaspiro[2.4]heptane-7-carboxamide;
*N*-(3-azabicyclo[3.2.1]octan-8-yl)-5-[8-(trifluoromethyl)quinoxalin-5-yl]-5-azaspiro[2.4]heptane-7-carboxamide;
5-(8-Cyanoquinoxalin-5-yl)-*N*-(morpholin-2-ylmethyl)-5-azaspiro[2.4]heptane-7-carboxamide;
*Trans*-1-(8-cyano-5-quinolyl)-4-cyclopropyl-*N*-(1-methyl-4-piperidyl)pyrrolidine-3-carboxamide;
*N*-(3-azabicyclo[3.3.1]nonan-7-yl)-5-[8-(trifluoromethyl)quinoxalin-5-yl]-5-azaspiro[2.4]heptane-7-carboxamide;
*(3R,4R)*-1-(8-cyano-5-quinolyl)-4-methyl-*N*-(1-methyl-4-piperidyl)pyrrolidine-3-carboxamide;
*Trans*-1-(8-cyano-5-quinolyl)-*N*-(1-methyl-4-piperidyl)-4-(trifluoromethyl)pyrrolidine-3-carboxamide;
1-(8-Cyano-5-quinolyl)-N (1-methyl-4-piperidyl)-4-(trifluoromethyl)pyrrolidine-3-carboxamide;
*Trans*-1-(8-cyano-5-quinolyl)-4-cyclopropyl-*N*-[2-(dimethylamino)ethyl]pyrrolidine-3-carboxamide;
*N*-(5-methyl-5-azaspiro[2.4]heptan-7-yl)-5-[8-(trifluoromethyl)quinoxalin-5-yl]-5-azaspiro[2.4]heptane-7-carboxamide;
*N*-(1,2,3,3a,4,5,6,6a-octahydrocyclopenta[c]pyrrol-5-yl)-5-[8-(trifluoromethyl)quinoxalin-5-yl]-5-azaspiro[2.4]heptane-7-carboxamide;
*N*-(6-aminospiro[3.3]heptan-2-yl)-5-[8-(trifluoromethyl)quinoxalin-5-yl]-5-azaspiro[2.4]heptane-7-carboxamide;
*N*-(8-azabicyclo[3.2.1]octan-3-yl)-5-[8-(trifluoromethyl)quinoxalin-5-yl]-5-azaspiro[2.4]heptane-7-carboxamide;
*N*-(4-aminocyclohexyl)-5-[8-(trifluoromethyl)quinoxalin-5-yl]-5-azaspiro[2.4]heptane-7-carboxamide;
*(3S,4S)*-4-Methyl-*N*-(1-methyl-4-piperidyl)-1-[8-(trifluoromethyl)quinoxalin-5-yl]pyrrolidine-3-carboxamide;
*N*-[3-[(dimethylamino)methyl]cyclobutyl]-5-[8-(trifluoromethyl)quinoxalin-5-yl]-5-azaspiro[2.4]heptane-7-carboxamide;
*(3R,4R)*-4-Methyl-N-(1-methyl-4-piperidyl)-1-(8-nitro-5-quinolyl)pyrrolidine-3-carboxamide;
*Trans*-1-(8-cyano-5-quinolyl)-4-methyl-*N*-(1-methyl-4-piperidyl)pyrrolidine-3 - carboxamide;
*Trans*-1-(8-cyano-5-quinolyl)-4-ethyl-*N*-(1-methyl-4-piperidyl)pyrrolidine-3-carboxamide;
*N*-(azepan-4-yl)-1-(8-cyano-5-quinolyl)-4-(trifluoromethyl)pyrrolidine-3-carboxamide;
*N*-[(3-aminocyclobutyl)methyl]-5-[8-(trifluoromethyl)quinoxalin-5-yl]-5-azaspiro[2.4]heptane-7-carboxamide;
*Trans*-5-[3-(3,9-diazaspiro[5.5]undecane-3-carbonyl)-4-methyl-pyrrolidin-1-yl]quinoline-8-carbonitrile;
*(3R,4R)*-4-Methyl-*N*-(1-methyl-4-piperidyl-4-piperidyl)-1-(8-methylquinoxalin-5-yl)pyrrolidine-3-carboxamide;
*N*-(7-azaspiro[3.5]nonan-2-yl)-5-[8-(trifluoromethyl)quinoxalin-5-yl]-5-azaspiro[2.4]heptane-7-carboxamide;
*(3R*,*4R)*-1-(8-Cyanoquinoxalin-5-yl)-*N*-[1-(4-piperidyl)-4-piperidyl]-4-(trifluoromethyl)pyrrolidine-3-carboxamide;
*N*-[(*1R*,*2S*,*4R*,*5S*)-4-amino-2-bicyclo[3.1.0]hexanyl]-5-[8-(trifluoromethyl)quinoxalin-5-yl]-5-azaspiro[2.4]heptane-7-carboxamide;
Trans-1-(8-cyano-5-quinolyl)-4-isopropyl-*N*-(1-methyl-4-piperidyl)pyrrolidine-3-carboxamide;
*N*-(3-Aminocyclobutyl)-5-[8-(trifluoromethyl)quinoxalin-5-yl]-5-azaspiro[2.4]heptane-7-carboxamide;
*N*-(4-Aminocyclohexyl)-5-[8-(trifluoromethyl)quinoxalin-5-yl]-5-azaspiro[2.4]heptane-7-carboxamide;
Trans-1-(8-cyano-5-quinolyl)-4-(difluoromethyl)-*N*-(1-methyl-4-piperidyl)pyrrolidine-3-carboxamide;
5-(8-Cyano-5-quinolyl)-*N*-(1-methyl-4-piperidyl)-5-azaspiro[2.4]heptane-7-carboxamide;
*N*-(3-Oxa-7-azabicyclo[3.3.1]nonan-9-yl)-5-[8-(trifluoromethyl)quinoxalin-5-yl]-5-azaspiro[2.4]heptane-7-carboxamide;
*Cis*-*N*-(3-aminocyclohexyl)-5-[8-(trifluoromethyl)quinoxalin-5-yl]-5-azaspiro[2.4]heptane-7-carboxamide;
*(3R*,*4R)*-1-(7-cyanopyrazolo[1,5-a]pyridin-4-yl)-4-methyl-*N*-(1-methyl-4-piperidyl)pyrrolidine-3-carboxamide;
*Trans*-1-(8-chloro-5-quinolyl)-*N*-(1-methyl-4-piperidyl)-4-(trifluoromethyl)pyrrolidine-3-carboxamide;
*Trans*-1-(8-cyano-5-quinolyl)-*N*-[(3S,4R)-4-fluoropyrrolidin-3-yl]-4-(trifluoromethyl)pyrrolidine-3-carboxamide;
*N*-(2-Azabicyclo[2.2.1]heptan-5-yl)-5-[8-(trifluoromethyl)quinoxalin-5-yl]-5-azaspiro[2.4]heptane-7-carboxamide;
*Trans*-1-(8-cyano-5-quinolyl)-*N*-[(*3S*,*4R*)-4-fluoropyrrolidin-3-yl]-4-isopropyl-pyrrolidine-3-carboxamide;
*(3R,4R)-*1-(8-chloro-5-quinolyl)-4-methyl-*N*-(1-methyl-4-piperidyl)pyrrolidine-3-carboxamide;
5-(8-Cyano-5-quinolyl)-*N*-[(1-methyl-3-piperidyl)methyl]-5-azaspiro[2.4]heptane-7-carboxamide;
*Trans-N*-(azepan-4-yl)-1-(8-cyano-5-quinolyl)-4-methyl-pyrrolidine-3-carboxamide;
*Trans*-5-[3-(2,7-diazaspiro[4.4]nonane-2-carbonyl)-4-methyl-pyrrolidin-1-yl]quinoline-8-carbonitrile;
(*3S*,*4S*)-1-(8-Cyano-5-quinolyl)-4-methyl-*N*-(1-methyl-4-piperidyl)pyrrolidine-3-carboxamide;
*Trans*-1-(8-cyano-5-quinolyl)-4-cyclopropyl-*N*-(2-morpholinoethyl)pyrrolidine-3-carboxamide;
*Trans*-1-(8-cyano-5-quinolyl)-*N*-[(*3S*,*4R*)-4-fluoropyrrolidin-3-yl]-4-methyl-pyrrolidine-3-carboxamide;
*N*-(Azepan-4-yl)-5-(8-cyano-5-quinolyl)-5-azaspiro[2.4]heptane-7-carboxamide;
(*3S*,*4S*)-1-(8-cyano-5-quinolyl)-*N*-(1-methyl-4-piperidyl)-4-(trifluoromethyl)pyrrolidine-3-carboxamide;
*N*-(2-Amino-2-methyl-propyl)-5-(8-cyano-5-quinolyl)-5-azaspiro[2.4]heptane-7-carboxamide;
(*3R,4R*)-1-(8-cyanoquinoxalin-5-yl)-4-methyl-*N*-[1-(4-piperidyl)-4-piperidyl]pyrrolidine-3-carboxamide;
*Trans-N*-(azepan-4-yl)-1-(8-cyano-5-quinolyl)-4-(difluoromethyl)pyrrolidine-3-carboxamide;
(*3S*,*4S*)-1-(8-Chloro-5-quinolyl)-4-methyl-*N*-(1-methyl-4-piperidyl)pyrrolidine-3-carboxamide;
5-(8-Cyano-5-quinolyl)-*N*-[(4-methylmorpholin-2-yl)methyl]-5-azaspiro[2.4]heptane-7-carboxamide;
1-(8-Cyano-5-quinolyl)-*N*-(1-methyl-4-piperidyl)pyrrolidine-3-carboxamide;
5-[7-(3-Aminoazetidine-1-carbonyl)-5-azaspiro[2.4]heptan-5-yl]quinoline-8-carbonitrile;
Methyl (*2R*,*4R*)-4-[[5-[8-(trifluoromethyl)quinoxalin-5-yl]-5-azaspiro[2.4]heptane-7-carbonyl]amino]pyrrolidine-2-carboxylate;
*Trans*-1-(8-cyano-5-quinolyl)-4-(difluoromethyl)-*N*-[(*3S*,*4R*)-4-fluoropyrrolidin-3-yl]pyrrolidine-3-carboxamide;
5-(8-Cyanoquinoxalin-5-yl)-*N*-[[(*2R*)-morpholin-2-yl]methyl]-5-azaspiro[2.4]heptane-7-carboxamide; and
5-(8-Cyanoquinoxalin-5-yl)-*N*-[[(*2S*)-morpholin-2-yl]methyl]-5-azaspiro[2.4]heptane-7-carboxamide;
or a pharmaceutically acceptable salt, enantiomer or diastereomer thereof.

10. A compound according to claim 9, selected from:
(*3R*, *4R*)-1-(8-cyanoquinoxalin-5-yl)-N-(1-methyl-4-piperidyl)-4-(trifluoromethyl) pyrrolidine-3-carboxamide;
*(3R,4R)*-*N-*(1-methyl-4-piperidyl)-4-(trifluoromethyl)-1-[8-(trifluoromethyl)quinoxalin-5-yl]pyrrolidine-3-carboxamide;
*(3R*,*4R)*-1-(8-cyanoquinoxalin-5-yl)-4-methyl-*N*-(1-methyl-4-piperidyl)pyrrolidine-3-carboxamide;
*(3R,4R)-*4-methyl-*N*-(1-methyl-4-piperidyl)-1-[8-(trifluoromethyl)quinoxalin-5-yl]pyrrolidine-3-carboxamide;
5-(8-Cyanoquinoxalin-5-yl)-*N*-(1-methyl-4-piperidyl)-5-azaspiro[2.4]heptane-7-carboxamide;
(7R)-5-(8-Cyanoquinoxalin-5-yl)-*N*-(1-methyl-4-piperidyl)-5-azaspiro[2.4]heptane-7-carboxamide; and
(7S)-5-(8-Cyanoquinoxalin-5-yl)-*N*-(1-methyl-4-piperidyl)-5-azaspiro[2.4]heptane-7-carboxamide;
or a pharmaceutically acceptable salt, enantiomer or diastereomer thereof.

11. A process for the preparation of a compound according to any one of claims 1 to 10 comprising the following step:
a) condensation of carboxylic acid (VI), 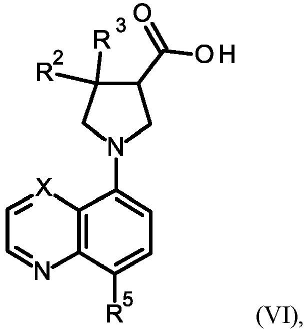
with amine (VII) in the presence of a coupling reagent;
wherein the coupling reagent is HATU; X is N or CH; R², R³ and R⁵ are defined as in any one of claims 1 to 9.

12. A compound or pharmaceutically acceptable salt, enantiomer or diastereomer according to any one of claims 1 to 10 for use as therapeutically active substance.

13. A pharmaceutical composition comprising a compound in accordance with any one of claims 1 to 10 and a therapeutically inert carrier.

14. A compound or pharmaceutically acceptable salt, enantiomer or diastereomer according to any one of claims 1 to 10 for use in the treatment or prophylaxis of systemic lupus erythematosus or lupus nephritis.

## Patentansprüche

1. Verbindung der Formel (I) 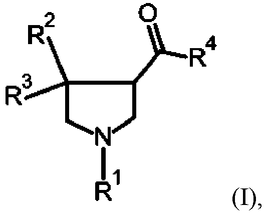 wobei
R¹ 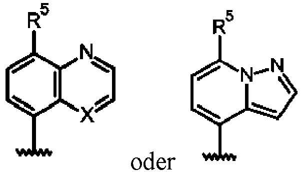 ist, wobei
R⁵ Cyano, C₁₋₆-Alkyl, Halogen, Halogen-C₁₋₆-alkyl oder Nitro ist;
X N oder CH ist;
R² und R³ unabhängig voneinander aus H, C₁₋₆-Alkyl, C₃₋₇-Cycloalkyl, Halogen-C₁₋₆-alkyl ausgewählt sind; oder R² und R³ zusammen mit dem Kohlenstoff, an den sie gebunden sind, C₃₋₇-Cycloalkyl bilden;
R⁴ Heterocyclyl, Heterocyclylamino, Heterocyclyl-C₁₋₆-alkylamino, (C₁₋₆-Alkyl)₂-amino-C₁₋₆-alkylamino, Amino-C₃₋₇-cycloalkylamino, [(C₁₋₆-Alkyl)₂-amino-C₁₋₆-alkyl]-C₃₋₇-cycloalkylamino, Amino-C₃₋₇-cycloalkyl-C₁₋₆-alkylamino oder Amino-C₁₋₆-alkylamino ist;
oder ein pharmazeutisch unbedenkliches Salz, Enantiomer oder Diastereomer davon.

2. Verbindung nach Anspruch 1, wobei
R¹ 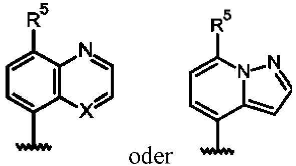 ist, wobei
R⁵ Cyano, C₁₋₆-Alkyl, Halogen, Halogen-C₁₋₆-alkyl oder Nitro ist;
X N oder CH ist;
R² H ist;
R³ H, C₁₋₆-Alkyl, C₃₋₇-Cycloalkyl, Halogen-C₁₋₆-alkyl ist;
oder R² und R³ zusammen mit dem Kohlenstoff, an den sie gebunden sind, C₃₋₇-Cycloalkyl bilden;
R⁴ Heterocyclyl, Heterocyclylamino, Heterocyclyl-C₁₋₆-alkylamino, (C₁₋₆-Alkyl)₂-amino-C₁₋₆-alkylamino, Amino-C₃₋₇-cycloalkylamino, [(C₁₋₆-Alkyl)₂-amino-C₁₋₆-alkyl]-C₃₋₇-cycloalkylamino, Amino-C₃₋₇-cycloalkyl-C₁₋₆-alkylamino oder Amino-C₁₋₆-alkylamino ist;
oder ein pharmazeutisch unbedenkliches Salz, Enantiomer oder Diastereomer davon.

3. Verbindung nach Anspruch 2, wobei R⁴ (C₁₋₆-Alkyl)₂-amino-C₁₋₆-alkylamino, (C₁₋₆-Alkylmorpholinyl)-C₁₋₆-alkylamino, (C₁₋₆-Alkylpiperidyl)-C₁₋₆-alkylamino, (Morpholinyl-C₁₋₆-alkyl)amino, [(C₁₋₆-Alkyl)₂-amino-C₁₋₆-alkyl]-C₃₋₇-cycloalkylamino, Aminoazetidinyl, Aminobicyclo[3.1.0]hexanylamino, Amino-C₁₋₆-alkylamino, Amino-C₃₋₇-cycloalkylamino, Amino-C₃₋₇-cycloalkyl-C₁₋₆-alkylamino, Aminospiro[3.3]heptanylamino, Azabicyclo[2.2.1]heptanylamino, Azabicyclo[3.2.1]octanylamino, Azabicyclo[3.3.1]nonanylamino, Azaspiro[3.5]nonanylamino, Azepanylamino, C₁₋₆-Alkoxycarbonylpyrrolidinylamino, C₁₋₆-Alkylazaspiro[2.4]heptanylamino, C₁₋₆-Alkylpiperidylamino, Diazaspiro[4.4]nonanyl, Diazaspiro[5.5]undecanyl, Halogenpyrrolidinylamino, Morpholinyl-C₁₋₆-alkylamino, Octahydrocyclopenta[c]pyrrolylamino, Oxaazabicyclo[3.3.1]nonanylamino oder Piperidylpiperidylamino ist.

4. Verbindung nach Anspruch 3, wobei R⁵ Cyano, Methyl, Chlor, Trifluormethyl oder Nitro ist.

5. Verbindung nach Anspruch 4, wobei R³ H, Methyl, Ethyl, Isopropyl, Difluormethyl, Trifluormethyl oder Cyclopropyl ist; oder R² und R³ zusammen mit dem Kohlenstoff, an den sie gebunden sind, Cyclopropyl bilden.

6. Verbindung nach Anspruch 5, wobei R³ Methyl oder Trifluormethyl ist; oder R² und R³ zusammen mit dem Kohlenstoff, an den sie gebunden sind, Cyclopropyl bilden.

7. Verbindung nach Anspruch 5 oder 6, wobei R⁴ (3-Aminocyclobutyl)methylamino, (Dimethylamino)ethylamino, (Methylmorpholinyl)methylamino, (Methylpiperidyl)methylamino, (Morpholinylethyl)amino, (Morpholinylmethyl)amino, [(Dimethylamino)methyl]cyclobutylamino, 1,2,3,3a,4,5,6,6a-Octahydrocyclopenta[c]pyrrol-5-ylamino, 2,7-Diazaspiro[4.4]nonanyl, 2-Azabicyclo[2.2.1]heptan-5-ylamino, 3,9-Diazaspiro[5.5]undecanyl, 3-Azabicyclo[3.2.1]octan-8-ylamino, 3-Azabicyclo[3.3.1]nonan-7-ylamino, 3-Azabicyclo[3.3.1]nonan-9-ylamino, 3-Oxa-7-azabicyclo[3.3.1]nonan-9-ylamino, 3-Oxa-9-azabicyclo[3.3.1]nonan-7-ylamino, 5-Methyl-5-azaspiro[2.4]heptan-7-ylamino, 6-Aminospiro[3.3]heptan-2-ylamino, 7-Azaspiro[3.5]nonan-2-ylamino, 8-Azabicyclo[3.2.1]octan-3-ylamino, 9-Azabicyclo[3.3.1]nonan-3-ylamino, Amino-2-bicyclo[3.1.0]hexanylamino, Aminoazetidinyl, Aminocyclobutylamino, Aminocyclohexylamino, Aminomethylpropylamino, Azepanylamino, Fluorpyrrolidinylamino, Methoxycarbonylpyrrolidinylamino, Methylpiperidylamino oder Piperidylpiperidylamino ist.

8. Verbindung nach Anspruch 7, wobei R⁴ Methylpiperidylamino ist.

9. Verbindung nach Anspruch 3, ausgewählt aus:
(*3R*,*4R*)-1-(8-Cyanochinoxalin-5-yl)-*N*-(1-methyl-4-piperidyl)-4-(trifluormethyl)pyrrolidin-3-carboxamid;
*(3R,4R)-N*-*(1-Methyl-4-piperidyl)-4-(trifluormethyl)-1-[8-(trifluormethyl)chinoxalin-5-*yl]pyrrolidin-3-carboxamid;
*(3R*,*4R)*-1-(8-Cyanochinoxalin-5-yl)-4-methyl-*N*-(1-methyl-4-piperidyl)pyrrolidin-3-carboxamid;
*(3R*,*4R)*-1-(8-Cyano-5-chinolyl)-*N*-(1-methyl-4-piperidyl)-4-(trifluormethyl)pyrrolidin-3-carboxamid;
*(3R*,*4R)*-4-Methyl-*N*-(1-methyl-4-piperidyl)-1-[8-(trifluormethyl)chinoxalin-5-yl]pyrrolidin-3-carboxamid;
*N*-(3-Oxa-9-azabicyclo[3.3.1]nonan-7-yl)-5-[8-(trifluormethyl)chinoxalin-5-yl]-5-azaspiro[2.4]heptan-7-carboxamid;
5-(8-Cyanochinoxalin-5-yl)-*N*-(1-methyl-4-piperidyl)-5-azaspiro[2.4]heptan-7-carboxamid;
(7R)-5-(8-Cyanochinoxalin-5-yl)-*N*-(1-methyl-4-piperidyl)-5-azaspiro[2.4]heptan-7-carboxamid;
(7S)-5-(8-Cyanochinoxalin-5-yl)-*N*-(1-methyl-4-piperidyl)-5-azaspiro[2.4]heptan-7-carboxamid;
*N*-(3-Azabicyclo[3.3.1]nonan-9-yl)-5-[8-(trifluormethyl)chinoxalin-5-yl]-5-azaspiro[2.4]heptan-7-carboxamid;
*N*-(9-Azabicyclo[3.3.1]nonan-3-yl)-5-[8-(trifluormethyl)chinoxalin-5-yl]-5-azaspiro[2.4]heptan-7-carboxamid;
*N*-(1-Methyl-4-piperidyl)-5-[8-(trifluormethyl)chinoxalin-5-yl]-5-azaspiro[2.4]heptan-7-carboxamid;
*N*-(3-Azabicyclo[3.2.1]octan-8-yl)-5-[8-(trifluormethyl)chinoxalin-5-yl]-5-azaspiro[2.4]heptan-7-carboxamid;
5-(8-Cyanochinoxalin-5-yl)-*N*-(morpholin-2-ylmethyl)-5-azaspiro[2.4]heptan-7-carboxamid;
*trans*-1-(8-Cyano-5-chinolyl)-4-cyclopropyl-*N*-(1-methyl-4-piperidyl)pyrrolidin-3-carboxamid;
*N*-(3-Azabicyclo[3.3.1]nonan-7-yl)-5-[8-(trifluormethyl)chinoxalin-5-yl]-5-azaspiro[2.4]heptan-7-carboxamid;
*(3R,4R)*-1-(8-Cyano-5-chinolyl)-4-methyl-*N*-(1-methyl-4-piperidyl)pyrrolidin-3-carboxamid;
*trans*-1-(8-Cyano-5-chinolyl)-*N*-(1-methyl-4-piperidyl)-4-(trifluormethyl)pyrrolidin-3-carboxamid;
1-(8-Cyano-5-chinolyl)-*N*-(1-methyl-4-piperidyl)-4-(trifluormethyl)pyrrolidin-3-carboxamid;
*trans*-1-(8-Cyano-5-chinolyl)-4-cyclopropyl-*N*-[2-(dimethylamino)ethyl]pyrrolidin-3-carboxamid;
*N*-(5-Methyl-5-azaspiro[2.4]heptan-7-yl)-5-[8-(trifluormethyl)chinoxalin-5-yl]-5-azaspiro[2.4]heptan-7-carboxamid;
*N*-(1,2,3,3a,4,5,6,6a-Octahydrocyclopenta[c]pyrrol-5-yl)-5-[8-(trifluormethyl)chinoxalin-5-yl]-5-azaspiro[2.4]heptan-7-carboxamid;
*N*-(6-Aminospiro[3.3]heptan-2-yl)-5-[8-(trifluormethyl)chinoxalin-5-yl]-5-azaspiro[2.4]heptan-7-carboxamid;
*N*-(8-Azabicyclo[3.2.1]octan-3-yl)-5-[8-(trifluormethyl)chinoxalin-5-yl]-5-azaspiro[2.4]heptan-7-carboxamid;
*N*-(4-Aminocyclohexyl)-5-[8-(trifluormethyl)chinoxalin-5-yl]-5-azaspiro[2,4]heptan-7-carboxamid;
*(3S*,*4S)*-4-Methyl-*N*-(1-methyl-4-piperidyl)-1-[8-(trifluormethyl)chinoxalin-5-yl]pyrrolidin-3-carboxamid;
*N*-[3-[(Dimethylamino)methyl]cyclobutyl]-5-[8-(trifluormethyl)chinoxalin-5-yl]-5-azaspiro[2.4]heptan-7-carboxamid;
*(3R*,*4R)*-4-Methyl-*N*-(1-methyl-4-piperidyl)-1-(8-nitro-5-chinolyl)pyrrolidin-3-carboxamid;
*trans*-1-(8-Cyano-5-chinolyl)-4-methyl-*N*-(1-methyl-4-piperidyl)pyrrolidin-3-carboxamid;
*trans*-1-(8-Cyano-5-chinolyl)-4-ethyl-*N*-(1-methyl-4-piperidyl)pyrrolidin-3-carboxamid;
*N*-(Azepan-4-yl)-1-(8-cyano-5-chinolyl)-4-(trifluormethyl)pyrrolidin-3-carboxamid;
*N*-[(3-Aminocyclobutyl)methyl]-5-[8-(trifluormethyl)chinoxalin-5-yl]-5-azaspiro[2.4]heptan-7-carboxamid;
*trans*-5-[3-(3,9-Diazaspiro[5.5]undecan-3-carbonyl)-4-methylpyrrolidin-1-yl]chinolin-8-carbonitril;
*(3R*,*4R)*-4-Methyl-*N*-(1-methyl-4-piperidyl)-1-(8-methylchinoxalin-5-yl)pyrrolidin-3-carboxamid;
*N*-(7-Azaspiro[3.5]nonan-2-yl)-5-[8-(trifluormethyl)chinoxalin-5-yl]-5-azaspiro[2.4]heptan-7-carboxamid;
*(3R*,*4R)*-1-(8-Cyanochinoxalin-5-yl)-N [1-(4-piperidyl)-4-piperidyl]-4-(trifluormethyl)pyrrolidin-3-carboxamid;
*N*-[(*1R*,*2S*,*4R*,*5S*)-4-Amino-2-bicyclo[3.1.0]hexanyl]-5-[8-(trifluormethyl)chinoxalin-5-yl]-5-azaspiro[2.4]heptan-7-carboxamid;
*trans*-1-(8-Cyano-5-chinolyl)-4-isopropyl-*N*-(1-methyl-4-piperidyl)pyrrolidin-3-carboxamid;
*N*-(3-Aminocyclobutyl)-5-[8-(trifluormethyl)chinoxalin-5-yl]-5-azaspiro[2.4]heptan-7-carboxamid;
*N*-(4-Aminocyclohexyl)-5-[8-(trifluormethyl)chinoxalin-5-yl]-5-azaspiro[2.4]heptan-7-carboxamid;
*trans*-1-(8-Cyano-5-chinolyl)-4-(difluormethyl)-*N*-(1-methyl-4-piperidyl)pyrrolidin-3-carboxamid;
5-(8-Cyano-5-chinolyl)-*N*-(1-methyl-4-piperidyl)-5-azaspiro[2.4]heptan-7-carboxamid;
*N*-(3-Oxa-7-azabicyclo[3.3.1]nonan-9-yl)-5-[8-(trifluormethyl)chinoxalin-5-yl]-5-azaspiro[2.4]heptan-7-carboxamid;
*cis*-*N*-(3-Aminocyclohexyl)-5-[8-(trifluormethyl)chinoxalin-5-yl]-5-azaspiro[2.4]heptan-7-carboxamid;
*(3R,4R)*-1-(7-Cyanopyrazolo[1,5-a]pyridin-4-yl)-4-methyl-*N*-(1-methyl-4-piperidyl)pyrrolidin-3-carboxamid;
*trans*-1-(8-Chlor-5-chinolyl)-*N*-(1-methyl-4-piperidyl)-4-(trifluormethyl)pyrrolidin-3-carboxamid;
*trans*-1-(8-Cyano-5-chinolyl)-*N*-[(3S,4R)-4-fluorpyrrolidin-3-yl]-4-(trifluormethyl)pyrrolidin-3-carboxamid;
*N*-(2-Azabicyclo[2.2.1]heptan-5-yl)-5-[8-(trifluormethyl)chinoxalin-5-yl]-5-azaspiro[2.4]heptan-7-carboxamid;
*trans*-1-(8-Cyano-5-chinolyl)-N-[(*3S*,*4R*)-4-fluorpyrrolidin-3-yl]-4-isopropylpyrrolidin-3-carboxamid;
(*3R,4R*)-1-(8-Chlor-5-chinolyl)-4-methyl-*N*-(1-methyl-4-piperidyl)pyrrolidin-3-carboxamid;
5-(8-Cyano-5-chinolyl)-N [(1-methyl-3-piperidyl)methyl]-5-azaspiro[2.4]heptan-7-carboxamid;
*trans-N*-(Azepan-4-yl)-1-(8-cyano-5-chinolyl)-4-methylpyrrolidin-3-carboxamid;
*trans*-5-[3-(2,7-Diazaspiro[4.4]nonan-2-carbonyl)-4-methylpyrrolidin-1-yl]chinolin-8-carbonitril;
*(3S*,*4S)*-1-(8-Cyano-5-chinolyl)-4-methyl-*N*-(1-methyl-4-piperidyl)pyrrolidin-3-carboxamid;
*trans*-1-(8-Cyano-5-chinolyl)-4-cyclopropyl-*N*-(2-morpholinoethyl)pyrrolidin-3-carboxamid;
*trans*-1-(8-Cyano-5-chinolyl)-*N*-[(*3S*,*4R*)-4-fluorpyrrolidin-3-yl]-4-methylpyrrolidin-3-carboxamid;
*N*-(Azepan-4-yl)-5-(8-cyano-5-chinolyl)-5-azaspiro[2.4]heptan-7-carboxamid;
(*3S*,*4S*)-1-(8-Cyano-5-chinolyl)-*N*-(1-methyl-4-piperidyl)-4-(trifluormethyl)pyrrolidin-3-carboxamid;
*N*-(2-Amino-2-methylpropyl)-5-(8-cyano-5-chinolyl)-5-azaspiro[2.4]heptan-7-carboxamid;
*(3R,4R*)-1-(8-Cyanochinoxalin-5-yl)-4-methyl-*N*-[1-(4-piperidyl)-4-piperidyl]pyrrolidin-3-carboxamid;
*trans-N*-(Azepan-4-yl)-1-(8-cyano-5-chinolyl)-4-(difluormethyl)pyrrolidin-3-carboxamid;
(*3S*,*4S*)-1-(8-Chlor-5-chinolyl)-4-methyl-*N*-(1-methyl-4-piperidyl)pyrrolidin-3-carboxamid;
5-(8-Cyano-5-chinolyl)-*N*-[(4-methylmorpholin-2-yl)methyl]-5-azaspiro[2.4]heptan-7-carboxamid;
1-(8-Cyano-5-chinolyl)-*N*-(1-methyl-4-piperidyl)pyrrolidin-3-carboxamid;
5-[7-(3-Aminoazetidin-1-carbonyl)-5-azaspiro[2.4]heptan-5-yl]chinolin-8-carbonitril;
Methyl-(*2R*,*4R*)-4-[[5-[8-(trifluormethyl)chinoxalin-5-yl]-5-azaspiro[2.4]heptan-7-carbonyl]amino]pyrrolidin-2-carboxylat;
*trans*-1-(8-Cyano-5-chinolyl)-4-(difluormethyl)-*N*-[(*3S*,*4R*)-4-fluorpyrrolidin-3-yl]pyrrolidin-3-carboxamid;
5-(8-Cyanochinoxalin-5-yl)-*N*-[[(2R)-morpholin-2-yl]methyl]-5-azaspiro[2.4]heptan-7-carboxamid; und
5-(8-Cyanochinoxalin-5-yl)-*N*-[[(2S)-morpholin-2-yl]methyl]-5-azaspiro[2.4]heptan-7-carboxamid;
oder ein pharmazeutisch unbedenkliches Salz, Enantiomer oder Diastereomer davon.

10. Verbindung nach Anspruch 9, ausgewählt aus:
*(3R*,*4R)*-1-(8-Cyanochinoxalin-5-yl)-*N*-(1-methyl-4-piperidyl)-4-(trifluormethyl)pyrrolidin-3-carboxamid;
*(3R,4R)-N*-*(1-Methyl-4-piperidyl)-4-(trifluormethyl)-1-[8-(trifluormethyl)chinoxalin-5-*yl]pyrrolidin-3 -carboxamid;
*(3R,4R)*-1-(8-Cyanochinoxalin-5-yl)-4-methyl-*N*-(1-methyl-4-piperidyl)pyrrolidin-3-carboxamid;
*(3R*,*4R)*-4-Methyl-*N*-(1-methyl-4-piperidyl)-1-[8-(trifluormethyl)chinoxalin-5-yl]pyrrolidin-3 -carboxamid;
5-(8-Cyanochinoxalin-5-yl)-*N*-(1-methyl-4-piperidyl)-5-azaspiro[2.4]heptan-7-carboxamid;
(7R)-5-(8-Cyanochinoxalin-5-yl)-*N*-(1-methyl-4-piperidyl)-5-azaspiro[2.4]heptan-7-carboxamid; und
(7S)-5-(8-Cyanochinoxalin-5-yl)-N-(1 -methyl-4-piperidyl)-5-azaspiro[2.4]heptan-7-carboxamid;
oder ein pharmazeutisch unbedenkliches Salz, Enantiomer oder Diastereomer davon.

11. Verfahren zur Herstellung einer Verbindung nach einem der Ansprüche 1 bis 10, umfassend den folgenden Schritt:
a) Kondensation von Carbonsäure (VI) 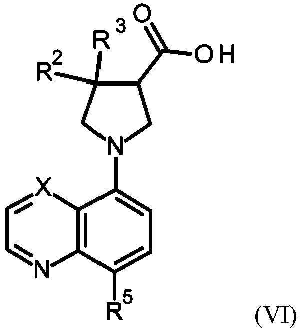
mit Amin (VII) in der Gegenwart eines Kopplungsreagens;
wobei das Kopplungsreagens HATU ist; X N oder CH ist; R², R³ und R⁵ wie in einem der Ansprüche 1 bis 9 definiert sind.

12. Verbindung oder pharmazeutisch unbedenkliches Salz, Enantiomer oder Diastereomer nach einem der Ansprüche 1 bis 10 zur Verwendung als therapeutisch wirksame Substanz.

13. Pharmazeutische Zusammensetzung, umfassend eine Verbindung nach einem der Ansprüche 1 bis 10 und einen therapeutisch inerten Träger.

14. Verbindung oder pharmazeutisch unbedenkliches Salz, Enantiomer oder Diastereomer nach einem der Ansprüche 1 bis 10 zur Verwendung bei der Behandlung oder Prophylaxe von systemischem Lupus erythematodes oder Lupusnephritis.

## Revendications

1. Composé de formule (1), 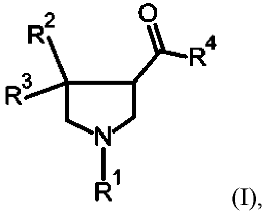 dans lequel
R¹ représente 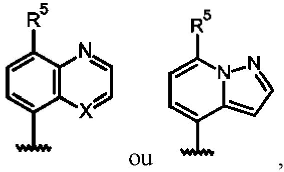 dans lequel
R⁵ représente un cyano, un alkyle en C₁₋₆, un halogène, un halogénoalkyle en C₁₋₆ ou un nitro ;
X représente N ou CH ;
R² et R³ sont indépendamment choisis parmi H, un alkyle en C₁₋₆, un cycloalkyle en C₃₋₇, un halogénoalkyle en C₁₋₆, ou R² et R³ conjointement avec le carbone auquel ils sont liés forment un cycloalkyle en C₃₋₇ ;
R⁴ représente un hétérocyclyle, un hétérocyclylamino, un hétérocyclylalkyle en C₁₋₆-amino, un (alkyle en C₁₋₆)₂aminoalkyle en C₁₋₆-amino, un aminocycloalkyle en C₃₋₇-amino, un [(alkyle en C₁₋₆)₂aminoalkyle en C₁₋₆]cycloalkyle en C₃₋₇-amino, un aminocycloalkyle en C₃₋₇-alkyle en C₁₋₆-amino ou un aminoalkyle en C₁₋₆-amino ;
ou un sel pharmaceutiquement acceptable, un énantiomère ou un diastéréoisomère de celui-ci.

2. Composé selon la revendication 1, dans lequel
R¹ représente 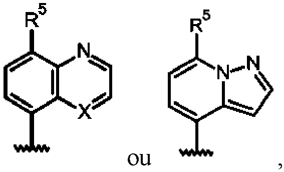 dans lequel
R⁵ représente un cyano, un alkyle en C₁₋₆, un halogène, un halogénoalkyle en C₁₋₆ ou un nitro ;
X représente N ou CH ;
R² représente H ;
R³ représente H, un alkyle en C₁₋₆, un cycloalkyle en C₃₋₇, un halogénoalkyle en C₁₋₆ ;
ou R² et R³ conjointement avec le carbone auquel ils sont liés forment un cycloalkyle en C₃₋₇ ;
R⁴ représente un hétérocyclyle, un hétérocyclylamino, un hétérocyclylalkyle en C₁₋₆-amino, un (alkyle en C₁₋₆)₂aminoalkyle en C₁₋₆-amino, un aminocycloalkyle en C₃₋₇-amino, un [(alkyle en C₁₋₆)₂aminoalkyle en C₁₋₆]cycloalkyle en C₃₋₇-amino, un aminocycloalkyle en C₃₋₇-alkyle en C₁₋₆-amino ou un aminoalkyle en C₁₋₆-amino ;
ou un sel pharmaceutiquement acceptable, un énantiomère ou un diastéréoisomère de celui-ci.

3. Composé selon la revendication 2, dans lequel R⁴ représente un (alkyle en C₁₋₆)₂aminoalkyle en C₁₋₆-amino, un (alkyle en C₁₋₆-morpholinyl)alkyle en C₁₋₆-amino, un (alkyle en C₁₋₆-pipéridyl)alkyle en C₁₋₆-amino, un (morpholinylalkyle en C₁₋₆)amino, un [(alkyle en C₁₋₆)₂aminoalkyle en C₁₋₆]cycloalkyle en C₃₋₇-amino, un aminoazétidinyle, un aminobicyclo[3.1.0]hexanylamino, un aminoalkyle en C₁₋₆-amino, un aminocycloalkyle en C₃₋₇-amino, un aminocycloalkyle en C₃₋₇-alkyle en C₁₋₆-amino, un aminospiro[3.3]heptanylamino, un azabicyclo[2.2.1]heptanylamino, un azabicyclo[3.2.1]octanylamino, un azabicyclo[3.3.1]nonanylamino, un azaspiro[3.5]nonanylamino, un azépanylamino, un alcoxy en C₁₋₆-carbonylpyrrolidinylamino, un alkyle en C₁₋₆-azaspiro[2.4]heptanylamino, un alkyle en C₁₋₆-pipéridylamino, un diazaspiro[4.4]nonanyle, un diazaspiro[5.5]undécanyle, un halogénopyrrolidinylamino, un morpholinylalkyle en C₁₋₆-amino, un octahydrocyclopenta[c]pyrrolylamino, un oxaazabicyclo[3.3.1]nonanylamino ou un pipéridylpipéridylamino.

4. Composé selon la revendication 3, dans lequel R⁵ représente un cyano, un méthyle, un chlore, un trifluorométhyle ou un nitro.

5. Composé selon la revendication 4, dans lequel R³ représente H, un méthyle, un éthyle, un isopropyle, un difluorométhyle, un trifluorométhyle ou un cyclopropyle ; ou R² et R³ conjointement avec le carbone auquel ils sont liés forment un cyclopropyle.

6. Composé selon la revendication 5, dans lequel R³ représente un méthyle ou un trifluorométhyle ; ou R² et R³ conjointement avec le carbone auquel ils sont liés forment un cyclopropyle.

7. Composé selon la revendication 5 ou 6, dans lequel R⁴ représente un (3-aminocyclobutyl)méthylamino, un (diméthylamino)éthylamino, un (méthylmorpholinyl)méthylamino, un (méthylpipéridyl)méthylamino, un (morpholinyléthyl)amino, un (morpholinylméthyl)amino, un [(diméthylamino)méthyl]cyclobutylamino, 1,2,3,3a,4,5,6,6a-octahydrocyclopenta[c]pyrrol-5-ylamino, un 2,7-diazaspiro[4.4]nonanyle, un 2-azabicyclo[2.2.1]heptan-5-ylamino, un 3,9-diazaspiro[5.5]undécanyle, un 3-azabicyclo[3.2.1]octan-8-ylamino, un 3-azabicyclo[3.3.1]nonan-7-ylamino, un 3-azabicyclo[3.3.1]nonan-9-ylamino, un 3-oxa-7-azabicyclo[3.3.1]nonan-9-ylamino, un 3-oxa-9-azabicyclo[3.3.1]nonan-7-ylamino, un 5-méthyl-5-azaspiro[2.4]heptan-7-ylamino, un 6-aminospiro[3.3]heptan-2-ylamino, un 7-azaspiro[3.5]nonan-2-ylamino, un 8-azabicyclo[3.2.1]octan-3-ylamino, un 9-azabicyclo[3.3.1]nonan-3-ylamino, un amino-2-bicyclo[3.1.0]hexanylamino, un aminoazétidinyle, un aminocyclobutylamino, un aminocyclohexylamino, un aminométhylpropylamino, un azépanylamino, un fluoropyrrolidinylamino, un méthoxycarbonylpyrrolidinylamino, un méthylpipéridylamino ou un pipéridylpipéridylamino.

8. Composé selon la revendication 7, dans lequel R⁴ représente un méthylpipéridylamino.

9. Composé selon la revendication 3, choisi parmi :
le (*3R*,*4R*)-1-(8-cyanoquinoxalin-5-yl)-*N*-(1-méthyl-4-pipéridyl)-4-(trifluorométhyl)pyrrolidine-3-carboxamide ;
le *(3R*,*4R)*-*N*-(1-méthyl-4-pipéridyl)-4-(trifluorométhyl)-1-[8-(trifluorométhyl)quinoxalin-5-yl]pyrrolidine-3-carboxamide ;
le *(3R*,*4R)*-1-(8-cyanoquinoxalin-5-yl)-4-méthyl-*N*-(1-méthyl-4-pipéridyl)pyrrolidine-3-carboxamide ;
le *(3R*,*4R)*-1-(8-cyano-5-quinoléyl)-*N*-(1-méthyl-4-pipéridyl)-4-(trifluorométhyl)pyrrolidine-3-carboxamide ;
le *(3R*,*4R)*-4-méthyl-*N*-(1-méthyl-4-pipéridyl)-1-[8-(trifluorométhyl)quinoxalin-5-yl]pyrrolidine-3-carboxamide ;
le *N*-(3-oxa-9-azabicyclo[3.3.1]nonan-7-yl)-5-[8-(trifluorométhyl)quinoxalin-5-yl]-5-azaspiro[2.4]heptane-7-carboxamide ;
le 5-(8-cyanoquinoxalin-5-yl)-*N*-(1-méthyl-4-pipéridyl)-5-azaspiro[2.4]heptane-7-carboxamide ;
le (7R)-5-(8-cyanoquinoxalin-5-yl)-*N*-(1-méthyl-4-pipéridyl)-5-azaspiro[2.4]heptane-7-carboxamide ;
le (7S)-5-(8-cyanoquinoxalin-5-yl)-*N*-(1-méthyl-4-pipéridyl)-5-azaspiro[2.4]heptane-7-carboxamide ;
le *N*-(3-azabicyclo[3.3.1]nonan-9-yl)-5-[8-(trifluorométhyl)quinoxalin-5-yl]-5-azaspiro[2.4]heptane-7-carboxamide ;
le *N*-(9-azabicyclo[3.3.1]nonan-3-yl)-5-[8-(trifluorométhyl)quinoxalin-5-yl]-5-azaspiro[2.4]heptane-7-carboxamide ;
le *N*-(1-méthyl-4-pipéridyl)-5-[8-(trifluorométhyl)quinoxalin-5-yl]-5-azaspiro[2.4]heptane-7-carboxamide ;
le *N*-(3-azabicyclo[3.2.1]octan-8-yl)-5-[8-(trifluorométhyl)quinoxalin-5-yl]-5-azaspiro[2.4]heptane-7-carboxamide ;
le 5-(8-cyanoquinoxalin-5-yl)-*N*-(morpholin-2-ylméthyl)-5-azaspiro[2.4]heptane-7-carboxamide ;
le *trans*-1-(8-cyano-5-quinoléyl)-4-cyclopropyl-*N*-(1-méthyl-4-pipéridyl)pyrrolidine-3-carboxamide ;
le *N*-(3-azabicyclo[3.3.1]nonan-7-yl)-5-[8-(trifluorométhyl)quinoxalin-5-yl]-5-azaspiro[2.4]heptane-7-carboxamide ;
le *(3R*,*4R)*-1-(8-cyano-5-quinoléyl)-4-méthyl-*N*-(1-méthyl-4-pipéridyl)pyrrolidine-3-carboxamide ;
le *trans*-1-(8-cyano-5-quinoléyl)-*N*-(1-méthyl-4-pipéridyl)-4-(trifluorométhyl)pyrrolidine-3-carboxamide ;
le 1-(8-cyano-5-quinoléyl)-*N*-(1-méthyl-4-pipéridyl)-4-(trifluorométhyl)pyrrolidine-3-carboxamide ;
le *trans*-1-(8-cyano-5-quinoléyl)-4-cyclopropyl-*N*-[2-(diméthylamino)éthyl]pyrrolidine-3-carboxamide ;
le *N*-(5-méthyl-5-azaspiro[2.4]heptan-7-yl)-5-[8-(trifluorométhyl)quinoxalin-5-yl]-5-azaspiro[2.4]heptane-7-carboxamide ;
le *N*-(1,2,3,3a,4,5,6,6a-octahydrocyclopenta[c]pyrrol-5-yl)-5-[8-(trifluorométhyl)quinoxalin-5-yl]-5-azaspiro[2.4]heptane-7-carboxamide ;
le *N*-(6-aminospiro[3.3]heptan-2-yl)-5-[8-(trifluorométhyl)quinoxalin-5-yl]-5-azaspiro[2.4]heptane-7-carboxamide ;
le *N*-(8-azabicyclo[3.2.1]octan-3-yl)-5-[8-(trifluorométhyl)quinoxalin-5-yl]-5-azaspiro[2.4]heptane-7-carboxamide ;
le *N*-(4-aminocyclohexyl)-5-[8-(trifluorométhyl)quinoxalin-5-yl]-5-azaspiro[2,4]heptane-7-carboxamide ;
le *(3S*,*4S)*-4-méthyl-*N*-(1-méthyl-4-pipéridyl)-1-[8-(trifluorométhyl)quinoxalin-5-yl]pyrrolidine-3-carboxamide ;
le *N*-[3-[(diméthylamino)méthyl]cyclobutyl]-5-[8-(trifluorométhyl)quinoxalin-5-yl]-5-azaspiro[2.4]heptane-7-carboxamide ;
le *(3R*,*4R)*-4-méthyl-N-(1-méthyl-4-pipéridyl)-1-(8-nitro-5-quinoléyl)pyrrolidine-3-carboxamide ;
le *trans*-1-(8-cyano-5-quinoléyl)-4-méthyl-*N*-(1-méthyl-4-pipéridyl)pyrrolidine-3-carboxamide ;
le *trans*-1-(8-cyano-5-quinoléyl)-4-éthyl-*N*-(1-méthyl-4-pipéridyl)pyrrolidine-3-carboxamide ;
le *N*-(azépan-4-yl)-1-(8-cyano-5-quinoléyl)-4-(trifluorométhyl)pyrrolidine-3-carboxamide ;
le *N*-[(3-aminocyclobutyl(méthyl]-5-[8-(trifluorométhyl)quinoxalin-5-yl]-5-azaspiro[2.4]heptane-7-carboxamide ;
le *trans*-5-[3-(3,9-diazaspiro[5.5]undécane-3-carbonyl)-4-méthyl-pyrrolidin-1-yl]quinoléine-8-carbonitrile ;
le *(3R*,*4R)*-4-méthyl-*N*-(1-méthyl-4-pipéridyl)-1-(8-méthylquinoxalin-5-yl)pyrrolidine-3-carboxamide ;
le *N*-(7-azaspiro[3,5]nonan-2-yl)-5-[8-(trifluorométhyl)quinoxalin-5-yl]-5-azaspiro[2.4]heptane-7-carboxamide ;
le *(3R*,*4R)*-1-(8-cyanoquinoxalin-5-yl)-*N*-[1-(4-pipéridyl)-4-pipéridyl]-4-(trifluorométhyl)pyrrolidine-3-carboxamide ;
le *N*-[(*1R*,*2S*,*4R*,*5S*)-4-amino-2-bicyclo[3.1.0]hexanyl]-5-[8-(trifluorométhyl)quinoxalin-5-yl]-5-azaspiro[2.4]heptane-7-carboxamide ;
le *trans*-1-(8-cyano-5-quinoléyl)-4-isopropyl-*N*-(1-méthyl-4-pipéridyl)pyrrolidine-3-carboxamide ;
le *N*-(3-aminocyclobutyl(-5-[8-(trifluorométhyl)quinoxalin-5-yl]-5-azaspiro[2.4]heptane-7-carboxamide ;
le *N*-(4-aminocyclohexyl(-5-[8-(trifluorométhyl)quinoxalin-5-yl]-5-azaspiro[2.4]heptane-7-carboxamide ;
le *trans*-1-(8-cyano-5-quinoléyl(-4-(difluorométhyl)-*N*-(1-méthyl-4-pipéridyl)pyrrolidine-3-carboxamide ;
le 5-(8-cyano-5-quinoléyl)-*N*-(1-méthyl-4-pipéridyl)-5-azaspiro[2.4]heptane-7-carboxamide ;
le *N*-(3-oxa-7-azabicyclo[3.3.1]nonan-9-yl)-5-[8-(trifluorométhyl)quinoxalin-5-yl]-5-azaspiro[2.4]heptane-7-carboxamide ;
le *cis*-*N*-(3-aminocyclohexyl)-5-[8-(trifluorométhyl)quinoxalin-5-yl]-5-azaspiro[2.4]heptane-7-carboxamide ;
le *(3R*,*4R)*-1-(7-cyanopyrazolo[1,5-a]pyridin-4-yl)-4-méthyl-*N*-(1-méthyl-4-pipéridyl)pyrrolidine-3-carboxamide ;
le *trans*-1-(8-chloro-5-quinoléyl)-*N*-(1-méthyl-4-pipéridyl)-4-(trifluorométhyl)pyrrolidine-3-carboxamide ;
le *trans*-1-(8-cyano-5-quinoléyl)-*N*-[(3S,4R)-4-fluoropyrrolidin-3-yl]-4-(trifluorométhyl)pyrrolidine-3-carboxamide ;
le *N*-(2-azabicyclo[2.2.1]heptan-5-yl)-5-[8-(trifluorométhyl)quinoxalin-5-yl]-5-azaspiro[2.4]heptane-7-carboxamide ;
le *trans*-1-(8-cyano-5-quinoléyl)-*N*-[(*3S*,*4R*)-4-fluoropyrrolidin-3-yl]-4-isopropyl-pyrrolidine-3-carboxamide ;
le (*3R*,*4R*)-1-(8-chloro-5-quinoléyl)-4-méthyl-*N*-(1-méthyl-4-pipéridyl)pyrrolidine-3-carboxamide ;
le 5-(8-cyano-5-quinoléyl)-N [(1-méthyl-3-pipéridyl)méthyl]-5-azaspiro[2.4]heptane-7-carboxamide ;
le *trans*-*N*-(azépan-4-yl)-1-(8-cyano-5-quinoléyl)-4-méthyl-pyrrolidine-3-carboxamide ;
le *trans*-5-[3-(2,7-diazaspiro[4.4]nonane-2-carbonyl)-4-méthyl-pyrrolidin-1-yl]quinoléine-8-carbonitrile ;
le (*3S*,*4S*)-1-(8-cyano-5-quinoléyl)-4-méthyl-*N*-(1-méthyl-4-pipéridyl)pyrrolidine-3-carboxamide ;
le *trans*-1-(8-cyano-5-quinoléyl)-4-cyclopropyl-*N*-(2-morpholinoéthyl)pyrrolidine-3-carboxamide ;
le *trans*-1-(8-cyano-5-quinoléyl)-*N*-[(*3S*,*4S*)-4-fluoropyrrolidin-3-yl]-4-méthyl-pyrrolidine-3-carboxamide ;
le *N*-(azépan-4-yl)-5-(8-cyano-5-quinoléyl)-5-azaspiro[2.4]heptane-7-carboxamide ;
le (*3S*,*4S*)-1-(8-cyano-5-quinoléyl)-*N*-(1-méthyl-4-pipéridyl)-4-(trifluorométhyl)pyrrolidine-3-carboxamide ;
le *N*-(2-amino-2-méthyl-propyl)-5-(8-cyano-5-quinoléyl)-5-azaspiro[2.4]heptane-7-carboxamide ;
le (*3R,4R*)-1-(8-cyanoquinoxalin-5-yl)-4-méthyl-*N*-[1 -(4-pipéridyl)-4-pipéridyl]pyrrolidine-3-carboxamide ;
le *trans*-*N*-(azépan-4-yl)-1-(8-cyano-5-quinoléyl)-4-(difluorométhyl)pyrrolidine-3-carboxamide ;
le (*3S*,*4S*)-1-(8-chloro-5-quinoléyl)-4-méthyl-*N*-(1-méthyl-4-pipéridyl)pyrrolidine-3-carboxamide ;
le 5-(8-cyano-5-quinoléyl)-*N*-[(4-méthylmorpholin-2-yl)méthyl]-5-azaspiro[2.4]heptane-7-carboxamide ;
le 1-(8-cyano-5-quinoléyl)-*N*-(1-méthyl-4-pipéridyl)pyrrolidine-3-carboxamide ;
le 5-[7-(3-aminoazétidine-1-carbonyl)-5-azaspiro[2.4]heptan-5-yl]quinoléine-8-carbonitrile ;
le (*2R*,*4R*)-4-[[5-[8-(trifluorométhyl)quinoxalin-5-yl]-5-azaspiro[2.4]heptane-7-carbonyl]amino]pyrrolidine-2-carboxylate de méthyle ;
le *trans*-1-(8-cyano-5-quinoléyl)-4-(difluorométhyl)-*N*-[(*3S*,*4S*)-4-fluoropyrrolidin-3-yl]pyrrolidine-3-carboxamide ;
le 5-(8-cyanoquinoxalin-5-yl)-*N*-[[(*2R*)-morpholin-2-yl]méthyl]-5-azaspiro[2.4]heptane-7-carboxamide ; et
le 5-(8-cyanoquinoxalin-5-yl)-*N*-[[(2S)-morpholin-2-yl]méthyl]-5-azaspiro[2.4]heptane-7-carboxamide ;
ou un sel pharmaceutiquement acceptable, un énantiomère ou un diastéréoisomère de ceux-ci.

10. Composé selon la revendication 9, choisi parmi :
le (*3R*,*4R*)-1-(8-cyanoquinoxalin-5-yl)-N-(1-méthyl-4-pipéridyl)-4-(trifluorométhyl)pyrrolidine-3-carboxamide ;
le *(3R*,*4R)*-*N*-(1-méthyl-4-pipéridyl)-4-(trifluorométhyl)-1-[8-(trifluorométhyl)quinoxalin-5-yl]pyrrolidine-3-carboxamide ;
le *(3R*,*4R)*-1-(8-cyanoquinoxalin-5-yl)-4-méthyl-*N*-(1-méthyl-4-pipéridyl)pyrrolidine-3-carboxamide ;
le *(3R*,*4R)*-4-méthyl-*N*-(1-méthyl-4-pipéridyl)-1-[8-(trifluorométhyl)quinoxalin-5-yl]pyrrolidine-3-carboxamide ;
le 5-(8-cyanoquinoxalin-5-yl)-*N*-(1-méthyl-4-pipéridyl)-5-azaspiro[2.4]heptane-7-carboxamide ;
le (7R)-5-(8-cyanoquinoxalin-5-yl)-*N*-(1-méthyl-4-pipéridyl)-5-azaspiro[2.4]heptane-7-carboxamide ; et
le (7S)-5-(8-cyanoquinoxalin-5-yl)-*N*-(1-méthyl-4-pipéridyl)-5-azaspiro[2.4]heptane-7-carboxamide ;
ou un sel pharmaceutiquement acceptable, un énantiomère ou un diastéréoisomère de ceux-ci.

11. Procédé de préparation d'un composé selon l'une quelconque des revendications 1 à 10 comprenant l'étape suivante :
a) la condensation d'un acide carboxylique (VI), 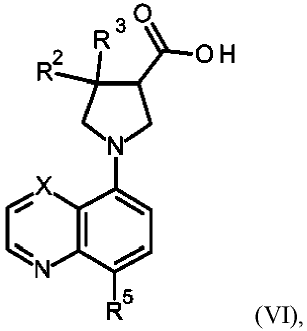
avec une amine (VII) en présence d'un réactif de couplage ;
dans lequel le réactif de couplage est le HATU ; X représente N ou CH ; R², R³ et R⁵ sont tels que définis dans l'une quelconque des revendications 1 à 9.

12. Composé ou sel pharmaceutiquement acceptable, énantiomère ou diastéréoisomère selon l'une quelconque des revendications 1 à 10 pour une utilisation comme substance thérapeutiquement active.

13. Composition pharmaceutique comprenant un composé selon l'une quelconque des revendications 1 à 10 et un véhicule thérapeutiquement inerte.

14. Composé ou sel pharmaceutiquement acceptable, énantiomère ou diastéréoisomère selon l'une quelconque des revendications 1 à 10 destiné à être utilisé dans le traitement ou la prophylaxie du lupus érythémateux disséminé ou de la néphropathie lupique.
